(19) Europäisches Patentamt
European Patent Office
Office européen des brevets

(11) Veröffentlichungsnummer: **0 076 489 B1**

(12) # EUROPÄISCHE PATENTSCHRIFT

(45) Veröffentlichungstag der Patentschrift: **16.05.90**

(21) Anmeldenummer: **82109094.1**

(22) Anmeldetag: **01.10.82**

(51) Int. Cl.$^5$: **C 12 N 15/00**, C 12 P 21/02, C 07 K 1/00, C 12 N 1/00, A 61 K 37/66, C 07 H 21/04 // C12R1/19

(54) **Desoxyribonucleinsäuren, rekombinante Desoxyribonucleinsäuren, sie enthaltende Wirte, Polypeptide und Verfahren zu ihrer Herstellung.**

(30) Priorität: **03.10.81 GB 8129937**
**26.03.82 GB 8208988**
**01.09.82 GB 8224871**

(43) Veröffentlichungstag der Anmeldung:
**13.04.83 Patentblatt 83/15**

(45) Bekanntmachung des Hinweises auf die Patenterteilung:
**16.05.90 Patentblatt 90/20**

(84) Benannte Vertragsstaaten:
**AT BE CH DE FR IT LI LU NL SE**

(56) Entgegenhaltungen:
**EP-A-0 028 033**
**EP-A-0 032 134**
**EP-A-0 034 307**
**EP-A-0 042 246**

**NATURE, Band 287, Nr. 5781, 2. Oktober 1980, Seiten 408-411, Macmillan Journals Ltd., Chesham, Bucks, GB G. Allen u.a.: "A family of structural genes for human lymphoblastoid (leukocyte-type) interferon"**

(73) Patentinhaber: **CIBA-GEIGY AG**
**Klybeckstrasse 141**
**CH-4002 Basel (CH)**

(72) Erfinder: **Meyer, François, Dr.**
**Pfaffenrainstrasse 4**
**CH-4103 Bottmingen (CH)**

(74) Vertreter: **Zumstein, Fritz jun., Dr. et al**
**Dr. F. Zumstein sen. Dr. E. Assmann Dr. R. Koenigsberger Dipl.-Ing. F. Klingseisen Dr. F. Zumstein jun. Bräuhausstrasse 4**
**D-8000 München 2 (DE)**

(56) Entgegenhaltungen:
**THE BIOLOGY OF THE INTERFERON SYSTEM, April 1981, Seiten 81-84, Elsevier North-Holland Biomedical Press, Rotterdam, NL D. SKUP u.a.: "Production of biologically active lymphoblastoid interferon in E.coli"**

**EUROPEAN JOURNAL OF CELL BIOLOGY, Band 25, 8-9, 1981, Seiten 8-9, Wissenschaftliche Verlagsgesellschaft mbH, Stuttgart, DE H. Seliger u.a.: "Synthesis of a DNA fragment specific for human interferons and its use as a probe for screening recombinant clones containing interferon sequences"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 076 489 B1

Courier Press, Leamington Spa, England.

## Beschreibung

Hintergrund der Erfindung

Interferone ("IFN") stellen ein Gruppe von meistens glycosylierten Polypeptiden mit Molekulargewichten im Bereich von 10 000 bis 40 000 dar. Sie werden von Wirbeltierzellen bei der Einwirkung eines IFN-Inducers, wie eines Virus, einer doppelsträngigen RNA, intrazellulärer Mikroben, Mikrobenprodukten oder verschiedener chemischer Agentien erhalten (1). IFNe werden im allgemeinen in normalen, gesunden Zellen nicht gefunden. Sie unterstützen die gesunden Zellen des Vertebraten in ihrer Verteidigung gegenüber viralen Infektionen und anderen Angriffen. Es wurde gefunden, daß IFNe immunomodulatorische Aktivitäten aufweisen.

Bischer wurden drei Klassen von Interferonen humanen Ursprungs ("HuIFN") identifiziert: HuIFN-α, HuIFN-β und HuIFN-γ. HuIFN-α wird von Humanleukocyten und durch Lymphoblastoidzellen bei der Induktion, z.B. mit einem Virus [E. A. Havell et al. (2) und A. D. Sagar et al. (3)], gebildet. Es ist bei pH 2 stabil und enthält ein Gemisch aus individuellen Interferon-polypeptiden, die sich hauptsächlich in ihrem Glycosylierungsgrad [z.B. M. Rubenstein et al., (4)] und in der Aminosäurezusammensetzung (vergl. die folgenden Ausführungen) unterscheiden. Von den beiden bis jetzt isolierten und gereinigten Komponenten ist eine mit einem Molekulargewicht von 15 000 bis 18 000 nicht glycosyliert, wohingegen die andere mit einem Molekulargewicht von 21 000 bis 22 000 glycosyliert ist. W. E. Stewart, II et al. (5) berichten, daß in dem nicht-glycosylierten Interferon die Hauptmenge oder die Gesamtmenge seiner HuIFN-α-Aktivität erhaltengeblieben ist. Teile der Aminosäure-Sequenz von HuIFN-α aus Lymphoblastoidzellen wurden ebenfalls in der Literatur beschrieben [K. C. Zoon et al., (6)]. Verschiedene Formen von HuIFN-α, die sich strukturell und physiologisch unterscheiden, sind ebenfalls bekannt. Besondere humane Individuen können allelomorphe Variationen von HuIFN-α bilden.

HuIFN-β wird durch human Fibroblasten bei der Induktion mit einer ds RNA und in geringem Ausmass zusammen mit HuIFN-α von humanen Lymphoblastoidzellen bei der Induktion mit einem Virus gebildet. HuIFN-β ist ebenfalls bei pH 2 stabil. Mindestens zwei Typen von HuIFN-β wurden bis jetzt beschrieben (7, 8). Die Molekulargewichte betragen etwa 20 000 bis 22 000. Die Aminosäure-Sequenz ist teilweise bekannt.

HuIFN-γ wird von T-Lymphocyten als Antwort auf Antigene oder Mitogene gebildet. Es ist bei pH 2 säurelabil und unterscheidet sich serologisch von HuIFN-α und HuIFN-β.

HuIFNe sind nützliche antivirale Mittel, Anti-Tumor- und Anti-Krebs-Mittel.

Als antivirale Mittel können sie zur Behandlung von viralen Infektionen des Atemtrakts, Herpes simplex-Keratitis, akuter haemorrhagischer Conjunctivitis, Varicella zoster, Hepatitus B, cytomegalischer Einschlußkrankheiten u.a. verwendet werden.

Als Anti-Tumor- oder Anti-Krebs-Mittel können HuIFNe zur Behandlung von beispielsweise Osteosarcoma, akuter Myeloidleukämie, multiplen Myeloma, Hodgkin'scher Krankheit, Melanoma, Brustkrebs, Lymphosarcoma und Papilloma und anderen verwendet werden.

HuIFNe können in Form pharmazeutischer Zubereitungen für die orale oder parenteral Verabreichung verwendet werden, z.B. als pharmazeutische Zubereitungen für die topische, intravenöse, intramuskuläre, intranasale, intradermale oder subkutane Verabreichung, beispielsweise in Form von Tabletten, Ampullen, Sirupen, Lösungen oder Suspensionen für die orale Verabreichung, Pulvern, Injektions- oder Infusionslösungen oder -suspensionen, Augentropfen, Salben, Sprays u.ä.

Die Zubereitungen werden im allgemeinen, beispielsweise intramuskulär, ein- bis dreimal täglich in Dosismengen von etwa $10^6$ bis etwa $10^7$ Einheiten verabreicht, wobei die Behandlung von der Krankheit, der Art der Anwendung und dem Zustand des Patienten abhängt. Virusinfektionen werden im allgemeinen täglich oder bis zu dreimal täglich während mehrerer Tage bis zu mehreren Wochen behandelt, wohingegen Tumore und Krebs während mehrere Monate oder Jahre entweder ein- bis mehrere Male täglich oder zwei- oder mehrere Male wöchentlich behandelt werden.

Bis heute kann man HuIFN-α nur in unzureichenden Mengen über induzierte Humanzellen, z.B. humane Lymphoblastoidzellen (z.B. aus Burkitt's Lymphoma-"Namalwa"-Zellen) oder humanen Leukocyten, die aus dem frischen Blut von Blutspendern erhalten werden, herstellen. HuIFN-β wird hauptsächlich aus humanen Fibroblasten erhalten. Es wurde in der Literatur beschrieben, daß nur $2{,}6 \times 10^9$ IE rohes HuIFN-α aus 800 l gezüchteten Namalwa-Zellen erhalten werden und daß nur etwa $10^{11}$ IE rohes HuIFN-α jährlich in sehr großen Blutzentren, z.B. dem Finnischen Roten Kreuz-Zentrum in Helsinki, erhalten werden können. Die spezifische Aktivität von HuIFN-α liegt in der Größenordnung von etwa $4 \times 10^8$ bis $10^9$ IE/mg. Die Menge en HuIFN-α, die für eine ausgedehnte und kommerzielle Anwendung erforderlich ist, wäre sehr niedrig, verglichen mit anderen pharmazeutischen Verbindungen.

100 g reines HuIFN-α würden 10 bis 30 Millionen Dosiseinheiten ergeben. Jedoch können solche Mengen unter Verwendung einer humanen Gewegekultur und der humanen Leukocyten-Technologie bei annehmbaren Kosten industriell nicht hergestellt werden.

Es ist ein weiterer Nachteil dieser Herstellungsverahren in großem Maßstab, daß nur Gemische aus Interferonen erhalten werden. Es ist schwierig und teuer, diese Gemische in die individuellen Subtypen aufzutrennen. Die therapeutischen Anwendungen von reinen, individuellen Interferon-Species konnten daher bislang noch nicht zufriedenstellend bestimmt werden.

Die industrielle Anwendung dieser Verfahren ist weiterhin auf HuIFNe, welche von humanen Zellen, die gezüchtet werden können (wie humane Tumorzellen und bestimmte Fibroblastzellen), oder auf humane Zellen, die in relativ grossen Mengen verfügbar sind (wie Leukocyten und Lymphocyten), beschränkt. Jedoch sind alle diese Verfahren teuer und kompliziert.

Man hat erkannt, dass die Lösung des Problems der industriellen Synthese grosser Mengen individueller Interferon-Species in den Fortschritten der Molekularbiologie liegen könnte, durch welche es ermöglicht wurde, ein spezifisches, nicht bakterielles, eukaryotisches Gen in Bakterienzellen auszudrücken. Kürzlich haven S. N. Cohen und H. W. Boyer (9) ein allgemeines Verfahren für die Replikation biologisch funktioneller DNA-Sequenzen beschrieben. Dieses Verfahren umfasst als Stufen die Spaltung von zirkulärer Plasmid-DNA, wobei man ein erstes lineares DNA-Segment erhält; das Einsetzen eines zweiten linearen DNA-Segments mit einem Gen für ein phenotypisches Merkmal in dieses erste Segment, wobei man ein rekombinantes DNA-Molekül erhält (ein modifiziertes, zirkuläres Plasmid); die Transformierung eines unizellulären Mikroorganismus mit diesem rekombinanten DNA-Molekül; das Züchten der Transformanten zusammen mit den nicht-transformierten Mikroorganismen bei geeigneten Nährbedingungen; und das Abtrennen der Transformanten von den unizellulären Stammikroorganismen. Die Transformanten können dann das gewünschte Protein produzieren.

Das Problem, eine für Interferon codierende lineare DNA-Sequenz und daraus eine rekominante DNA herzustellen, wurde von Cohen und Boyer nicht gelöst.

Beschreibung des Standes der Technik

In verschiedenen Patentanmeldungen und anderen Publikationen wurden DNA-Sequenzen, die sich von humanen Leukocyten und Fibroblasten, die für Polypeptide mit HuIFN-α-artiger Aktivität kodieren, ableiten, rekombinante DNA-Moleküle, die diese DNA-Sequenzen enthalten, Wirte, die mit diesen rekombinanten DNA-Molekülen transformiert wurden, Polypeptide oder Kulturflüssigkeiten, die solche Polypeptide enthalten, und Verfahren zur Herstellung dieser Stoffe beschrieben.

Unter Verwendung humaner Leukocyten, die mit Sendai-Virus induziert sind, als Ausgangsmaterial wurden verschiedene Polypeptide mit HuIFN-α-ähnlicher Aktivität, DNA-Sequenzen, rekombinante DNA-Moleküle und Wirte für ihre Herstellung von C. Weissmann beschrieben [(10); vergl. ebenfalls S. Nagata et al. (11), N. Mantei et al. (12) und M. Streuli et al. (13)].

Ein sich ebenfalls von Leukocyten, insbesondere von der mit Sendai-Virus induzierten humanen Myeloblastoidzell-Linie KG—1 ableitendes, teilweise gereinigtes HuIFN-α-artiges Polypeptid mit einem Molekulargewicht von 21 000, und die entsprechende DNA werden von D. V. Goeddel et al. (14) beschrieben.

Goeddel et al. (15) belegen, dass humanes Leukozyteninterferon eine Familie homologer, jedoch strukturell verschiedener Proteine darstellt und beschreiben acht spezifische Leukozyteninterferon cDNA-Klone sowie die von diesen kodierten Interferone (LeIF A—H).

Ein Verfahren zur Herstellung eines Polypeptids mit HuIFN-α-artiger Aktivität unter Verwendung von humanen Lymphoblastoid-Namalwa-Zellen oder humanen Blutleukocyten, die beide mit Newcastle-Disease-Virus induziert sind, wird in allgemeiner Form in der europäischen Patentanmeldung Nr. 34 307 (16) beschrieben, wobei jedoch keine konkreten Werte oder Einzelheiten aufgeführt werden.

Humanes Lymphoblastoid-Interferon "HuIFN(Ly)" kann in unterschiedlichen Mengen aus Namalwa-Zellen bei der Stimulierung mit verschiedenen Inducern [M. D. Johnston et al. (17)] hergestellt werden. Es wurde gezeigt, daß HuIFN(Ly) das aus Namalwa-Zellen, die durch Sendai-Virus induziert wurden, hergestellt wurde, HuIFN-α(Ly) (70—90%) und HuIFN-β(Ly) enthält. Es besteht aus mindestens sieben Komponenten [K. C. Zoon et al. (6); vergl. ebenfalls E. A. Havell et al. (2) und A. D. Sagar et al. (3)]. Obgleich die Gesamtstruktur der Lymphoblastoid-Interferon-Polypeptide derzeit noch unbekannt ist, ist es Offensichtlich, daß sich HuIFN-α(Ly) von HuIFN-α(Le) unterscheidet. Die Hauptkomponenten des Lymphoblastoid-Interferons scheinen nicht oder nur wenig glycosyliert zu sein. Die verschiedenen Komponenten wurden bischer noch nicht getrennt und gereinigt.

Gegenstand der Erfindung

Da klinische Versuche mit Gemischen aus Lymphoblastoid-HuIFNen durchgeführt wurden, ist es wünschenswert, die verschiedenen Komponenten zu trennen und sie individuell herzustellen, damit ihr therapeutisches Potential bestimmt werden kann. Keines der in der Literatur beschriebenen rekombinanten DNA-Verfahren bezieht sich auf die Synthese von humanen Lymphoblastoid-Interferonen. Der vorliegenden Erfindung liegt die Aufgabe zugrunde, dieses Problem mittels der rekombinanten DNA-Technologie zu lösen. Weiterhin soll die Struktur (Aminosäure-Sequenzen) der verschiedenen Subtypen von HuLyIFNen geklärt werden, und es sollen Verfahren zur Verfügung gestellt werden, die es erlauben, daß die individuellen Interferone in ausreichenden Mengen hergestellt werden können, um die Behandlung einer großen Anzahl möglicher Patienten sicherzustellen.

Gemäss vorliegender Erfindung kann ein individuelles Polypeptid, welches die biologische Aktivität von lymphoblastoiden Interferonen aufweist, hergestellt werden.

Die Erfindung betrifft weiterhin pharmazeutische Zubereitungen, die dieses Polypeptid enthalten, und seine Verwendung.

Detaillierte Beschreibung der Erfindung

Die Erfindung betrifft eine rekombinante DNA, welche eine für ein lymphoblastoides α-Interferon

kodierende DNA-Sequenz, ausgewählt aus der für HLyclFN-$5_1$ kodierenden DNA-Sequenz.

ATGGCCTTGACTTTTTATTTACTGGTGGCCC
TAGTGGTGCTCAGCTACAAGTCATTCAGCTCTCTGGGCTGTGATCTGCCTCAGACTCACAGCCTGGG
TAACAGGAGGGCCTTGATACTCCTGGCACAAATGCGAAGAATCTCTCCTTTCTCCTGCCTGAAGGAC
AGACATGACTTTGAATTCCCCCAGGAGGAGTTTGATGATAAACAGTTCCAGAAGGCTCAAGCCATCT
CTGTCCTCCATGAGATGATCCAGCAGACCTTCAACCTCTTCAGCACAAAGGACTCATCTGCTGCTTT
GGATGAGACCCTTCTAGATGAATTCTACATCGAACTTGACCAGCAGCTGAATGACCTGGAG
TCCTGTGTGATGCAGGAAGTGGGGGTGATAGAGTCTCCCCTGATGTACGAGGACTCCATCCTGGCTG
TGAGGAAATACTTCCAAAGAATCACTCTATATCTGACAGAGAAGAAATACAGCTCTTGTGCCTGGGA
GGTTGTCAGAGCAGAAATCATGAGATCCTTCTCTTTATCAATCAACTTGCAAAAAAGATTGAAGAGT
AAGGAA

und der für LyIFN-α-2 kodierenden DNA-Sequenz

ATGTGTGATCTGCCTC
AGACTCACAGCCTGGGTAACAGGAGGGCCTTGATACTCCTGGCACAAATGCGAAGAATCTCTCCTT
TCTCCTGCCTGAAGGACAGACATGACTTTGAATTCCCCCAGGAGGAGTTTGATGATAAACAGTTCC
AGAAGGCTCAAGCCATCTCTGTCCTCCATGAGATGATCCAGCAGACCTTCAACCTCTTCAGCACAA
AGGACTCATCTGCTGCTTTGGATGAGACCCTTCTAGATGAATTCTACATCGAACTTGACCAGCAGC
TGAATGACCTGGAGTCCTGTGTGATGCAGGAAGTGGGGGTGATAGAGTCTCCCCTGATGTACGAGG
ACTCCATCCTGGCTGTGAGGAAATACTTCCAAAGAATCACTCTATATCTGACAGAGAAGAAATACA
GCTCTTGTGCCTGGGAGGTTGTCAGAGCAGAAATCATGAGATCCTTCTCTTTATCAATCAACTTGC
AAAAAAGATTGAAGAGTAAGGAA.

enthält, einen mit mindestens einer der gennanten rekombinanten DNAs transformierten E. coli-Wirtsstamm, ein lymphoblastoids α-Interferon ausgewählt aus der Gruppe HLyclFN-$5_1$ der Formel

MET ALA LEU THR PHE TYR LEU LEU VAL ALA LEU VAL VAL LEU SER TYR LYS
SER PHE SER SER LEU GLY CYS ASP LEU PRO GLN THR HIS SER LEU GLY ASN
ARG ARG ALA LEU ILE LEU LEU ALA GLN MET ARG ARG ILE SER PRO PHE SER
CYS LEU LYS ASP ARG HIS ASP PHE GLU PHE PRO GLN GLU GLU PHE ASP ASP
LYS GLN PHE GLN LYS ALA GLN ALA ILE SER VAL LEU HIS GLU MET ILE GLN
GLN THR PHE ASN LEU PHE SER THR LYS ASP SER SER ALA ALA LEU ASP GLU
THR LEU LEU ASP GLU PHE TYR ILE GLU LEU ASP GLN GLN LEU ASN ASP LEU
GLU SER CYS VAL MET GLN GLU VAL GLY VAL ILE GLU SER PRO LEU MET TYR
GLU ASP SER ILE LEU ALA VAL ARG LYS TYR PHE GLN ARG ILE THR LEU TYR
LEU THR GLU LYS LYS TYR SER SER CYS ALA TRP GLU VAL VAL ARG ALA GLU
ILE MET ARG SER PHE SER LEU SER ILE ASN LEU GLN LYS ARG LEU LYS SER
LYS GLU.

und LyIFN-α-2, dadurch gekennzeichnet, dass es von einem transformierten E.-coli-Wirtsstamm, der eine rekombinante DNA enthaltend die DNA-Sequenz

ATGTGTGATCTGCCTC

AGACTCACAGCCTGGGTAACAGGAGGGCCTTGATACTCCTGGCACAAATGCGAAGAATCTCTCCTT

TCTCCTGCCTGAAGGACAGACATGACTTTGAATTCCCCCAGGAGGAGTTTGATGATAAACAGTTCC

AGAAGGCTCAAGCCATCTCTGTCCTCCATGAGATGATCCAGCAGACCTTCAACCTCTTCAGCACAA

AGGACTCATCTGCTGCTTTGGATGAGACCCTTCTAGATGAATTCTACATCGAACTTGACCAGCAGC

TGAATGACCTGGAGTCCTGTGTGATGCAGGAAGTGGGGGTGATAGAGTCTCCCCTGATGTACGAGG

ACTCCATCCTGGCTGTGAGGAAATACTTCCAAAGAATCACTCTATATCTGACAGAGAAGAAATACA

GCTCTTGTGCCTGGGAGGTTGTCAGAGCAGAAATCATGAGATCCTTCTCTTTATCAATCAACTTGC

AAAAAAGATTGAAGAGTAAGGAA.

trägt, hergestellt wird, ein pharmazeutisches Präparat, das besagtes Interferon enthält und besagtes Interferon zur Anwendung in einem Verfahren zur therapeutischen Behandlung des menschlichen Körpers.

Die erfindungsgemässen Lymphoblastoid-Interferone sind strukturell von den in der Literatur beschriebenen α-Interferonen, insbesondere von den 8 von Goeddel et al (15) beschriebenen α-Interferonen, verschieden und somit neu.

Die Erfindung betrifft weiterhin ein Verfahren zur Herstellung eines lymphoblastoiden α-Interferons ausgewählt aus der Gruppe HLycIFN-$5_1$ und LyIFN-α-2, dadurch gekennzeichnet, dass man einen mit mindestens einer der gennanten rekombinanten DNAs transformierten E. coli-Wirtsstamm züchtet und das Interferon isoliert.

Das Verfahren zur Herstellung eines transformierten E. coli-Wirtsstammes unfasst die Stufen

— Isolierung humaner Lymphoblastoid-Poly(A)-RNA aus induzierten humanen Lymphoblastoidzellen und ihre Anreicherung an HuLyIFN-mRNA,

— Herstellung einer einsträngigen, komplementären DNA aus diesem Template daraus einer doppelsträngigen cDNA,

— Einführung der ds cDNA in eine geeignete Vektor-DNA,

— Transformierung eines geeigneten E. coli-Wirtsstammes mit der erhaltenen rekombinaten DNA,

— Züchtung des Wirtsstammes und Auswahl der Klone, die mit rekombinanter DNA enthaltend eine für HLycIFN-$5_1$ oder LyIFN-α-2 kodierende DNA-Sequenz transformiert sind.

1. Induktion humaner Lymphoblastoidzellen und Isolierung humaner Lymphoblastoid-Poly(A)-RNA, angereichert an HuLyIFN mRNA

Die Isolierung humaner Lymphoblastoid-Poly(A)-RNA, angereichert an HuLyIFN mRNA, kann unter Verwendung verschiedener, an sich bekannter Verfahren erfolgen. Das bei der vorliegenden Erfindung verwendete Verfahren umfaßt die folgenden Stufen:

— Induktion humaner Lymphoblastoidzellen zur LyIFN-Synthese,

— Zerstörung der induzierten Zellen,

— Abtrennung der Lymphoblastoid-Poly(A)-RNA von den verunreinigenden Proteinen, Lipoproteinen, DNAs und RNAs anderert Art,

— Anreicherung an LyIFN-spezifischer mRNA.

Als Ergebnis der Einwirkung eines IFN-Inducers bilden humane Lymphoblastoidzellen LyIFN mRNA und anschließend humanes LyIFN.

Geeignete IFN-Inducer sind z.B. verschiedene chemische Mittel, eine doppelstrangige RNA, z.B. Poly(I):Poly(C), oder speziell bestimmte Viren, vor allen Mitglieder der Paramyxoviren, Pseudomyxoviren- und Reoviridial-Familien.

Als humane Lymphoblastoidzellen werden bevorzugt solche verwendet, die sich von Patienten mit Burkitt's Lymphoma herleiten. Es wurde gezeigt, daß eine solche Zellinie, Namalwa, hohe Gehalte an IFN bei der viralen Stimulierung erzeugt (18).

Die Induktion der Lymphoblastoidzellen erfolgt in an sich bekannter Weise gemäss den in der Literatur beschriebenen, analogen Verfahren. Die in geeignetem Kulturmedium gezüchteten Lymphoblastoidzellen werden zurch Zugabe geeigneter Viren während einer ausreichenden Zeit induziert. Der IFN Titer kann anhand der Messung der IFN Aktivität gemäss dem von Armstrong (19) entwickelten Farbstoffaufnahme-Verfahren bestimmt werden.

Zur Isolierung der Nucleinsäuren werden die induzierten Zellen durch mechanische Kräft (z.B. durch Homogenisieren, osmotischen Schock, Ultraschall) oder durch lytische, chemische Mittel (z.B. anionische Detergentien) zerstört. Das freigesetzte Nucleinsäuregemisch wird durch Zusatz von Proteasen (z.B. Pronase oder Protease P) und wiederholte Extraktion mit Phenol und Chloroform deproteinisiert.

Zur Reinigung von RNA kann die verunreinigende DNA mit Hilfe von RNAse-freier Desoxyribonuclease abgebaut werden, oder das Nucleinsäuregemisch kann zur Auftrennung von RNA und DNA einer Gleich-

gewichtszentrifugation in CsCl-Gradienten unterworfen werden. Aufgrund der Anwesenheit von Poly(A)-Ketten am 3'-Ende können die m-RNA Moleküle spezifisch über die Adsorption an Oligo(dT)-Cellulose oder Poly(U)-Sepharose von den anderen RNA-Species abgetrennt werden.

Zu diesem Zeitpunkt kann die Poly(A)-RNA auf ihre Kapazität, die Synthese von Polypeptiden, die HuIFN-Aktivität aufweisen, zu dirigieren, in einem in vitro-Translationssystem (z.B.: Reticulocyt-Translationssystem, Xenopus laevis Oocyten) geprüft werden. Die IFN-spezifischen Polypeptide können unter Verwendung eines Radio-Immunoassays oder, insbesondere, eines cytopathischen Bioassays gemäss Stewart et al. (20) identifiziert werden.

Die Anreichtung der Poly(A)-RNA-Fraktion an Lymphoblastoid-HuIFN-mRNA kann nach verschiedenen, an sich aus der Literatur bekannten Verfahren erfolgen. Sie beruht im wesentlichen auf den unterschiedlichen Molekulargrößen der einzelnen, in Frage kommenden mRNA-Species.

Die Fraktionierung der Poly(A)-RNA entsprechend der Größe kann beispielsweise durch Gelfiltration an Säulen aus Dextranderivaten oder Polyacrylamid erfolgen. Weiterhin kann ein Gemisch aus Poly(A)-RNA-Species durch Zonenelektrophorese in Polyacrylamid-, Stärke- oder Agarosegelen fraktioniert werden oder entsprechend der Sedimentationsgeschwindigkeiten der einzelnen RNA-Species durch Zonenzentrifugation in Saccharose-Dichtegradienten getrennt werden. Die einzelnen Fraktionen werden gesammelt und können auf ihre IFN mRNA-Aktivität analysiert werden. Die Fraktionen, die die höchste IFN mRNA-Aktivität zeigen, werden vereinigt und auf eine Oligo(dT)-Cellulose- oder eine Poly(U)-Sepharose-Säule aufgebracht. Die gebundene Poly(A)-RNA, die stark an HuLyIFN mRNA angereichtet ist, wird mit Wasser eluiert und mit Äthanol präzipitiert.

2. Herstellung von lymphoblastoider, doppelstrangiger, HuLyIFN ds cDNA enthaltender cDNA

Die Poly(A)-RNA, angereichert an HuLyIFN mRNA und wie oben beschrieben hergestellt, kann als Template zur Herstellung von doppelstrangiger cDNA verwendet werden. Diese Umwandlung umfaßt die Herstellung einer einstrangigen cDNA, die Synthese des zweiten DNA-Strangs und den Abbau der zuerst erzeugten, terminalen "Haarnadel"-Struktur.

Eine einstrangige DNA, die zu der Poly(A)-RNA komplementär ist, kann durch reverse Transkription der RNA hergestellt werden. Die Synthese wird durch eine RNA-abhängige DNA-Polymerase (reverse Transcriptase) katalysiert. Als Primer für den Ansatzpunkt der reversen Transcriptase auf dem RNA-Template kann Oligo-desoxythymidilat [Oligo(dT)] oder Poly(U) verwendet werden, wodurch eine doppelsträngige RNA im Bereich des 3'Poly(A)-Endes gebildet wird. Die Synthese der einstrangigen cDNA erfolgt in einem an sich bekannten Puffergemisch, wobei die Desoxynucleosid-triphosphate (dATP, dGTP, dCTP, dTTP) im Ueberschuss eingesetzt werden, um die Synthese vollständiger Kopien zu begünstigen. Nach Abstoppen der Synthese-Reaktion durch Zugabe eines Inhibierungsgemisches, das beispielsweise EDTA und SDS enthält, wird die cDNA deproteinisiert, die freien Desoxynucleosidtriphosphate werden über eine Sephadexsäule abgretrennt und die verunreinigende Template-RNA wird durch den Verdau mit Ribonuclease oder durch alkalische Hydrolyse entfernt. Die Länge der verbleibender cDNA kann beispielweise aus ihrer elektrophoretischen Mobilität in einem alkalischen Agarosegel oder in einem Polyacrylamidgel relativ zu Marker-DNAs bekannter Länge bestimmt werden (vergl. 35).

Die einstrangige cDNA, besitzt eine 3'-terminale "Haarnadel"-Struktur. Diese "Haarnadel"-Struktur stellt einen kurzen doppelsträngigen Bereich dar, der ein Selbst-"Priming" der cDNA bei der nachfolgenden Synthese des zweiten DNA-Strangs bewirkt. Somit ist kein zusätzlicher Primer erforderlich.

Die doppelsträngige cDNA kann durch eine RNA-abhängige DNA-Polymerase, z.B. reverse Transcriptase, auf eine Weise synthetisiert werden, die ähnlich ist, wie sie oben bei der Synthese der einstrangigen cDNA beschrieben wurde, mit der Ausnahme, daß Poly(A)-RNA durch einstrangige cDNA ersetzt und der Primer weggelassen wird. Alternativ können andere Enzyme, die die Synthese von DNA aus ihren Desoxyribonucleotid-Vorstufen katalysieren, verwendet werden, z.B. T4 DNA-Polymerise, *E. Coli*-DNA-Polymerase (Klenow-Fragment) oder, vorzugsweise, *E. coli*-DNA-Polymerase I.

Die erhaltene ds DNA enthält eine "Haarnadel"-Schleife, die die beiden DNA-Stränge verbindet. Die Schleife kann durch S1 Nuclease abgeschnitten werden, wobei man eine ds cDNA mit basengepaarten Enden erhält. Die ds cDNA wird anschliessend deproteinisiert, über eine Sephadexsäule gereinigt und mit Hilfe von Gelfiltration, Elektrophorese an Polyacrylamidgel oder an Agarosegel oder Zonenzentrifugation in einem Saccharosegradienten angereichert. Coelektrophorese und Cozentrifugation der DNA-Moleküle bekannter Molekülegröße in Parallelversuchen erlauben die Lokalisierung solcher ds cDNA-Moleküle, die die Molekulargröße besitzen, die für IFN ds cDNAs erwartet wird (700 bis 1200 Basenpaare, entsprechend den zuvor publizierten Daten: 15, 21, 22, 7).

3. Die Klonierung der an HuLyIFN ds cDNA angereicherten ds cDNA

Die Klonierung der wie oben beschrieben hergestellten ds cDNA kann nach an sich bekannten Verfahren erfolgen. Das Verfahren umfaßt

— die Verknüpfung der ds cDNA mit einer geeigneten Vektor-DNA und

— Uebertragung der entstandenen rekombinanten DNA in eine geeignete Wirtszelle (Transformation), in der sie sich vervielfältigen kann.

Eine Vektor-DNA enthält neben genetischen Funktionen, die sicherstellen, dass sie sich selbst vervielfältigt, wenn sie in eine Wirtszelle übertragen wird, sogenannte "Markergene", wie z.B. Antibiotika-Resistenzgene, die das Auffinden der transformierten Wirtszellen aus einer grossen Population von nicht-transformierten Wildtypzellen erlauben. Allgemein in der Gentechnologie verwendete Vektoren sind z.B. zirkulare Plasmid-DNAs (insbesondere das Plasmid col EI oder seine Derivate, z.B. pMB 9, pSF 2124, pBR 317 und insbesondere pBR 322) und die DNA bestimmter Bakteriophagen, an die ds cDNA experimentell gebunden werden kann, z.B. Derivate des Bakteriophagen λ.

Damit mat ein rekombinantes DNA-Molekül erhält, wird beispielsweise ein geeignetes Plasmid, z.B. pBR 322, mit Hilfe eines geeigneten Restriktionsenzyms gespalten, die ds cDNA wird in das linearisierte Plasmid eingefügt und der Ring wird erneut geschlossen, wobei ein vergrössertes rekombinantes Plasmidmolekül gebildet wird, welches das eingefügte ds cDNA-Segment enthält.

DNA-Segmente werden im allgemeinen über ihre einsträngigen, kohäsiven Enden verbunden und kovalent mittels einer DNA-Ligase, z.B. T4 DNA-Ligase, geschlossen. Komplementäre Enden können auf zwei unterschiedlichen Wegen gebildet werden: entweger durch Spaltung mit Restriktionsenzymen, welche gestaffelte Schnitte und kohäsive Termini bilden, oder durch die Addition definierter, einstrangiger Sequenzen (z.B. homopolymerer Enden). Alternativ können voll basengepaarte DNA-Duplexe, glatte Enden, mit T4-Ligase verbunden werden.

Nützliche Wirte sind beispielsweise Hefen und insbesondere Bakterien, die der Transformation zugänglich sind und keine Restriktionsenzyme und Modifizierungsenzyme besistzen, z.B. Stämme von *E. coli*, z.B. *E. coli* X 1776 oder *E. coli* HB 101, oder Stämme von *Bacillus subtilis, Bacillus stearothermophilus, Pseudomonas, Haemophilus, Streptococcus* und anderen Bakterien und ihre Mutanten.

Das rekombinante DNA-Molekül, hergestellt wie oben beschrieben, kann in eine geeignete Wirtszelle mittels üblicher Transformationsverfahren einschließlich einer $Ca^{2+}$-Vorbehandlung der Wirtszellen übertragen werden. Zellen, die eine rekombinante Plasmid-DNA enthalten, die eine phenotypische Eigenschaft, z.B. Tetracyclinresistenz, auf die Wirtszelle überträgt, werden selektiert, indem man in einem selektiven, beispielsweise Tetracyclin enthaltenden Nährmedium auf Agarplatten plattiert.

Bei der vorliegenden Erfindung ist die bevorzugte Vektor-DNA das Plasmid pBR 322, welches nach der Spaltung mit einer geeigneten Restriktionsendonuclease, insbesondere Pst I, an die ds cDNA via komplementäre homopolymere Enden, insbesondere dG:dC-Enden, gebunden wird. Die entstrehende rekombinante DNA wird in *E. coli* HB 101 übertragen.

Anstelle der IFN-Gene, die über den ds cDNA-Syntheseweg entsprechend den vorstehenden Kapiteln hergestellt wurden, kann auch die entsprechende chromosomale DNA für die Herstellung von Klonen verwendet werden, die Polypeptide mit IFN-Aktivität produzieren.

Der bevorzugte Vektor der vorliegenden Erfindung ist das Plasmid pBR 322. Es enthält zwei Resistenz-Gene ($amp^r$, $tet^r$) und einige einmalige ("unique") Restriktionsstellen. Eine einzige Pst I-Stelle ist innerhalb des $amp^r$-Gens, während die einzigen BamHI-, Hind III- und Sal I-Stellen innerhalb des $tet^r$-Gens auftreten. Eine einzige EcoR I-Stelle ist an anderer Stelle vorhanden (23). Bei Anwendung von einer der oben erwähnten Restriktionsendonucleasen bleibt entweder das $amp^r$-Gen oder das $tet^e$-Gen oder beide Gene intakt. Daher ist jedes der angeführten Enzyme für die Spaltung und die Linearisierung von pBR 322 geeignet.

Nach der Linearisierung des Plasmids pBR 322 können Desoxynucleotid-Ketten an beide 3'-Enden in Anwesenheit von terminaler Desoxynucleotidyl-Transferase angefügt werden. Bevorzugt werden etwa 20 bis 50 Desoxynucleotid-Reste angefügt, um eine stabile Verbindung an die ds cDNA, verlängert durch Ketten der komplementären Desoxynucleotide, sicherzustellen. Beispielsweise wird das Plasmid pBR 322 in einem geeignet gepufferten, wäßrigen Medium zusätzlich mit einer Restriktionsendonuclease, z.B. Pst I, behandelt. Nach Beendigung der Spaltung wird die Lösung mit beispielsweise Phenol deproteinisiert. Die terminale Addition von Desoxynucleotidresten erfolgt in einem üblichen Puffersystem mit einer ausreichenden Menge an Desoxynucleosid-triphosphaten, z.B. dGTP, und terminaler Desoxynucleotidyl-Transferase.

Die Verlängerung der wie oben beschrieben erhaltenen (Absatz 2) ds cDNA kann auf gleiche Weise erfolgen, wie es bei der Synthese des linearisierten, Desoxynucleotidverlängerten Plasmid pBR 322 beschrieben wurde, unter Verwendung des komplementären Desoxynucleosid-triphosphats (z.B. dCTP anstelle von dGTP).

Das linearisierte, kettenverlängerte Plasmid pBR 322 und die kettenverlängerte ds cDNA können hybridisiert und in an sich bekannter Weise, d.h. durch Basenpaarung der komplementären Desoxynucleotid-Ketten-Ketten, wieder zum Ring geschlossen werden.

Beispielsweise wird ein Gemisch aus Desoxynucleotid-verlängerter (z.B. dCMP-verlängerter) ds cDNA und linearisiertem, Desoxynucleotid-verlängertem (z.B. dGMP-verlängertem) pBR 322 bei vier aufeinanderfolgenden, einstündigen Stufen bei unterschiedlichen Temperaturen (z.B. 65°C, 46°C, 37°C und 20°C) inkubiert. Die hybridisierte, ringgeschlossene DNA kann direkt zur Transformation in ein kompatibles Bakterium z.B. *E. coli* HB 101, verwendet werden.

Die erhaltene Hybridplasmide können zur Transformation von *E. coli* HB 101 verwendet werden.

Die transformation von *E. coli* HB 101 mit den ringgeschlossenen Hybridplasmiden kann nach aus der Literatur bekannten Verfahren erfolgen. Das Verfahren umfaßt die $Ca^{2+}$-Vorbehandlung der Zellen, so daß eine DNA-Aufnahme möglich wird [z.B. (24)] und die Inkubation mit dem Hybridplasmid. Dann können die Zellen in ein selektives Wachstumsmedium übertragen werden, das die Abtrennung der transformierten Zellen von den Stammzellen erlaubt. Da das Hybridplasmid noch ein $tet^r$-Gen enthält, wird bevorzugt ein Agarmedium, das Tetracyclin als Wachstumsinhibierungssubstanz enthält, verwendet.

4. Identifizierung der Lymphoblastoid-IFN cDNA enthaltenden Klone

Kolonien, die spezifische Gene enthalten, können nach verschiedenen Verfahren identifiziert werden, z.B. durch RNA-Selektionshybridisierung, Differentialhybridisierung oder Hybridisierung mit einer synthetischen Sonde, oder Klone, die ein besonderes Genprodukt bilden, können durch immunologische oder biologische Assays identifiziert werden.

Während die immunologischen und biologischen Verfahren auf der Herstellung des immunologisch und biologisch nachweisbaren Genproduktes basieren, hängt der erste Satz von Verfahren hauptsächlich von der Verfügbarkeit einer geeigneten Sonde ab, die in ausreichend gereinigter Form vorliegt, um eine nichtspezifische Hybridbildung zu verhindern. Geeignete Sonden sind die mRNA, komplementär zu dem gewünschten Gen, oder die entsprechende cDNA.

Das in vorliegender Erfindung benutzte Screeningverfahren umfasst die Synthese eines 5'-terminal markierten Oligodesoxynucleotids, komplementär sowohl zu IFN-α als auch zu IFN-β mRNAs, die reverse Transkription von Poly(A)RNA, angereichert an LyIFN, mRNA, unter Verwendung des besagten Oligodesoxynucleotids als Primer, und die in situ-Koloniehybridisierung (25) der filtergebundenen Plasmid-DNAs mit einer markierten cDNA-Sonde.

Ein Vergleich der codierenden Bereiche der cDNAs von kloniertem, humanem IFN-α und IFN-β hat einen Bereich von 13 Nucleotiden gezeigt, der beiden cDNAs (und offensichtlich den entsprechenden mRNAs ebenso) gemeinsam ist (26) Ein entsprechendes synthetisches 13-meres Oligodesoxynucleotid der Formel

5'—CCTTCTGGAACTG—3'

kann daher verwendet werden, um die cDNA-Synthese aus humaner Lymphoblastoid-IFN-α und -β mRNA zu starten. Das Oligodesoxynucleotid kann nach an sich bekannten Verfahren (z.B. 27) hergestellt werden.

Die Markierung mit $^{32}P$ erfolgt nach ebenfalls an sich bekannten Verfahren.

Die an LyIFN mRNA angereicherte Poly(A)RNA (vergl. Stufe 1) wird als Template für die Synthese einer IFN-α und -β spezifischen cDNA-Sonde wie folgt verwendet: Die cDNA-Synthese erfolgt im wesentlichen auf gleiche Weise, wie oben (Stufe 2) beschreiben, mit der Ausnahme, daß das 5'-markierte, synthetische Oligodesoxynucleotid anstelle von Oligo(dT) als Primer eingesetzt wird. Das markierte cDNA-Produkt wird mit beispielweise Phenol deproteinisiert und durch Präzipitation mit Äthanol gesammelt. Eine weitere Reinigung kann beispielsweise durch Polyacrylamidgel-Elektrophorese des cDNA-Produkts erfolgen. Das Produkt kann autoradiographisch sichtbar gemacht werden.

Eine einzige DNA-Bande, die spezifisch für Poly(A)RNA aus induzierten Zellen ist und in dem Produkt, das man unter Verwendung von Poly(A)RNA aus nichtinduzierten Zellen erhält, nicht vorhanden ist, wird aus dem Gel extrahiert. Ihre Größe kann beispielsweise aus der relativen Mobilität, bezogen auf markierte Marker-DNAs bekannter Länge, bestimmt werden. Das Produkt stellt eine $^{32}P$-markierte, humane, LyIFN-α und -β spezifische cDNA-Sonde dar.

Kolonien, die auf einem Agarmedium, supplementiert mit Tetracyclin (vergl. Absatz 3) überleben und wachsen, werden mit dem in sit-Koloniehybridisierungsverfahren auf Klone geprüft, die Lymphoblastoid-IFN cDNA enthalten.

Die transformierten Kolonien werden auf Nitrocellulosefilter übertragen, und ein Vergleichssatz dieser Kolonien wird dann durch Replica-Plattieren auf zusätzliche Agarplatten erhalten. Die Kolonien auf den Filtern werden lysiert, ihre DNA wird denaturiert und auf die Filter in situ fixiert (25).

Vor dem Hybridisierungsverfahren wird die denaturierte DNA auf den Filtern bevorzugt mit einem Gemische prähybridisiert, das inter alia eine DNA fremder Provenienz enthält, um den Hintergrund niedrig zu erhalten und nichtspezifische Hybridisierungsstellen zu sättigen. Ananschliessen wird die oben beschriebene radioaktiv markierte cDNA-Sonde mit der am Filter gebundenen DNA hybridisiert. Nach dem Waschen der Filter kann das Ergebnis des Hybridisierungsverfahrens durch autoradiographische Analyse auf einem Röntgenfilm verfolgt werden. Kolonien, die auf dem Röntgenfilm positive ansprechen, können aus dem Vergleichssatz herausgepickt und für die weitere Untersuchung verwendet werden.

Bevorzugt wird nur ein Teil der transformierten Kolonien auf de Nitrocellulosefilter übertragen. Die verbleibenden Kolonien werden für weitere Screeningverfahren, die im folgenden erläutert werden, verwendet.

Die identifizierten Kolonien enthalten rekombinante DNAs mit Inserts, komplementär zu solchen der cDNA-Sonde, d.h. DNA-Segmente, die den humanen Lymphoblastoid-Genen oder ihren Fragmenten entsprechen. Da die primären Klone der transformierten Zellen gelengentlich mehr als eines Species rekombinanter DNA-Moleküle enthalten, werden die Hybridsplasmid-DNAs aus den positiv hybridisierten Klonen isoliert und zur Retransformierung von *E. coli* HB 101, wie zuvor beschrieben (Absatz 3), verwendet. Die Hybridsplasmid-DNAs können nach an sich bekannten Verfahren isoliert werden. Die Hybridplasmid-DNAs werden der Restriktionsanalyse unterworfen, und eine vollständige oder partiale Nucleotidsequenzanalyse des cDNA-Inserts wird nach an sich bekannten Verfahren durchgeführt, um Hybrid-DNAs auszuwählen, die für die weiteren Verfahren geeignet sind. Weiterhin kann eine partielle Sequenzanalyse klären, ob die eingefügten IFN cDNA-Segmente den humanen Lymphoblastoid-IFN-α- oder -β-Genen entsprechen.

Wie oben beschrieben, wird ein Teil der transformierten Kolonien auf Nitrocellulosefilter übertragen, und ihre fixierte DNA der in situ Koloniehybridisierung unter Verwendung einer radioaktiv. markierten cDNA als Sonde, z.B. einer IFN-spezifischen cDNA-Sonde, unterworfen. Die rekombinanten DNAs von positiv hybridisierenden Kolonien können als Screeningmaterial für zusätzliche Klone verwendet werden, die rekombinante DNA-Moleküle mit dem gleichen oder einem verwandten DNA-Insert (z.B. IFN-verwandte Sequenzen) enthalten.

5. Synthese von Polypeptiden mit HuLyIFN-artiger Aktivität durch E. coli, welche HuLyIFN-spezifische, rekombinante DNAs enthalten

Die erfindungsgemässen HuLyIFN-Inserts werden durch Hybridisierung in die Pst I-Stelle von pBR 322 (siehe oben) engesetzt. Da die Pst I-Stelle von pBR 322 innerhalb des β-Lactamasegens liegt, kann ein fusioniertes Protein entstehen, wenn der cDNA-Insert bezüglich der Transkription in der richtigen Orientierung und bezüglich der Translation in dem richtigen Leseraster eingefügt ist. Die IFN-Aktivität der Klone kann nach an sich bekannten Verfahren bestimmt werden. Im übrigen sind selbst jene Klone wertvoll, die keine IFN-Aktivität aufweisen, obgleich eine HuLyIFN cDNA vorhanden ist. In diesem Fall kann der cDNA-Insert isoliert und mit dem Expressionskontrollbereich auf geeignete Weise (siehe Absatz 7) verknüpft werden, um einem hohen Expressionsgrad an gewünschtem Polypeptid zu erhalten.

Klone, die Polypeptide mit HuLyIFN-Aktivität in zufriedenstellendem Ausmaß synthetisieren, sind für die Produktion in großem Maßstab geeignet. Die Klone können gezüchtet werden, und die Polypeptide können gemäß den Kapiteln 7 und 8 gewonnen werden.

6. Konstruktion von rekombinanten Plasmiden, welche hohe Werte an Polypeptiden mit HuLyIFN-Aktivität ausdrücken können

Damit eine ausreichende Expression stattfindet, muss ein Gen im Hinblick auf den Kontrollbereich einschliesslich des Initiators für die Transkription (Promoter) und für die Translation (ribosomale Bindungsstelle) in richtiger Weise eingeordnet werden.

Wie im Absatz 3 beschrieben, kann die rekombinante Plasmid-DNA eine HuLy cDNA enthalten, welche beispielsweise in die PstI Schnittstelle und damit innerhalb des β-Lactamase Gens von pBR 322 integriert ist. Wenn die Verknüpfung in der richtigen Orientierung und dem richtigen Leseraster erfolgte, kann ein fusioniertes Protein entstehen, das aus einem Teil der β-Lactamasekette gefolgt von der HuLyIFN-Aminosäuresequenz besteht.

Um die Expressionsausbeute des HuLyIFN cDNA-Inserts zu erhöhen, ist es erforderlich, den HuLyIFN cDNA-Insert in die Nähe der obenerwähnten Expressionskontrollsequenz einzuordnen, so das keine zusätzlichen Nucleotide (und damit Aminosäuren) den Gen vorhergehen (und somit dem Polypeptid mit HuLyIFN-Aktivität). Weiterhin sind im Falle von HuLyIFNen die primären Translationsprodukte Prä-Interferone, die Signalpeptide enthalten, die an den N-Terminus von "reifen" ("mature") Interferonen gebunden sind. Die Signalpeptidsequenzen werden post-translational in den ursprünglichen Progenitorzellen entfernt. Jedoch ist *E. coli* nicht imstande, die Präsequenzen proteolytisch zu entfernen. Daher werden die Präsequenzen bevorzugt aus dem cDNA-Insert mittels geeigneter Verfahren (siehe unten) entfernt, so daß das primäre Translationsprodukt ein "reifes" ("mature") IFN-artiges Polypeptid ist. Zu diesem Zweck wird das Gen, welches für das reife HuLyIFN codiert, in vitro rekonstituiert und erneut in ein Plasmid eingesetzt, nahe bei (operationsfähig verbunden mit) einer Expressionskontrollsequenz, z.B. der Expressionskontrollsequenz des β-Lactamase-Gens.

Da der "reife" cDNA-Insert, der für ein "reifes" HuLyIFN-artiges Polypeptid codiert, nicht mit dem Codon ATG beginnt, welches für die Initiierung der Translation erforderlich ist, muß das ATG-Triplett synthetisch eingebracht werden. Wenn man beispielsweise die Nucleotid-Basensequenzen und dementsprechend die Restriktionsendonucleasemuster sowohl von pBR 322 als auch von HuLyIFN cDNA kennt, kann man folgendermaßen vorgehen. Das Plasmid pBR 322 wird mit Pst I innerhalb des β-Lactamasegens gespalten und mit einer Exonuclease, z.B. Bal 31, digeriert, um die β-Lactamase-Codierungssequenz zu verkürzen. Das verkürzte Plasmid wird mit einem ds DNA-Linker verknüpft. Der Linker umfaßt die Erkennungssequenz einer geeigneten Restriktions-Endonuclease, z.B. Bcl I (Sau 3A). Das entstehende Plasmidfragment wird mit einer Restriktions-Endonuclease, die für den hybridisierten Linker (z.B. Bcl I) charakteristisch ist, und danach mit EcoR I (es gift eine EcoR I-Stelle innerhalb von pBR 322, die in der Nähe der β-Lactamase-Expressionskontrollsequenz lokalisiert ist) gespalten. Das entstehende DNA-Fragment, z.B. das EcoR — Bcl I DNA-Fragment, besteht im wesentlichen aus der Expressionskontrollsequenz der β-Lactamase (ApPr) und dem hybridisierten Linker und kann mittels Polyacrylamid-Elektrophorese isoliert werden.

Andererseits kann der HuLyIFN cDNA-Insert aus dem rekombinanten DNA-Molekül, welches diesen enthält, herausgeschnitten werden, z.B. durch Spalten mit der Restriktions-Endonuclease Pst I. Der isolierte HuLyIFN cDNA-Insert wird weiter mit einer anderen Restriktions-Endonuclease gespalten (oder, sofern erforderlich, mit zwei anderen Restriktions-Endonucleasen und teilweise wiederverknüpft), um die DNA-Sequenz zu entfernen, die für das Signalpeptid codiert. Die entstehende "reife" HuLyIFN cDNA besitzt ein gestaffeltes Ende, welches komplementär zu dem oben erwähnten ApBr DNA-Fragments ist (z.B. ein Saue 3A-Ende). Die "reife" HuLyIFN cDNA und die ApPr DNA-Fragmente werden mit Ligase, wie üblich, ligiert (vergl. Absatz 3). Die Hybridierung muss die Bildung eines ATG-Codons bewirken, das dem ersten Codon der "reifen" cDNA vorangeht, um das richtige Leseraster sicherzustellen. Die entstehende Hybrid-DNA enthält den β-Lactamase-Expressionskontrollbereich, ein ATG-Translations-Startcodon, eine DNA-Sequenz, die für das vollständige HuLyIFN codiert, und zwei Restriktions-Endonucleaseenden (z.B. EcoR I- und Pst I-Enden), die zur Einfügung der Hybrid-DNA in das entsprechend gespaltene Plasmid pBR 322 geeignet sind.

Das entstehende Hybridplasmid kann für die Transformierung von *E. coli* HB 101 und zur Steuerung der Synthese von hohen Gehalten an erfindungsgemässem Polypeptid mit HuLyIFN-Aktivität verwendet werden.

7. Kultivierung von HuLyIFN-spezifischen, rekombinante DNAs enthaltenden Klonen

Die erfindungsgemässen transformierten Wirte, die sich morphologisch von den zur Transformation verwendeten Ausgangsstämmen nicht unterscheiden, können zur Herstellung von erfindungsgemässen Polypeptiden mit HuLyIFN-Aktivität verwendet werden. Das Verfahren zur Herstellung dieser Polypeptide zeichnet sich dadurch aus, dass der transformierte *E. coli*-Stamm in einem flüssigen Nährmedium, das assimilierbare Quellen von Kohlenstoff und Stickstoff und anorganische Salze enthält, gezüchtet wird.

Verschiedene Kohlenstoffquellen können verwendet werden. Beispiele bevorzugter Kohlenstoffquellen sind assimilierbare Kohlenhydrate, wie Glucose, Maltose, Mannit oder Lactose, oder ein Acetat, welche entweder allein oder in geeigneten Gemischen verwendet werden können. Geeignete Stickstoffquellen sind beispielsweise Aminosäuren, wie Casaminosäuren, Peptide und Proteine und ihre Abbauprodukte, wie Trypton, Pepton oder Fleischextrakte; weiterhin Hefeextrakte, Malzextrakt, Maisquell-Flüssigkeit, wie auch Ammoniumsalze, z.B. Ammoniumchlorid, -sulfat oder -nitrat, die entweder allein oder in geeigneten Mischungen verwendet werden können. Anorganische Salze, die verwendet werden können, zind z.B. Sulfate, Chloride, Phosphate und Carbonate von Natrium, Kalium, Magnesium und Calcium.

Weiterhin kann das Nährmedium ebenfalls Substanzen zur Aktivierung des Wachstums wie z.B. Spurenelemente oder individuelle Aminosäuren und/oder Substanzen wie z.B. Antibiotika enthalten, die einen Selektionsdruck ausüben, um den Verlust an HuLyIFN-spezifisher, rekombinanter DNA zu verhindern.

Die Züchtung erfolgt unter Verwendung an sich bekannter Verfahren. Die Züchtungsbedingungen, wie die Temperatur, der pH-Wert des Mediums und die Fermentationszeit, werden auf solche Weise ausgewählt, daß maximale Gehalte an IFN-artigen Polypeptiden gebildet werden. Ein ausgewählter *E. coli*-Stamm wird bevorzugt bei aeroben Bedingungen in submerser Kultur unter Schütteln oder Rühren bei einer Temperatur von etwa 20 bis 40°C, vorzugsweise etwa 30°C, und einem pH-Wert von 4 bis 9, vorzugsweise bei pH 7, während etwa 4 bis 20 h, vorzugsweise 8 bis 12 h, gezüchtet. Als Ergebnis der Züchtung sammeln sich IFN-artige Polypeptide intrazellulär an.

8. Isolierung und Reinigung der Polypeptide mit IFN-Aktivität

Die erfindungsgemäßen humanen Lymphoblastoid-Interferone können aus der Kulturbrühe isoliert werden, indem man die Polypeptide aus den Zellen des transformierten Wirts freisetzt und sie reinigt.

Nachdem die transformierten *E. coli*-Zellen bis zu einer zufriedenstellenden Zelldichte gewachsen sind, besteht die erste Stufe bei der Gewinnung des zur Expression gebrachten Polypeptids in seiner Freisetzung aus dem Zellinneren. Zu diesem Zweck werden die Zellen durch Behandlung mit einem Detergens, wie SDS oder Triton, lysiert. Alternativ kann man mechanische Kräfte, wie Scherkräfte (z.B. eine X-Presse, French-Presse) oder Schütteln mit Glasperlen oder Aluminiumoxid, zum Aufbrechen der Zellen verwenden. Das entstehende Polypeptidgemisch kann an humanen LyIFN nach an sich bekannten Verfahren, wie Präzipitation mit Ammoniumsulfat oder Trichloressigsäure, Gelektophorese, Dialyse, Chromatographie, z.B. Ionenaustauschchromatographie, Größen-Exklusionschromatographie oder Umkehrphasen-HPLC und dergl., angereichert werden. Die Endreinigung des vorgereinigten Produktes kann beispielsweise mittels Antikörperaffinitäts-Chromatographie erfolgen. Im Prinzip können die Reinigungsstufen nach dem Verfahren von Staehelin et al. (28) durchgeführt werden, welches für die Reinigung von humanem Leukocyt-Interferon entwickelt wurde. Damit man ein ausreichend reines Produkt erhält, können zusätzliche Reinigungsstufen erforderlich sein, z.B. Kationen- oder Anionenaustauschchromatographie, Adsorption an Hydroxylapatit, Umkehrphasen-HPLC etc.

Die Erfindung betrifft insbesondere die rekombinanten DNAs und die Polypeptide der vorliegenden Erfindung in im wesentlichen reiner Form.

Die erfindungsgemässen Polypeptide werden in Analogie zu den bekannten Interferonen für die Behandlung viraler Infektionen, von Tumoren und Krebs des menschlichen Körpers, gegebenenfalls zusammen mit anderen antiviralen, Anti-Tumor- oder Anti-Krebs-Mitteln, bevorzugt in Form pharmazeutischer Präparate, die eine wirksame Menge des aktiven Bestandteils zusammen oder in Gemisch mit anorganischen oder organischen, festen oder flüssigen, pharmazeutisch annehmbaren Trägern enthalten, die für die parenteral Verabreichung geeignet sind, verwendet.

Die erfindungsgemaßen pharmakologisch aktiven Verbindungen werden bevorzugt in Form von Präparaten oder Infusionslösungen für die parenterale, z.B. intramuskuläre oder intravenöse, Verabreichung verwendet. Solche Lösungen sind bevorzugt isotonische, wäßrige Lösungen oder Suspensionen, die vor der Verwendung beispielsweise aus lyophilisierten Präparaten, die den aktiven Bestandteil allein oder zusammen mit einem pharmazeutisch annehmbaren Träger enthalten, hergestellt werden können. Die pharmazeutischen Präparate können sterilisiert werden oder sie können Adjuvantien, z.B. Konservierungsmittel, Stabilisatoren, Benetzungsmittel und/oder Emulgatoren, Solubilisierungsmittel, Salze zur Regulierung des osmotischen Drucks und/oder Puffer enthalten. Die erfindungsgemäßen pharmazeutischen Präparationen, die gegebenenfalls weitere pharmakologisch wertvolle Substanzen enthalten, werden in an sich bekannter Weise hergestellt, z.B. durch übliche Auflösungs- oder Lyophilisierungsverfahren, und sie enthalten engefähr 0,1 bis 100%, insbesondere ungefähr 1 bis ungefähr 50%, und im Falle der Lyophilisate bis zu 100% an aktivem Bestandteil.

Die Erfindung betrifft weiterhin ein Verfahren zur Herstellung eines pharmazeutischen Präparats, das dadurch gekennzeichnet ist, daß die erfindungsgemäße pharmakologisch aktive Verbindung mit einem pharmazeutisch annehmbaren Träger vermischt wird.

Abhängig von der Art der Krankheit und dem Zustand des Patienten, werden die Zubereitungen im allgemeinen einbis dreimal täglich in Dosiseinheiten von etwa $10^6$ bis etwa $10^7$ Einheiten verabreicht, z.B. intramuskulär.

Die folgenden Beispiele erläutern die Erfindung aber sollten nicht als eine Beschränkung ausgelegt werden.

## Beispiele

Die folgenden Abkürzungen werden in den Beispielen verwendet:

| | |
|---|---|
| EtBr | Äthidiumbromid |
| BSA | Rinderserumalbumin |
| DTT | 1,4-Dithiothreit (1,4-Dimercapto-2,3-butandiol) |
| EDTA | Äthylendiamin-tetraessigsäure |
| SDS | Natriumdodecylsulfat |
| TNE | Lösung, enthaltend 100 mM NaCl, 50 mM Tris.HCl (pH 7,5) und 5 mM EDTA |
| Tris(Trizma) | Tris-(hydroxymethyl)-aminomethan |
| Tris.HCl | Monohydrochlorid von Tris |

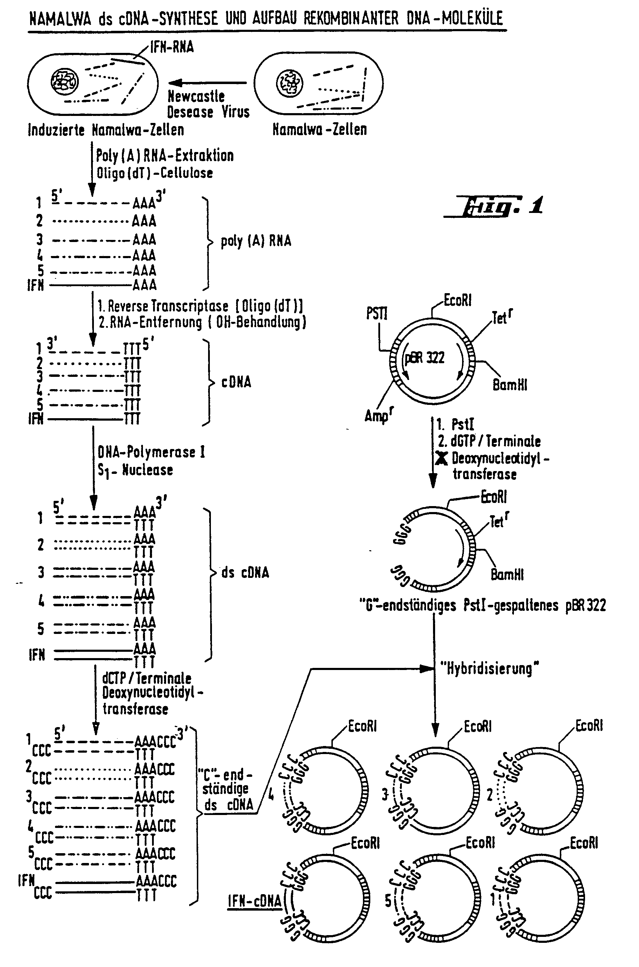

1. Isolierung von Poly(A) RNA, angereichert an HuIFN mRNA (Fig. 1)

a) *Induktion von Namalwa-Zellen*

Namalwa-Zellen werden in dem Kulturmedim RPMI 1640, das 10% fötales Kalbsserum enthält, bei 37°C gezüchtet. Nachdem eine Zelldichte von 3 × $10^6$ Zellen/ml erreicht ist, wird die Suspension bei 800 × g während 10 min bei Zimmertemperatur zentrifugiert. Die gesammelten Zellen werden erneut in 200 ml Kulturmedium suspendiert, welches Glutamin (0,027 Vol-%), Penicillin (200 Einheiten/ml) und Streptomycin (50 µg/ml) enthält. Die Zellen werden 90 min bei 37°C mit Newcastle Disease Virus (NDV 110) bei einem Verhältnis von 190 HAU/$10^6$ Zellen [HAU = Haemagglutinationseinheiten) inkubiert. Durch Zugabe von frischem Kulturmedium wird die Zelldichte auf 1,3 × $10^6$ Zellen/ml eingestellt und die Zellsuspension wird bei 34°C und 100 U/min geschüttelt. Nach 12 h werden 6 × $10^9$ Zellen geerntet und erneut in 50 ml Phosphatgepufferter Salzlösung suspendiert ("PBS"; 1 l PBS enthält 80 g NaCl, 2 g KCl, 14,4 g $Na_2HPO_4$ und 2 g $KH_2PO_4$). Vor dem Ernten der Zellen wird eine Probe entnommen, und die Interferonaktivität wird gemäß dem Verfahren von Armstrong (19) unter Verwendung humaner CCL-23-Zellen und des vesikularen Stomatitis-Virus (VSV) als Challenge-Virus bestimmt. Es werden 4300 IFN-Einheiten/ml gefunden.

b. *Zerostörung der Zellen und Deproteinisierung*

Die Zellsuspension (6 × $10^9$ Zellen in 50 ml PBS) wird bei Zimmertemperatur zu 800 ml Lysepuffer gegeben, der 0,05 M Tris.HCl (pH 7,5), 0,1 M NaCl, 5 mM EDTA und 2% SDS (kristalliner research grade, Serva) enthält. Das Lysat wird mit 0,2 mg/ml präinkubierter (2 h bei 37°C) Protease (Protease P, Typ VI, Sigma) 1 h bei Zimmertemperatur unter Rühren der Lösung digeriert. Die Lösung wird durch dreimaliges Extrahieren mit 500 ml Phenol, welches mit TNE gesättigt ist, und fünfmaliges Extrahieren mit 500 ml Chloroform deproteinisiert. Es werden 500 mg Nucleinsäuren erhalten, bestimmt durch die Absorption bei 260 nm.

c. *Entfernung der verunreinigenden DNA und RNA*

Die gemäß Stufe 1b erhaltene etwas viskose wäßrige Lösung wird auf 0,3 M NaCl eingestellt und mit 1 g Oligo(dT)-Cellulose (Typ 7, P—L Biochemicals) versetzt. Nach 30 minütigem Rühren bei Zimmertemperatur wird die Suspension in 1 l Sorvall-Flaschen in einer Sorvall RC—3 Zentrifuge 10 min bei 4000 U/min und bei Zimmertemperatur zentrifugiert, und die Oligo(dT)-Cellulose-Aufschlämmung wird zweimal mit 40 ml 2 × TNE, enthaltend 0,5% SDS, gewaschen. Die gebundene Poly(A) RNA wird dann durch fünf aufeinanforderfolgende Waschvorgänge mit 2,5 ml Wasser eluiert. Die Ausbeute beträgt 720 µg Poly(A) RNA, bestimmt durch Messung der optischen Dichte. Die überstehende RNA-Lösung aus der ersten Adsorption wird ein zweites Man an 1 g Oligo(dT)-Cellulose adsorbiert und, wie oben beschrieben, eluiert. Man erhält 320 µg Poly(A) RNA. Die Eluate werden vereinigt, auf TNE eingestellt und die Poly(A) RNA mit 67% Äthanol bei −20°C während 10 h präzipitiert. Die RNA wird durch Zentrifugieren bei 10 000 U/min in einer Sorvall RC-5B-Zentrifuge während 10 min bei 0°C gesammelt. Der Niederschlag (1 mg) wird erneut in 1 ml 1 mM EDTA gelöst.

Die HuIFN mRNA-Aktivität der RNA wird analysiert, indem man in Oocyten von Xenopus laevis wie folgt injiziert: 50 nl der RNA-Lösung werden in jede einzelne von 20 Oocyten injiziert. Die Oocyten werden in Barth-Medium (2 mM Tris, 88 mM NaCl, 1 mM KCl, 0,33 mM $Ca(NP_3)_2 \cdot H_2O$, 0,41 mM $CaCL_2 \cdot 2H_2O$, 0,82 mM $MgSO_4 \cdot 7H_2O$, 2,4 mM $NaHCO_3$, 0,01 mg/ml Pencillin, 0,01 mg/ml Streptomycin; der pH der Lösung wird mit HCl auf 7,6 eingestellt) gemäß Gurdon (29), Barth (30) und Colman et al (31) inkubiert. Die injizierten Oocyten werden 42 bis 48 h inkubiert und das Inkubationsmedium entfernt, dann wird in einer Eppendorf-Zentrifuge 5 min zentrifugiert und der Überstand wird bei −20 oder −80°C gelagert, bis er für die Analyse verwendet wird. Die IFN-Aktivität wird im wesentlichen entsprechend Armstrong (19) bestimmt, mit der Aenderung, daß VSV als Challenge-Virus auf Hep-2-Zellen (Flow Laboratories) verwendet wird. Der Oocytenextrakt besitzt eine spezifische Aktivität von 600 IE Interferon/µg injizierter RNA.

d. *Anreicherung der Poly(A) RNA an HuIFN mRNA*

Die Poly(A) RNA wird durch eine Chelex-100-Säule (200 bis 400 mesh, Bio-Rad) mit 0,5 ml Bettvolumen geleitet. Die Säule wird mit 1 ml 1 mM EDTA gespült.

Das Eluat [1 mg Poly(A) RNA in 2 ml EDTA] wird 2 min bei 100°C erhitzt und durch einen Saccharose-Dichtegradienten [sechs 14 ml Saccharoselösungen mit steigender Saccharosekonzentation von 5 bis 23% (M/V) und mit einem Gehalt an 50 mM Tris.HCl (pH 7,5), 0,2 M NaCl und 1 mM EDTA] zentrifugiert. Das Zentrifugieren erfolgt in einem TST 41 Rotor (Kontron AG) bei 35 000 U/min während 16 h bei 5°C. 0,3 ml-Fraktionen werden mit einem ISCO-Gradientenkollektor gesammelt. 2 Vol. Äthanol werden zu jeder Fraktion zugesetzt und die Lösung wird 10 h bei −20°C stehengelassen. Die ausgefallene mRNA wird durch Zentrifugieren (Sorvall, HB-4 Rotor bei 0°C, 10 000 U/min während 10 min) gesammelt. Der Niederschlag jeder Fraktion wird erneut in 25 µl 1 mM EDTA gelöst und jede Fraktion wird auf ihre Human-IFN mRNA-Aktivität, wie oben (Stufe 1c) beschrieben, analysiert, mit der Aenderung, daß nur zehn Oocyten pro RNA-Probe anstelle von zwanzig injiziert werden. Die Ergebnisse sind in Tabelle 1 aufgeführt.

TABELLE 1
HuIFN mRNA-Aktivität für die Fraktionen des Saccharose-Dichtegradienten

| Fraktion Nr. | IFN-Aktivität (Einheiten/ml) |
|---|---|
| 1—18 | — |
| 19 | 162 |
| 20 | 162 |
| 21 | 162 |
| 22 | 162 |
| 23 | nicht geprüft |
| 24 | 729 |
| 25 | Nicht geprüft |
| 26 | 405 |
| 27 | nicht geprüft |
| 28 | 486 |
| 29 | nicht geprüft |
| 30 | 162 |
| 31 | nicht geprüft |
| 32 | 162 |
| 33 | nicht geprüft |
| 34 | 54 |
| 34—40 | nicht geprüft |

Die Fraktionen 23 bis 29 werden vereinigt und die Poly(A) RNA weiter wie folgt gereinigt.

Die Poly(A) RNA-Lösung wird auf 2 × TNE in 0,5% SDS eingestellt und auf eine 200 µl Oligo(dT)-Cellulosesäule gegeben. Die Säule wird mit 2 ml von 2 × TNE in 0,5% SDS gewaschen und die Poly(A) RNA durch fünfmaliges Waschen mit 0,5 ml Wasser eluiert. Das Eluat wird auf TNE eingestellt und die Lösung zweimal mit einem gleichen Volumen Phenol (gesättigt in TNE) und zweimal mit dem gleichen Volumen Chloroform extrahiert. Die Poly(A) RNA wird mit 2 Vol. Äthanol bei −20°C während 10 h präzipitiert und durch Zentrifugieren in einem HB-4 Rotor, wie zuvor beschrieben, gesammelt.

Die Poly(A) RNA wird in 100 µl von 0,5 mM EDTA gelöst. Die Ausbeute beträgt 40 µg, bestimmt durch Messung der optischen Dichte.

Ein Teil der Poly(A) RNA wird auf ihre Human-IFN-Aktivität, wie oben beschrieben, unter Verwendung von 20 Oocyten/Assay analysiert. Die Poly(A) RNA-Präparation besitzt eine spezifische Aktivität von 8100 IE Interferon/µg RNA.

2. Herstellung der doppelsträngigen cDNA (Fig. 1)

An HuIFN mRNA angereicherte Poly(A) RNA (siehe Stufe 1d) wird als Templat zur Herstellung doppelsträngiger cDNA verwendet, wobei man im wesentlichen gemäß Efstratiadis et al. (32), Maniatis et al. (33) und Hoeijmakers et al (34) vorgeht.

a. *Synthese des ersten Strangs*

250 µl Reaktionsgemisch mit einem Gehalt an 40 mM Tris. HCl (pH 7,5), 30 mM NaCl, 5 mM $MgCl_2$, 0,5 mM DDT (Calbiochem), 1 mM dGTP, dCTP, dTTP (P-L Biochemicals) und 1 mM $^{32}$P-dATP (Amersham, spezifische Aktivität 50 000 cpm/nMol), 20 µg/ml Oligo$(dT)_{12-18}$ (P-L-Biochemicals), 40 µg/ml Poly(A) RNA und 100 Einheiten Vogelmyeloblastosis Virus (AMV)-reverse Transcriptase (Life Sciences, Inc., St. Petersburg, Florida) werden 80 min bei 37°C inkubiert. Die Reaktion wird beendigt, indem man die Lösung auf 10 mM EDTA und 0,1% SDS einstellt. Das Gemisch wird einmal mit 1 Vol Phenol extrahiert. Die wäßrige Phase wird erneut mit 1 Vol. Cloroform extrahiert und auf eine 3 ml Sephadex G—50 (Pharmacia, fein)-Säule gegeben. Es werden 0,1 ml-Fraktionen gesammelt. Die Radioaktivität jeder Fraktion wird durch Messung der Cerenkov-Radiation bestimmt. Radioaktive Fraktionen werden gesammelt und die Nucleinsäuren werden mit 2 Vol. Äthanol bei −20°C während 10 h präzipitiert. Die Probe wird in einem HB-4 Rotor 20 min bei 10 000 U/min bei 0°C zentrifugiert. Das Präzipitat wird in 95 µl Wasser gelöst. 5 µl 10N NaOH werden zugesetzt und die Mischung wird 40 min bei 25°C inkubiert. Nach der Neutralisation mit 5 M Essigsäure werden 50 µl Wasser und 2 Vol. Äthanol zugesetzt und die Probe wird 10 h bei −20°C gelagert. Das Präzipitat wird durch Zentrifugieren, wie zuvor beschrieben, gesammelt und erneut in 200 µl 0,1 mM EDTA gelöst. Die Ausbeute an einstrangiger cDNA beträgt 3,7 µg. Die Länge der cDNA beträgt 700 bis 1500 Nucleotide, bestimmt aus ihrer elektrophoretischen Mobilität in einem 6%igen Polyacrylamidgel in Tris-Borat-EDTA (108 g Tris, 9,3 g Dinatrium-EDTA und 55 g Borsäure pro 1 l Lösung bei pH 8,3), enthaltend 7 M Harnstoff, bezogen auf Marker-DNAs bekannter Länge (35).

b. *Synthese des zweiten Strangs und $S_1$-Endonuclease-Spaltung*

Die erhaltene cDNA-Lösung wird 90 sec bei 100°C erhitzt, abgekühlt und in einem 400 µl Reaktionsgemisch mit einem Gehalt an 0,1 M Kaliumphosphatpuffer (pH 6,9), 10 mM $MgCl_2$, 10 mM DTT (Calbiochem), 1 mM dATP, 1 mM dCTP, 1 mM dTTP (P-L, Biochemicals), 1 mM $^{3}$H-dGTP (Amersham, spezifische Aktivität 94 000 cpm/nMol) und 165 Einheiten/ml *E. coli* DNA-Polymerase I (Biolabs, New England) 8 h bei 15°C inkubiert. Die Reaktion wird beendet, indem man EDTA und SDS bis zu Endkonzentrationen von 10 mM bzw. 0,1% zugibt. Die Mischung wird mit Phenol und Chloroform extrahiert, über Sephadex G-50 (Pharmacia, fein, 2 ml Bettvolumen) chromatographiert und mit Äthanol, wie oben beschrieben (Stufe 2a), präzipitiert.

Die entstandene DNA wird 30 min bei 37°C in einer 50 µl Inkubationsmischung mit einem Gehalt an 0,25 M NaCl, 50 mM Natriumacetat (pH 4,5) und 1 mM $ZnSO_4$ mit sechs Einheiten $S_1$ Endonuclease (P-L Biochemicals) behandelt. Die Reaktion wird mit 0,1% SDS und 10 mM EDTA gestoppt. Das Reaktionsgemisch wird mit 1 Vol. Phenol (gesättigt in 50 mM Natriumacetat, pH 4,5) und Chloroform deproteinisiert. Die wäßrige Phase wird an einer 2 ml Sephadex G—50 (Pharmacia, fein)-Säule in TNE chromatographiert. 100 µl-Fraktionen werden gesammelt und die Cerenkov-Strahlung jeder Fraktion wird bestimmt. Die ausgeschlossenen Fraktionen werden vereinigt und die DNA wird mit 2 Vol. Äthanol bei −20°C während 10 h, wie oben beschrieben, präzipitiert. Das Präzipitat wird in einem HB-4 Rotor (siehe oben) zentrifugiert und der gesammelte Niederschlag in einer 100 µl Lösung mit einem Gehalt an 10 mM Tris.HCl (pH 7,5) und 0,5 mM EDTA gelöst. Man erhält 4 µg DNA.

Die DNA wird über einen Saccharose-Dichtegradienten (5 bis 23%) in 50 mM Tris-HCl (pH 7,5) und 1 mM EDTA in einem TST-60 Rotor (Kontron AG) fraktioniert. Das Zentrifugieren erfolgt bei 35 000 U/min während 5 h bei 15°C. Die DNA, die schneller als eine 800 Basenpaar-Marker DNA sedimentiert, die in einem Parallelgradienten geprüft wird, wird vereinigt, auf TNE eingestellt und mit 67% Äthanol bei −20°C während 10 h präziptiert. Man erhält 0,4 µg doppelstrangige cDNA.

3. Herstellung von pBR 322-verknüfter cDNA (Fig. 1)

a. *Herstellung von dCMP-verlängerter cDNA*

Die 3'-Termini von 0,1 µg der erhaltenen ds cDNA werden mit Poly(dC)-Enden in einem 10 µl Reaktionsvolumen versehen, das 100 mM Natriumcacodylat (pH 7,2), 2,5 mM $CoCl_2$, 50 µg BSA (Calbiochem.) pro ml, 1 mM dCTP und 10 Einheiten terminaler Desoxynucleotidyl-Transferase (P-L Biochemicals) pro µg ds cDNA enthält. Nach der Inkubierung (20 min bei 27°C) wird EDTA bis zu einem Gehalt von 10 mM zugegeben und die Probe bei −20°C bis zur Verwendung gelagert.

b. *Herstellung von Pst I gespaltenem, dGMP verlängertem pBR 322*

10 µg pBR 322 Plasmid DNA werden mit 10 Einheiten Pst I-Endonuclease (Biolabs) in einer 100 µl Lösung mit einem Gehalt an 50 mM NaCl, 6 mM Tris.HCl (pH 7,5), 6 mM $MgCl_2$, 6 mM 2-Mercaptoäthanol und 100 µg/ml Gelatine 1 h bei 37°C digeriert. Die Lösung wird mit 1 Vol. Phenol und Chloroform extrahiert. Die Lösung wird auf TNE eingestellt und die linearisierte DNA mit 2 Vol. Äthanol bei −20°C während 5 h präzipitiert.

Die linearisierte Plasmid-DNA wird mit dGMP in einem 200 µl Reaktionsvolumen verlängert, das 100 mM Natriumcacodylat (pH 7,2), 2 mM $MgCl_2$, 20 mM $NaH_2PO_4$, 50 µg BSA pro ml, 1 mM dGTP und 100 Einheiten terminale Desoxynucleotidyl-Transferase (P-L Biochemicals) enthält. Nach 20 minütiger Inkubation bei 37°C wird EDTA bis zu einem Gehalt von 10 mM zugesetzt und das Reaktionsgemisch bei −20°C bis zur Verwendung eingefroren.

c. *Hybridisierung von dGMP-verlängertem pBR 322 mit dCMP-verlängerter ds cDNA*

Ein Gemisch von dCMP-verlängerter, doppelsträngiger cDNA (0,1 µg) und dGMP-verlängertem, linearisiertem pBR 322 (0,5 µg) in 500 µl TNE-Puffer wird 1 h bei 65°C, 1 h bei 46°C, 1 h bei 37°C und 1 h bei 20°C inkubiert. Die die mit pBR 322 verknüpfe cDNA enthaltende Lösung wird auf Eis gegeben und unmittelbar für die Transformation verwendet.

4. Transformation von E. coli HB 101 mit dem Hybridsplasmid

Mit Calcium behandeltes *E. coli* HB 101 wird gemäß dem Verfahren von Mandel et al. (24) für die Transformation hergestellt.

10 µl des Reaktionsgemisches, das die, wie oben beschrieben (Stufe 3c), hergestellten pBR 322 Hybridsplasmid DNAs enthält, werden zu einem Gemisch gegeben, das 150 µl mit Calcium behandeltes *E. coli* HB 101 in 10 mM $MgCl_2$, 10 mM $CaCl_2$ und 10 mM Tris.HCl (pH 7,5) in einem Gesamtvolumen von 200 µl enthält.

Das Gemisch wird 20 min in Eis gekühlt, 1 min auf 42°C erhitzt und 10 min bei 20°C inkubiert. 1 ml Trypton-Medium [Trypton-Medium enthält 10 g Bacto-Trypton (Difco); 1 g Hefeextrakt (Difco); 1 g Glucose) 8 g NaCl und 294 mg $CaCl_2 \cdot 2H_2O$ in 1 l destilliertem Wasser] wird zugesetzt und das Gemisch 30 min bei 37°C unter Schütteln bei 300 U/min inkubiert. Die Mischung wird auf zwei Agarplatten (Mc Conkey Agar, Difco; 0,6 ml/Platte), supplementiert mit 10 µg/ml Tetracyclin (Sigma), plattiert. Die Platten werden 12 bis 17 h bei 37°C inkubiert. Es werden etwa 5600 Tetracyclin-resistente Kolonien von transformiertem *E. coli* HB 101 hergestellt.

5. Identifizierung der HuIFN cDNA enthaltenden Klone

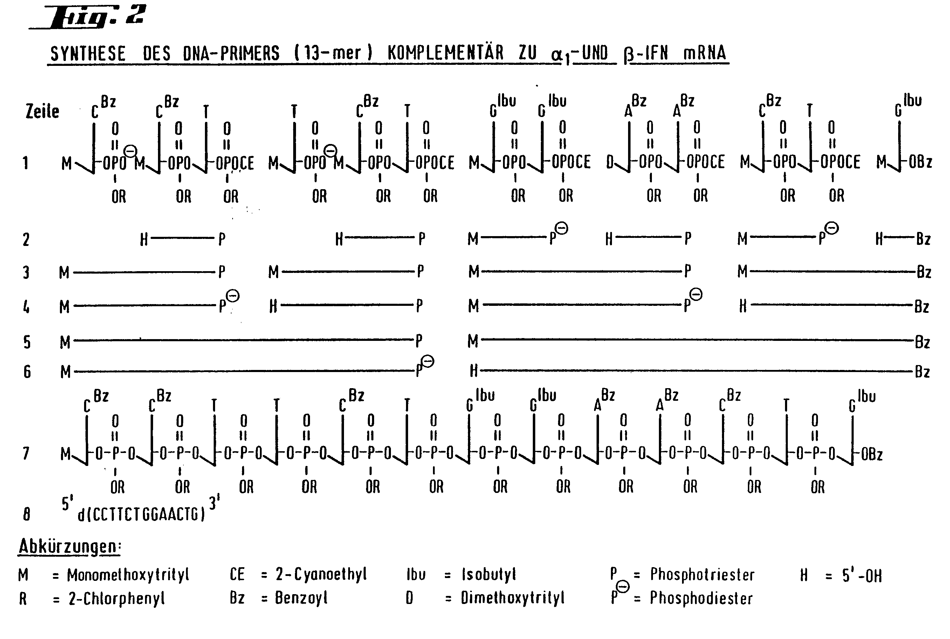

a. *Synthese des 13-meren Oligodesoxynucleotid-Primers (Fig. 2)*

Ein Oligodesoxynucleotid, welches komplementär zu einem Bereich von 13 Nucleotiden, der sowohl HuIFN-$\alpha_1$ als auch HuIFN-β mRNA gemeinsam ist, wird chemisch nach dem Phosphotriester-Verfahren (vergl. Itakura et al. (36), de Rooij et al. (37)] synthetisiert. Die einzelnen Stufen der Synthese sind in Fig. 2 dargestellt. Die in Zeile 1 der Fig. 2 dargestellten Ausgangsmaterialien (Schutzgruppen tragende Mono- und Didesoxynucleotide) sind aus der Literatur bekannt. Die Schutzgruppen werden gemäß den von Itakura et al. beschriebenen Verfahren abgespalten: Die Deblockierung von 5'-Monomethoxytrityl (M)- oder Dimethoxytrityl (D)-substituierten Hydroxylgruppen erfolgt mit Essigsäure (80%ig) bei Zimmertemperatur, und die β-Cyanoäthylphosphat-Gruppen werden mit 0,1N Natriumhydroxid in Dioxan-Wasser (4:1) bei Zimmertemperatur abgespalten. Die Kondensation der Baublöcke erfolgt unter Verwendung von Triisopropylbenzosulfonylchlorid als Aktivierungsmittel, wobei man Oligodesoxynucleotide bis zu dem vollständig geschützten 13-meren Primer, der in Zeile 7 der Fig. 2 dargestellt ist, erhält. Die letzte Stufe (vollständige Entfernung aller Schutzgruppen) wird wie folgt durchgeführt:

Eine 64,6 mg des vollständig geschützten 13-meren Oligodesoxynucleotids in 3 ml Dioxan und 1 ml Acetonitril enthaltende Lösung wird mit 200 mg syn-p-Nitrobenzaldoxim und 124 mg $N^1,N^1,N^3,N^3$-Tetramethylguanidin behandelt und 27 h stehengelassen. 10 ml Ammoniak (25%ig) werden zugefügt und die Lösung 24 h bei 50°C gelagert. Nach Verdampfen des Lösungsmittels im Vakuum wird der Rückstand in Wasser gelöst, mit Essigsäure auf pH 4 eingestellt und die Lösung 20 mal mit Chloroform extrahiert. Die wäßrige Lösung wird im Vakuum eingedampft und der Rückstand in 1 ml Essigsäure (80%ig) gelöst. Die Lösung wird 1 h stehengelassen, mit 6 ml Wasser verdünnt, dreimal mit Chloroform extrahiert und lyophilisiert. Ein Drittel des erhaltenen Produktes wird durch Chromatographie an DEAE-Sephadex A 25 (Säulengröße: 10 × 1,3 cm) durch einen 200 ml 0,2—1,2 M Triäthylammoniumbicarbonat-Gradienten gereinigt. Die Eluierung der Hauptfraktion tritt bei einer Gradientenkonzentration von 0,87 M auf. Die Hauptfraktion, die das reine Produkt enthält, was durch einen HPLC-Test erkennbar ist, wird dreimal mit Wasser eingedampft, durch 10 ml Dowex 50 W ($NH_4$-Salz) filtriert und lyophilisiert. HPLC (Permaphase AAX, Säulengröße 90 × 0,3 cm, 60°C, 2 ml/min; Gradient: A = 0,005 M $KH_2PO_4$, B = 0,5 M $KH_2PO_4$, 0,5 M KCl, pH 4,5; 20% A → 100% B in 30 min): $t_R$ 11,8 min.

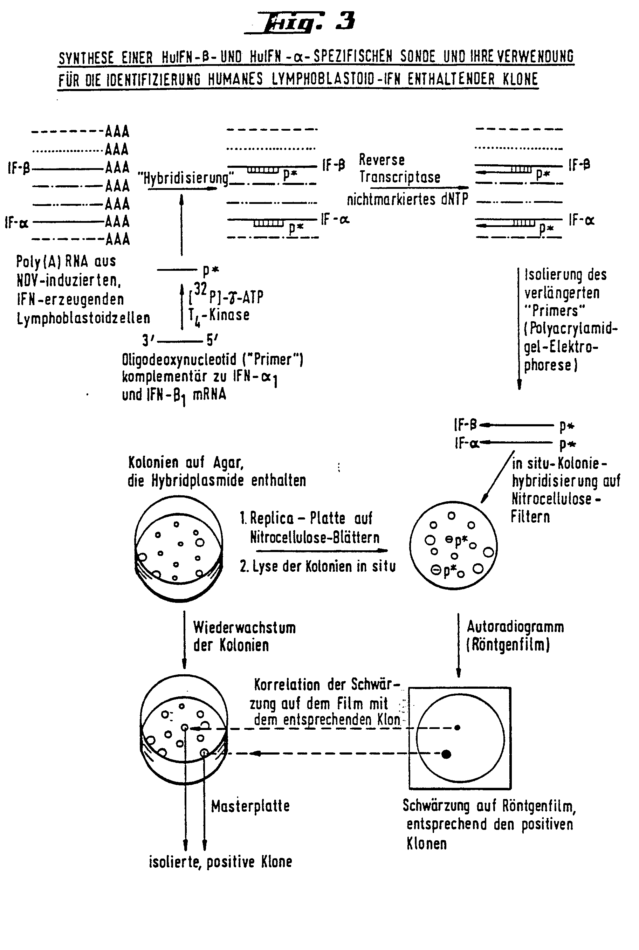

b. *Herstellung einer $^{32}$P-markierten, humanen IFN-α- und IFN-β spezifischen cDNA- Sonde (Fig. 3)*

40 pmol des synthetischen 13-meren Oligodesoxynucleotid-Primers (vergl. Stufe 5a) und 40 pmol [γ-$^{32}$P]-ATP (5700 Ci·mMol$^{-1}$, Amersham) werden in 100 µl 50 mM Tris.HCl (pH 9,5), 10 mM $MgCl_2$ und 5 mM DTT vereinigt. 50 Einheiten $T_4$ Polynucleotid-Kinase (P-L Biochemicals) werden zugesetzt und nach 30 min bei 37°C werden weitere 20 Einheiten des Enzyms zugesetzt. Die Inkubierung erfolgt während weiterer 15 min bei 37°C. Die den $^{32}$P-markierten Primer enthaltende wäßrige Lösung wird durch Phenolextraktion gereinigt. Eine weitere Reinigung erfolgt durch Chromatographie an einer 4 ml Sephadex G-50 (Pharmacia, fein)-Säule in 1 mM Tris.HCl (pH 8,0). Es werden 0,1 ml-Fraktionen gesammelt. Die Radioaktivität jeder Fraktion wird durch Messung der Cerenkov-Strahlung bestimmt. Eine spezifische Aktivität von 4 × $10^6$Cerenkov cpm/pmol Oligodesoxynucleotid wird erhalten. Der $^{32}$p-markierte Primer (40 mol) wird lyophilisiert, erneut in 91 µl Wasser, enthaltend 14 µg Poly(A) RNA (aus induzierten Namalwa-Zellen, hergestellt gemäss Stufe 1), suspendiert und 60 sec bei 100°C erhitzt. 9 µl 4 M KCl werden zugesetzt und das Gemisch wird 60 min bei 25°C inkubiert. 450 µl eines reverse Transcriptase-Gemisches werden so zugegeben, daß das Reaktionsvolumen 40 mM Tris.HCl (pH 8), 4 mM $MgCl_2$, 1 mM DTT (Calbiochem., Inc.), 74 mM KCl, 1 mM von jeweils dATP, dGTP, dCTP, dTTP (P-L-Biochemicals) und 90 Einheiten Vogelmyeloblastosis Virus (AMV)-reverse Transcriptase enthält. Die Inkubation wird 1 h bei 37°C weitergeführt. Die Lösung wird mit 1 Vol. Phenol (gesättigt in TNE) extrahiert und die Nucleinsäuren werden mit 2 Vol. Äthanol 10 h bei −20°C präzipitiert. Das Präzipitat wird durch Zentrifugation (HB-4 Rotor, 20 min, 10 000 U/min, 0°C) gesammelt und in 20 µl Farbstoffgemisch gelöst, das 90% (V/V) Formamid (Merck, pro Analyse), 1 mM EDTA, 0,05% Brom-Phenolblau und 0,05% Xylolcyanolblau enthält. Die Probe wird 2 min bei 90°C erhitzt und auf ein 5%iges Polyacrylamidgel in Tris-Borat-EDTA [vergl. Peacock et al. (35)] gegeben. Auf dem Autoradiogramm ist eine einzige Bande erkennbar, die zwischen den 267 bp- und 435 bp $^{32}$P-markierten Marker-DNA-Fragmenten, erhalten aus dem Hae III-Digest des Plasmids pBR 322, migriert. Das $^{32}$P-markierte cDNA-Fragment wird aus dem Gel extrahiert und gemäß Mueller et al. (38) gereinigt. Man erhält 20 000 Cerenkov cpm der $^{32}$P-markierten humanen IFN-α- und IFN-β spezifischen cDNA-Sonde.

c. *Screening nach HuIFN cDNA enthaltende Kolonien (Fig. 3)*

1650 Transformant-Kolonien, hergestellt gemäß Stufe 4, werden auf Nitrocellulose-Filter BA 85 (Schleicher & Schuell, 8 cm Durchmesser) übertragen. Die Zellen werden lysiert, ihre DNA denaturiert und in situ an die Filter gemäß Grunstein und Hogness (25) fixiert. Die die Kolonien tragenden Filter werden in 4 × SET [eine Lösung mit einem Gehalt an 0,15 M NaCl, 30 mM Tris.HCl (pH 8,0), 1 mM EDTA], 0,1% (Gew./Vol) Ficoll 400 (Pharmacia), 0,1% (Gew./Vol) Polyvinylpyrrolidon (PVP-360, Sigma), 0,1% (Vol/Vol) BSA, 0,5% SDS, 50 µg/ml denaturierte Kalbsthymus-DNA [hergestellt wie folgt: 5 mg Kalbsthymus-DNA (Typ I, Sigma) werden 10 min in 0,5 M NaOH zum Scheren der DNA gekocht, mit 5 M Essigsäure neutralisiert und mit 2 Vol. Äthanol bei −20°C präzipitiert] prähybridisiert. Das Präzipitat wird durch Zentrifugieren in einem HB-4-Rotor während 10 min bei 0°C gesammelt und erneut in 500 µl 0,5 mM EDTA bei 65°C während 4 h unter Verwendung von 20 ml Gemische/Filter gelöst und mit $10^3$ Cerenkov cpm der $^{32}$P-markierten Probe pro Nitrocellulose-Filter in 5 × SET, 0,02% (Gew./Vol) Ficoll, 0,01% Polyvinylpyrrolidon, 0,02% (Vol/Vol) BSA, 0,2% SDS und 50 µg/ml denaturierte Kalbsthymus-DNA hybridisiert. Die Hybridisierung wird 36 h bei 65°C durchgeführt.

Die Filter werden einmal in Chloroform, zweimal in SET 0,5% SDS bei Zimmertemperatur und zweimal in SET, 0,5% SDS während 1 h bei 60°C und einmal mit 3 mM Trizma-Base 1 h bei Zimmertemperatur gespült. Die Filter werden getrocknet, indem man sie auf 3MM-Papier (Whatman) legt, und ein Röntgenfilm (Fuji) wird den Filtern unter Verwendung eines Filmfilters (Ilford Verstärkungsfilters) bei 80°C während 72 h ausgesetzt.

Auf dem Autoradiogramm werden neun positive Kolonien identifiziert und diese werden für die weitere Untersuchung verwendet.

Da die primären Klone von transformierten Zellen gelegentlich mehr als eine Species rekombinanter DNA-Moleküle enthalten, werden die Hybridplasmid-DNAs aus den neun positiv hybridisierten Klonen isoliert und zur Retransformierung von *E. coli* HB 101, wie oben beschrieben, verwendet.

Die Hybridplasmid-DNA wird wie folgt isoliert: 1 Kolonie wird zur Inokulierung von 10 ml Trypton-Medium, supplementiert mit 10 µg/ml Tetracyclin, wie oben, in einem 25 ml Erlenmeyerkolben verwendet. Die Kultur wird 15 bis 18 h bei 37°C und 300 U/min geschüttelt. Die Zellen werden durch Zentrifugieren (Sorvall, HS-4 Rotor, 10 min bei 4000 U/min, 4°C) geerntet. Man erhält etwa 0,1 g Zellen, und diese werden in 1 ml 50 mM Tris.HCl (pH 8,0) resuspendiert. 0,25 ml Lysozymlösung [10 mg/ml in 50 mM Tris.HCl (pH 8,0); Lysozym wird von Sigma vertrieben] werden zugesetzt und nach 10 minütiger Inkubation bei 0°C werden 0,15 ml 0,5 M EDTA (pH 7,5) zugegeben. Nach weiteren 10 min bei 0°C werden 60 µl 2%iges Triton X-100 (Merck) zugegeben. Nach 30 min bei 0°C wird die Probe 30 min bei 15 000 U/min und 4°C in einem Sorvall SA-600 Rotor zentrifugiert. Der Überstand wird mit 1 Vol. Phenol (gesättigt in TNE) deproteinisiert. Die Phasen werden durch Zentrifugieren (Sorvall HB-4 Rotor) während 10 min bei 5000 U/min und 4°C getrennt. Die obere Phase wird zweimal mit 1 Vol. Chloroform extrahiert. Pankreatische RNAse A (Sigma; 10 mg/ml in TNE 10 min bei 85°C vorerhitzt) wird bis zu einer Endkonzentration von 25 µg/ml zugegeben und das Gemisch 40 min bei 37°C inkubiert. Die Lösung wird dann auf 1 M NaCl und 10% Polyäthylenglykol 6000 (Fluka, 20 min bei 120°C in Autoklaven behandelt) eingestellt und 2 h bei −10°C inkubiert. Das Präzipitat wird in einem Sorvall HB-4 Rotor (20 min bei 10 000 U/min, 0°C) gesammelt und erneut in 100 µl TNE gelöst. Die DNA-Lösung wird mit 1 Vol. Phenol extrahiert und die DNA wird mit 2 Vol. Äthanol 10 min bei −80°C präzipitiert. Das Präzipitat wird durch Zentrifugieren in einer Eppendorf-Zentrifuge gesammelt und die DNA erneut in 20 µl 10 mM Tris.HCl (pH 7,5) und 0,5 mM EDTA gelöst. Aus einer 10 ml Kultur gewinnt man 8 bis 10 µg Hybridplasmid-DNA.

*E. coli* HB 101 wird mit jeder der neun isolierten Hybrid-DNAs transformiert, und die transformierten Zellen werden auf Agarplatten, die Tetracyclin enthalten, wie zuvor beschrieben (Stufe 4), plattiert. Aus jeder Transformation werden drei Tetracyclin-resistente Klone herausgepickt; es werden 10 ml-Kulturen hergestellt und die Hybrid-DNAs werden aus den Kulturen, wie zuvor beschrieben, isoliert.

Alle DNA-Proben vor und nach der Retransformation werden durch Spaltung mit Pst I-Endonuclease und Elektrophorese durch ein 1%iges Agarosegel in 50 mM Tris-Acetat (pH 7,8) und 1 mM EDTA analysiert. Alle Proben zeigen identische Spaltungsmuster vor und nach der Retransformation.

Eines der reklonierten rekombinanten DNA-Moleküle ergibt zwei Banden, von denen eine die Mobilität von Pst I-gespaltenem pBR 322 und die andere die Mobilität entsprechend etwa 1000 bp zeigt. Sie wird als CG-pBR 322/HLycIFN-1'b bezeichnet.

Eine andere rekombinante DNA ergibt drei Banden, wovon eine der Mobilität von Pst I-gespaltenem pBR 322, eine der Mobilität von etwa 600 bp und eine der Mobilität von etwa 150 bp entspricht. Die rekombinanten DNA-Moleküle in diesem Klon werden als CG-pBR 322/HLycIFN-$\beta_1$ bezeichnet.

d. *Charakterisierung der Klone CG-pBR 322/HLycIFN-1'b und CG-pBR 322/HLycIFN-*$\beta_1$

Die rekombinanten Plasmid-DNAs der Klone CG-pBR 322/HLycIFN-1'b und CG-pBR 322/HLycIFN-$\beta_1$ werden aus den Kulturen, wie oben beschrieben (Stufe 5c), isoliert und charakterisiert, indem man die Nucleotid-Sequenz des cDNA-Inserts unter Verwendung des von Maxam und Gilbert (39) beschriebenen Verfahrens etabliert. Grundsätzlich wird das folgende Verfahren verwendet.

Die isolierte, rekombinante Plasmid-DNA wird mit verschiedenen Restriktionsendonucleasen digeriert. Die Enzyme werden im wesentlichen nach den Angaben des Herstellers (New England Biolabs) angewendet, mit der Aenderung, dass BSA durch Gelatine in den Enzympuffern ersetzt wird. Die Lösung, welche die der Restriktion unterworfene DNA enthält, wird mit Phenol (gesättigt mit TNE) deproteinisiert. Die DNA wird mit Aethanol präzipitiert, erneut in 50 mM Tris-HCl (pH 8,0) bei einer DNA-Konzentration von 50 µg/ml gelöst und mit 0,1 Einheiten intestinaler alkalischer Kalb-Phosphatase (Boehringer) pro pmol DNA-5'-Enden während 30 min bei 37°C inkubiert. Das Enzym wird durch Erhitzen der Lösung während 60 min bei 65°C inaktiviert. Die DNA wird durch DEAE-Cellulose-Chromatographie gemäß Mueller et al. (38) gereinigt und mit Äthanol präzipitiert. Die DNA wird dann 5'-terminal mit [$\gamma$-$^{32}$P]-ATP (>5000 Ci/mmol, Amersham) und T4 Polynucleotid-Kinase (P-L Biochemicals) im wesentlichen gemäß dem von Maxam und Gilbert (39) beschriebenen Verfahren markiert, mit der Aenderung, daß die DNA vor der Kinasereaktion nicht denaturiert wird. Im allgemeinen betragen die spezifischen Aktivitäten 1 bis 3 × $10^6$ cpm/pmol 5'-Enden.

Die markierten DNA-Fragmente werden mit einer zweiten Restriktionsendonuclease gespalten und die Produkte werden durch Elektrophorese durch ein 6%, 8% oder 10% Polyacrylamidgel in Tris-Borat-EDTA-Puffer getrennt. Die DNA-Fragmente werden aus dem Gel extrahiert und gemäß Mueller et al. (38) gereinigt. Für die Bestimmung der Nucleotidsequenzen werden die DNA-Fragmente chemisch abgebaut und die Produkte durch Polyacrylamidgel-Elektrophorese gemäß Maxam und Gilbert (39) getrennt.

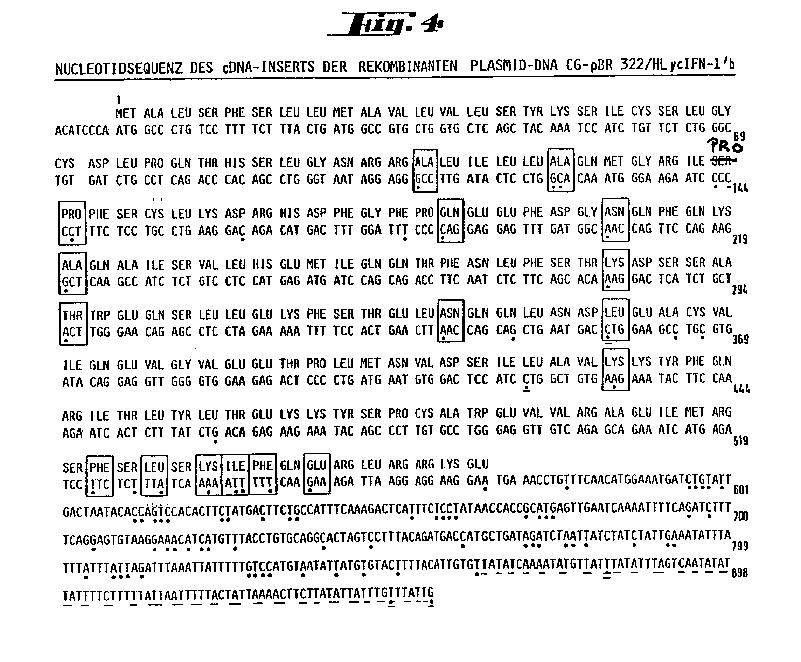

Insbesondere werden die isolierten Plasmid-DNAs des Klons CG-pBR 322/HLycIFN-1'b wie folgt behandelt. Einerseits werden 5 µg der Plasmid-DNA mit Bgl II digeriert, 5'-terminal markiert und mit Pvu II gespalten. Die Pvu II-Bgl II$^+$-(+ bezeichnet die markierte Stelle) und Bgl II-Pvu II$^+$ DNA-Fragmente werden an einem 6%igen Polyacrylamidgel isoliert. Andererseits werden 5 µg des Plasmids mit Alu I digeriert, 5'-terminal markiert und mit Pst I gespalten. Das Pst I-Alu I$^+$ DNA-Fragment wird an einem 8%igen Polyacrylamidgel isoliert. Die einzelnen Fragmente werden nacheinander abgebaut und dann wird gemäß Maxam und Gilbert die Sequenz bestimmt. Die erhaltene Nucleotidsequenz ist in Fig. 4 dargestellt. Ein Bereich von etwa 25 bis 35 Desoxyguanosin-Resten geht dem 5'-Ende des cDNA-Inserts voraus. Die gezeigte Nucleotidsequenz ist ähnlich der von IFN-α(Typ F) cDNA, beschrieben von Goeddel et al. [(15); vergl. auch Weissmann (10)], zeigt jedoch viele deutliche Abweichungen (Punkt-Mutationen), von denen einige die resultierenden Aminosäuren beeinflussen (siehe Fig. 4).

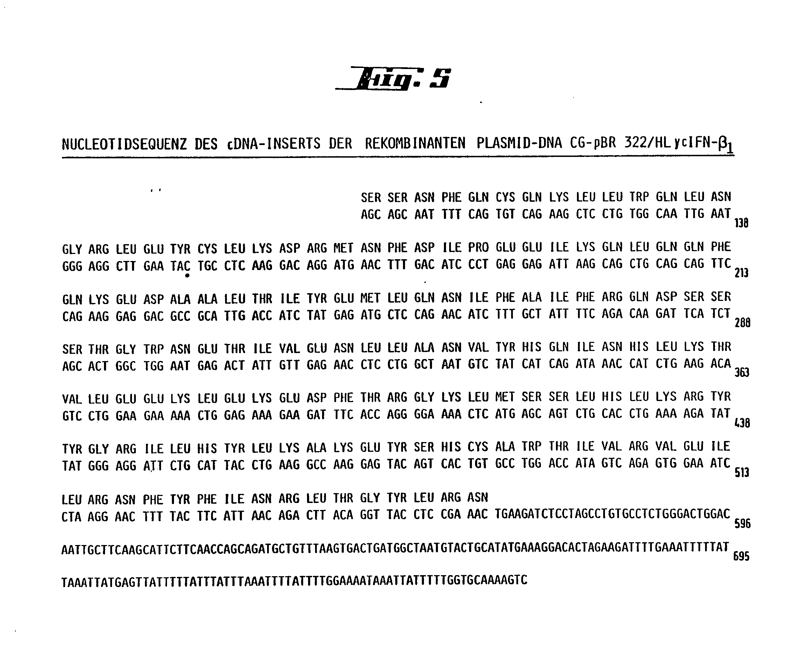

Die isolierte Plasmid-DNA des Klons CG-pBR 322/HLycIFN-$\beta_1$ wird auf ähnliche Weise behandelt. 5 µg des Plasmids werden mit Pvu II digeriert und 5'-terminal markiert. Eine Hälfte der Mischung wird mit Pst I gespalten und der Rest mit Bgl II. Die Pst I-Pvu II$^+$- und Bgl II-Pvu II$^+$-Fragmente werden durch Elektrophorese an einem 6%igen Polyacrylamidgel isoliert und, wie oben beschrieben, abgebaut. Die Nucleotidsequenz (N-terminale Sequenz) ist aus Fig. 5 ersichtlich und zeigt, daß der cDNA- Insert bei der Nucleötidzahl 102 der IFN-$\beta_1$ cDNA, wie von Taniguchi et al. (40) beschrieben, beginnt. Daher besitzt der cDNA-Insert die Kapazität, für humanes IFN-$\beta_1$, dem 11 Aminosäuren am N-Terminus fehlen, zu codieren. Der cDNA-Insert wird an seinem 5'-Ende durch einen Bereich von etwa 20 bis 25 Desoxyguanosin-Resten flankiert und zeigt eine Punktmutation in der Stellung 153, wo ein C- zu einem T-Rest umgewandelt wurde, ohne daß die entsprechende Aminosäure beeinflußt wird.

e. *Identifizierung der Klone, die rekombinante DNA-Moleküle enthalten, welche mit den Inserts von CG-pBR 322/HLycIFN-1'b und CG-pBR 322/HLycIFN-$\beta_1$ kreuzhybridisieren*

Die rekombinanten Plasmid-DNAs der Klone CG-pBR 322/HLycIFN-1'b und CG-pBR 322/HLycIFN-$\beta_1$ werden aus den Kulturen gemäß Stufe 5C isoliert. Die CG-pBR 322/HLycIFN-1'b-Plasmid-DNA (5 µg) wird mit Bgl II digeriert, 5'-terminal markiert und mit Pvu II gespalten. Andererseits wird die isolierte CG-pBR 322/HLycIFN-$\beta_1$ Plasmid-DNA (5 µg) mit Pvu II digeriert, 5'-terminal markiert und mit Bgl II gespalten. Das Pvu II-Bgl II$^+$(351 bp) DNA-Fragment (Sonde A) und das Pvu II$^+$-Bgl II (368 bp) DNA-Fragment (Sonde B) werden aus einem 8%igen Polyacrylamidgel gemäß Stufe 5d isoliert und für die in situ-Koloniehybridisierung (vergl. unten) verwendet. Die Restriktion der Plasmid-DNAs die Markierung und die Reinigung der DNA-Fragmente erfolgen auf gleiche Weise wie bei Stufe 5d oben beschrieben.

4000 der gemäß obiger Stufe 4 hergestellten Transformantkolonien werden auf Nitrocellulose-Filter BA 85 (Schleicher & Schuell, 8 cm Durchmesser) übertragen. Die Zellen werden lysiert, ihre DNA wird denaturiert und an die Filter in situ gemäß Grunstein und Hogness (25) fixiert. Die Hybridisierung der Sonden A und B (beide Proben sind vermischt) erfolgt gemäß Stufe 5c. Durch Autoradiographie werden sechs positive Kolonien identifiziert, von denen drei, bezeichnet als

*E. coli* HB 101 CG-pBR 322/HLycIFN-$4_1$.
*E. coli* HB 101 CG-pBR 322/HLycIFN-$5_1$ und
*E. coli* HB 101 CG-pBR 322/HLycIFN-$8'_1$

für die weitere Untersuchung verwendet werden. Die Plasmid-DNAs dieser Klone werden isoliert, retransformiert und re-isoliert, wie in den obigen Stufen 5c und 5d beschrieben.

Zur Etablierung der Natur der Inserts der rekombinanten DNAs werden die Nucleotidsequenzen der cDNA-Inserts (teilweise oder vollständig) unter Verwendung des in obiger Stufe 5d beschriebenen Verfahrens bestimmt.

Im einzelnen werden jeweils 5 µg der isolierten Plasmid-DNAs CG-pBR 322/HLycIFN-$4_1$ und CG-pBR 322/HLycIFN-$8'_1$ mit Pvu II digeriert, 5'-terminal markiert und mit Pst I gespalten. Die DNA-Fragmente werden an einem 8%igen Polyacrylamidgel fraktioniert und Pst I-Pvu II$^+$ (~120 bp) aus $8'_1$ DNA und Pst I-Pvu II$^+$ (82 bp) aus $4_1$ DNA auf übliche Weise isoliert.

Die isolierte Plasmid-DNA CG-pBR 322/HLycIFN-$5_1$ wird wie folgt behandelt. Einerseits werden 5 µg der Plasmid-DNA mit Hae III digeriert, 5'-terminal markiert und mit Pst I gespalten. Das Pst I-Hae III$^+$ (57 bp) DNA-Fragment wird an 10%igem Polyacrylamidgel isoliert. Andererseits werden 5 µg des Plasmids mit EcoR I digeriert, 5'-terminal markiert und mit Pst I gespalten. Die Pst I-EcoR I$^+$ (235 bp) und EcoR I$^+$-Pst I (~700 bp) DNA-Fragmente werden an 8%igem Polyacrylamidgel isoliert. Die verschiedenen DNA-Fragmente werden der Sequenzanalyse gemäß Maxam und Gilbert (39) unterzogen.

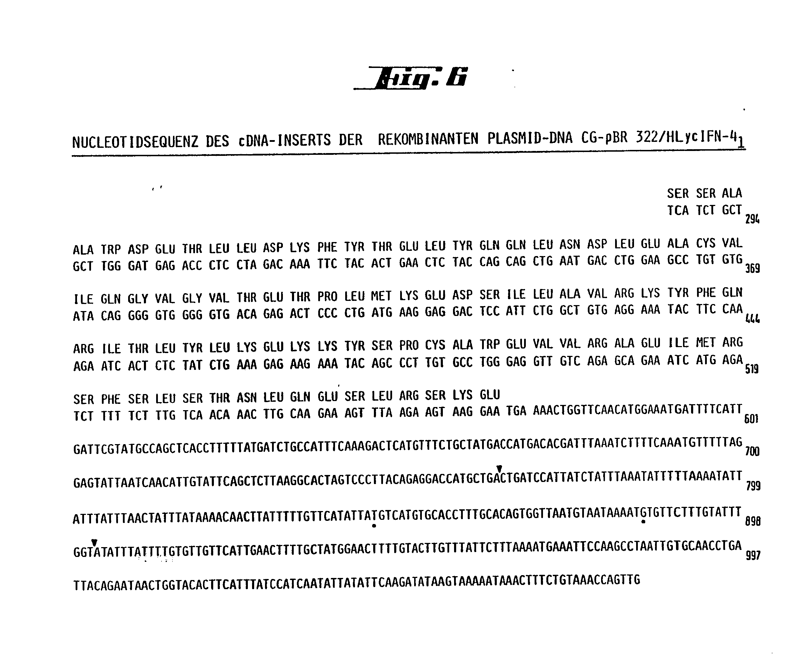

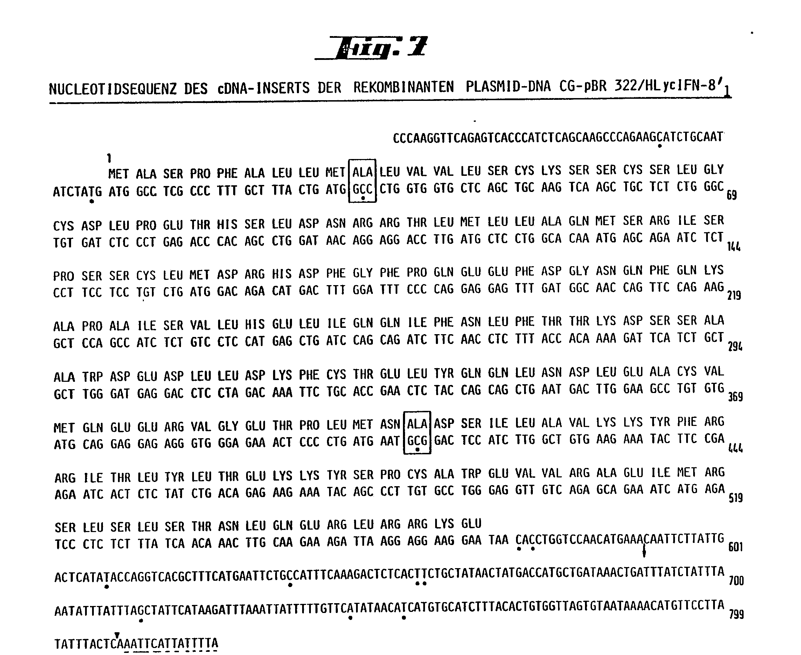

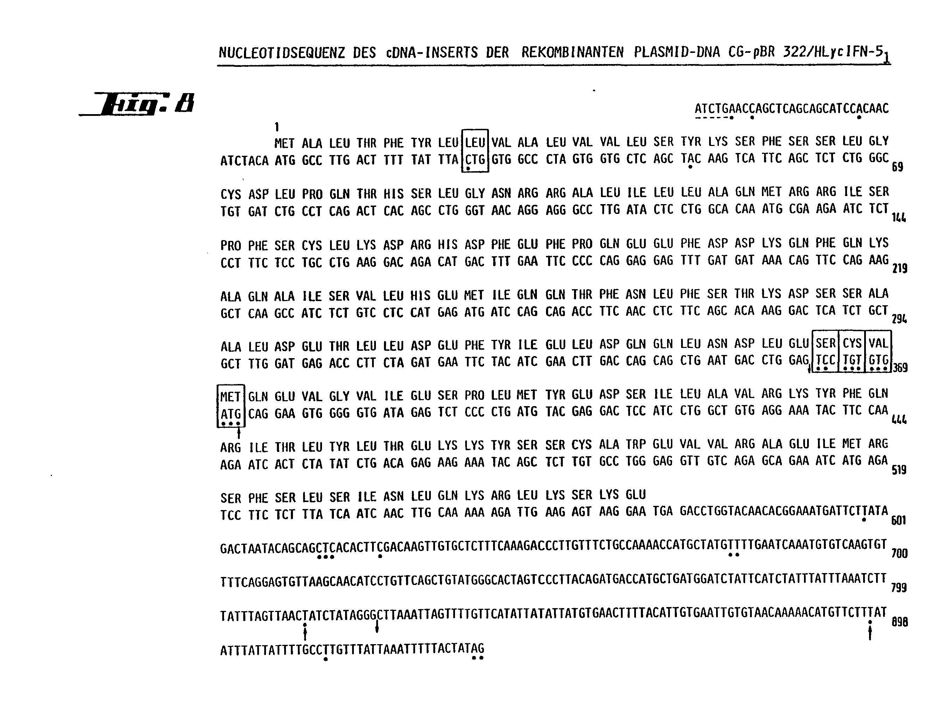

Die Nucleotidsequenzen der cDNA-Inserts sind aus den Fig. 6 bis 8 ersichtlich. In Fig. 6 ist die Nucleotidsequenz des cDNA-Inserts von CG-pBR 322/HLycIFN-$4_1$ dargestellt. Der Insert ist am 5'-Ende durch einen Bereich von 23 Desoxyguanosin-Resten flankiert und umfasst einen Teil der IFN-$\alpha_2$(Le) cDNA, wie von Streuli et al. (13) beschrieben. In dem 3'-extra-cistronischen Bereich treten einige geringe Abweichungen (Punktmutationen) auf und ein Bereich von zusätzlichen 318 Nucleotiden. Die Nucleotidsequenz des cDNA-Inserts von CG-pBR 322/HLycIFN-$8'_1$ ist in Fig. 7 gezeigt. Der Insert ist am 5'-Ende durch einen Bereich von 20 bis 23 Desoxyguanosin-Resten flankiert und ist ähnlich, jedoch nicht identisch, der von Goeddel et al. [(15); vergl. auch Mantei et al. (12)] beschriebenen IFN-α(Typ D) cDNA. Neben den Unterschieden in den cDNA-Bereichen, die der IFN-Codierungssequenz vorhergehen und folgen, enthält das IFN-Gen an den Stellen 28 bis 30 ein GCC-Triplett und an den Stellen 409 bis 411 ein GCG-Triplett, codierend für Alanin, anstelle von GTC bzw. GTG und codierend für Valin. Schließlich zeigt die Nucleotidsequenz des cDNA-Inserts von CG-pBR 322/HLycIFN-$5_1$ (siehe Fig. 8) einen Bereich von 17 Desoxyguanosin-Resten am 5'-Ende. Die Nucleotidsequenz steht in Beziehung zu derjenigen von IFN-α(Typ B) cDNA, wie von Goeddel et al. (15) beschrieben wird. Es treten jedoch zusätzliche Nucleotide am 5'-Ende de cDNA-Inserts von HLycIFN-$5_1$, Punktmutationen, Exzisionen und Einfügungen in dem extracistronischen Bereich und in der IFN-Codierungssequenz, insbesondere in den Positionen 22 und 361 bis 372, auf.

6. Synthese von Humaninterferonen durch E. coli, enthaltend humane IFN-spezifische rekombinante DNA-Moleküle

Die fünf Klone, von denen gezeigt wurde, daß sie humane IFN-spezifische rekombinante DNA-Moleküle enthalten, nämlich

*E. coli* HB 101 CG-pBR 322/HLycIFN-1'b
*E. coli* HB 101 CG-pBR 322/HLycIFN-$4_1$,
*E. coli* HB 101 CG-pBR 322/HLycIFN-$5_1$,
*E. coli* HB 101 CG-pBR 322/HLycIFN-$8'_1$, und
*E. coli* HB 101 CG-pBR 322/HLycIFN-$\beta_1$,

werden auf die IFN-Aktivität geprüft, wobei man in jedem Fall folgendermaßen vorgeht:

Kulturen des entsprechenden *E. coli*-Klons (30 ml Suspensionen) werden in Trypton-Medium bis zu einer optischen Dichte ($OD_{650}$) von etwa 1 gezüchtet. Die Zellen werden geerntet und in 0,5 ml einer wäßrigen Lösung resuspendiert, die 30 mM NaCl und 50 mM Tris-HCl (pH 8,0) enthält. Lysozym (Sigma) wird bis zu 1 mg/ml zugegeben. Nach 30 min bei 0°C werden die Suspensionen gefroren (flüssiger Stickstoff) und bei 37°C aufgetaut (fünfmal) und 20 min bei 20 000 U/min in einem SS 34 Sorvall Rotor bei 4°C zentrifugiert. Die Überstände werden auf die IFN-Aktivität unter Verwendung des cythopathischen Bioassays gemäß Armstrong (19), wie in Stufe 1c beschrieben, analysiert. Es werden die folgenden Aktivitäten festgestellt:

| Extraktquelle | IFN-Aktivität |
|---|---|
| *E. coli* HB 101, enthaltend rekombinante DNA | (IE/ml) |
| CG-pBR 322/HLycIFN-1'b | 0;0 |
| CG-pBR 322/HLycIFN-$4_1$ | 0;0 |
| CG-pBR 322/HLycIFN-$5_1$ | 10 000;10 000 |
| CG-pBR 322/HLycIFN-$8'_1$ | 100;100 |
| CG-pBR 322/HLycIFN-$\beta_1$ | 0;0 |

Möglicherweise enthalten Klone, die keine messbaren IFN-Aktivitäten zeigen, rekombinante DNAs, in denen der HuLyIFN cDNA-Insert die falsche Orientierung in bezug auf die Transkriptionsrichtung aufweist. Daher wird die rekombinante DNA eines solchen Klons (CG-pBR 322/HLycIFN-1'b), die einen vollständigen cDNA-Insert enthält, wie folgt reorientiert:

Die Plasmid-DNA des Klons *E. coli* HB 101 CG-pBR 322/HLycIFN-1'b wird gemäß Stufe 5c isoliert und mit Pst I gespalten. 0,5 µg der gespaltenen DNA in 20 µl eines Puffergemisches mit einem Gehalt an 20 mM Tris-HCl (pH 7,8), 10 mM $MgCl_2$, 10 mM DTT, 25 mM NaCl und 50 µg/ml Gelatine werden mit 0,2 Einheiten T4 DNA-Ligase (Biolabs) und 0,5 mM ATP während 2 h bei 15°C behandelt. *E. coli* HB 101 wird mit dem cDNA-Gemisch gemäß Stufe 4 transformiert. Transformierte Kolonien werden auf Mc Conkey-Agarplatten, supplementiert mit Tetracyclin, selektiert und danach auf Nitrocellulose-Filter replica-plattiert. Vier Bakterienkolonien, die mit dem $^{32}P$-markierten Pvu II-Bgl $II^{+}$-Fragment (351 bp) der rekombinanten DNA CG-pBR 322/HLycIFN-1'b (vergl. Stufe 5e) hybridisieren, werden als *E. coli* HB 101 CG-pBR 322/HLycIFN-1'$b_1$ bis -1'$b_4$ bezeichnet. Extrakte der vier Klone werden hergestellt und auf die IFN-Aktivität, wie oben beschrieben, geprüft. Die folgenden Aktivitäten werden gefunden:

| Extraktquelle | IFN-Aktivität |
|---|---|
| *E. coli* HB 101, enthaltend rekombinante DNA | (IE/ml) |
| CG-pBR 322/HLycIFN-1'$b_1$ | 0;0 |
| CG-pBR 322/HLycIFN-1'$b_2$ | 0;0 |
| CG-pBR 322/HLycIFN-1'$b_3$ | 0;0 |
| CG-pBR 322/HLycIFN-1'$b_4$ | 30;30 |

Das Plasmid CG-pBR 322/HLycIFN-1'$b_4$ enthält einen cDNA-Insert, der die Synthese eines Polypeptids mit IFN-Aktivität dirigieren kann.

7. Aufbau rekombinanter Plasmide, die hohe Gehalte an Polypeptiden mit IFN-Aktivität produzieren können, und Transformation von E. coli HB 101 mit diesen Plasmiden

A. *Aufbau des rekombinanten Plasmids CG-pBR (AP)/LyIFN-α-1*

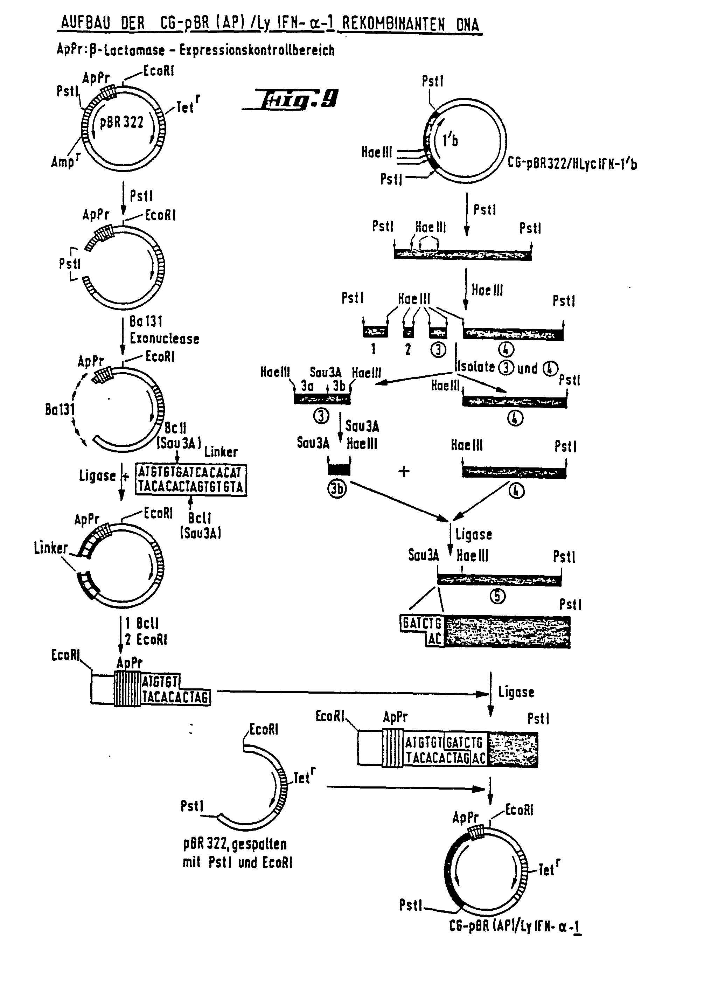

Zur Verbesserung der IFN spezifischen Proteinausbeute des Klons *E. coli* HB 101 CG-pBR 322/HLycIFN-1'b wird die folgende Konstruktion, wie schematisch in Fig. 9 angedeutet, durchgeführt.

a. *Herstellung des cDNA-Inserts*

Die rekombinante Plasmid-DNA (150 µg) des Klons *E. coli* HB 101 CG-pBR 322/HLycIFN-1'b wird mit Pst I (Biolabs) unter Verwendung von Standardverfahren (vergl. Stufe 5d) gespalten. Anschließend an die Phenolextraktion und die Äthanolpräzipitation wird der exzidierte Insert mittels Saccharose-Dichtegradienten-Zentrifugation (5 bis 23%) in 50 mM Tris-HCl (pH 8,0) und 1 mM EDTA isoliert. Die Zentrifugation erfolgt bei 35 000 U/min in einem TST 41 Rotor (Kontron AG) bei 15°C während 16 h. 0,3 ml-Fraktionen Werden mit einem ISCO-Gradientkollektor bei 1 ml/min gesammelt. Die das kleine Fragment (d.h. den Insert) enthaltenden Fraktionen werden vereinigt. Die DNA wird auf übliche Weise mit Äthanol präzipitiert und das Präzipitat durch Zentrifugieren in einem HB-4 Rotor (Sorvall) bei 10 000 U/min und 0°C während 10 min gesammelt. Das Präzipitat wird erneut in 60 µl 10 mM Tris-HCl (pH 7,5) und 0,05 mM EDTA gelöst. Man gewinnt 30 µg DNA, bestimmt durch Messung der optischen Dichte.

Die Insert-DNA (10 µg) wird mit Hae III (Biolabs) digeriert und die Fragmente werden an 2%igem Agarosegel in einer Lösung mit einem Gehalt an 50 mM Tris, 50 mM Borsäure, 1 mM EDTA und 0,5 µg/ml Äthidiumbromid fraktioniert. Die größten DNA-Fragmente, Hae III-Pst I (869 bp) und Hae III-Hae III (82 bp, vergl. Fig. 9, Fragmente 3 bzw. 4) werden jeweils aus dem Gel exzidiert, durch eine dünne Nadel mit einer Spritze in 5 ml 0,15 M NaCl, 50 mM Tris.HCl (pH 8,0), 1 mM EDTA gespritzt und über Nacht durch Schütteln eluiert. Das Eluat wird durch eine 100 µl DE-52 (Whatman) Pasteur-Pipetten-Säule zur Adsorption von DNA geleitet. Die Säule wird mit 2 ml des gleichen Puffers gewaschen und die DNA mit 400 µl einer Lösung eluiert, die 1,5 M NaCl, 50 mM Tris (pH 8,0) und 1 mM EDTA enthält. Die DNA wird mit 2 Vol. Äthanol bei −20°C über Nacht präzipitiert. Das Präzipitat wird durch Zentrifugieren in einer Eppendorf-Zentrifuge gesammelt.

Das Hae III-Hae III DNA-Fragment (82 bp) wird erneut gelöst und mit Sau 3A (Biolabs) digeriert. Das Enzym wird 30 min bei 65°C in der Wärme inaktiviert. 1 µg des Hae III-Pst I DNA-Fragments (869 bp) wird zugesetzt, die Lösung wird auf 10 mM $MgCl_2$, 10 mM DTT und 0,5 mM ATP eingestellt und T4 DNA-Ligase (Biolabs) wird zu 30 Einheiten/µl Reaktionsvolumen zugesetzt. Die Lösung wird 10 h bei 15°C inkubiert. Anschließend an die Extraktion mit Phenol und Chloroform wird die Mischung an 2%igem Agarosegel in Tris-Borat-EDTA in Anwesenheit von Äthidiumbromid fraktioniert. Das Sau 3A-Pst I DNA-Fragment (vergl. Fig. 9, Fragment 5) wird, wie zuvor beschrieben, extrahiert, mit Äthanol präzipitiert und erneut in 10 µl einer Lösung mit einem Gehalt von 10 mM Tris.HCl (pH 7,5) und 0,05 mM EDTA gelöst.

b. *Herstellung des DNA-Fragments, das den regulatorischen Bereich des β-Lactamase-Gens (ApPr) von pBR 322 enthält*

Das Plasmid pBR 322 wird mit Pst I gespalten (vergl. Stufe 3b) und mit 4 E/ml Exonuclease Bal 31 (Bethesda Research Lab.) 4 bis 10 min bei 30°C zur Entfernung des β-Lactamase-Codierungssegments behandelt.

Ein chemischer DNA-Linker der Formel

5'-ATGTGTGATCACACAT-3'

wird unter Verwendung des in Stufe 5a beschriebenen Verfahrens synthetisiert. Der Linker wird zu der Bal 31 behandelten pBR 322 DNA mittels an sich bekannter Verknüpfung angefügt. Das entstehende Hybridmolekül wird mit den Restriktionsendonucleasen Bcl I (Biolabs) und EcoR I gespalten. Die Digestionsprodukte werden an 8%igem Polyacrylamidgel in Tris-Borat-EDTA gemäß Stufe 2a fraktioniert. DNA-Fragmente (ApPr DNA-Fragmente), die zwischen 184bp und 234 bp Marker-DNAs migrieren, werden gemäß Stufe 7a isoliert und auf übliche Weise mit Äthanol präzipitiert. Das Präzipitat wird erneut in einer Lösung, enthaltend 10 mM Tris.HCl (pH 7,5) und 0,05 mM EDTA, gelöst.

c. *Verknüpfung des ApPr-DNA-Fragments mit dem cDNA-Insert und Herstellung des Plasmids CG-pBR(AP)/LyIFN-α-1*

Die die ApPr DNA-Fragmente und den cDNA-Insert enthaltenden Lösungen werden vereinigt. Die Mischung wird auf 10 mM $MgCl_2$, 10 mM DTT und 0,5 mM ATP eingestellt und 12 h bei 15°C mit 30 E/µl T4 DNA-Ligase (Biolabs) inkubiert. Anschließend an die Extraktion mit Phenol und Chloroform wird die Mischung an 1%igem niedrigschmelzenden Agarosegel (Biorad) fraktioniert. Das erhaltene ApPr-cDNA-Fragment wird mit dem großen Fragment von pBR 322, das sowohl mit Pst I (Biolabs) als auch mit EcoR I (Biolabs) gespalten wurde, folgendermassen verbunden. Das das ApPr cDNA-Fragment (etwa 20 µl) enthaltende Gelstück wird mit dem Pst I-EcoR I-Fragment von pBr 322 vermischt, 2 min bei 65°C geschmolzen, auf 37°C gekühlt, auf 0,5 mM ATP, 10 mM DTT und 10 mM $MGCl_2$ eingestellt und 12 h bei 15°C mit 30 E/ µl T4 DNA-Ligase (Biolabs) inkubiert, wobei man eine Lösung erhält, die das rekombinante Plasmid, mit CG-pBR(AP)/LyIFN-α-1 bezeichnet, enthält.

d. *Transformation von E. coli HB 101 mit dem Plasmid CG-pBR(AP)/LyIFN-α-1*

1/10 Volumen einer Lösung mit einem Gehalt an 100 mM Tris.HCl (pH 7,5), 100 mM $CaCl_2$ und 100 mM $MgCl_2$ wird zu der das Plasmid CG-pBR(AP)/LyIFN-α-1 enthaltenden Lösung gegeben. Die vereinigten Lösungen werden 10 min bei 65°C zur Inaktivierung der Ligase erhitzt und auf 37°C abgekühlt. Die Lösung wird dann verwendet, um $Ca^{2+}$-behandeltes *E. coli* HB 101 gemäß Stufe 4 zu transformieren, und auf Mc Conkey-Agarplatten, supplementiert mit 10 µg/ml Tetracyclin, plattiert. Die transformierten Kolonien werden auf ihre IFN-Aktivität (vergl. Stufe 6) geprüft. Der Klon, der den höchsten Gehalt an IFN-Aktivität synthetisiert, wird ausgewählt und als *E. coli* HB 101 CG-pBR(AP)/LyIFN-α-1 bezeichnet. Man stellt eine Aktivität von 40 000 (IE/ml) fest, was einer 1300 fachen Stimulierung entspricht, verglichen mit dem ursprünglichen Klon *E. coli* HB 101 CG-pBR 322/HLycIFN-1'b.

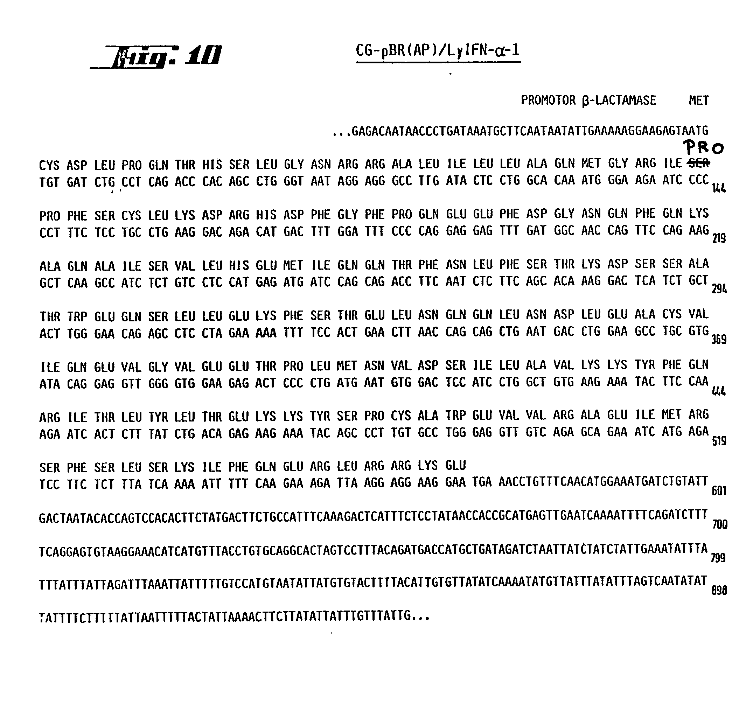

Die rekombinante Plasmid-DNA des Klons CG-pBR(AP)/LyIFN-α-1 wird aus der Kultur gemäß Stufe 3c isoliert und charakterisiert, indem man die Nucleotidsequenz des cDNA-Inserts (IFN-Gen) und den β-Lactamase-regulatorischen Bereich ermittelt. Das Ergebnis ist in Fig. 10 dargestellt.

B. *Aufbau des rekombinanten Plasmid CG-pBR(AP)/LyIFN-α-3*

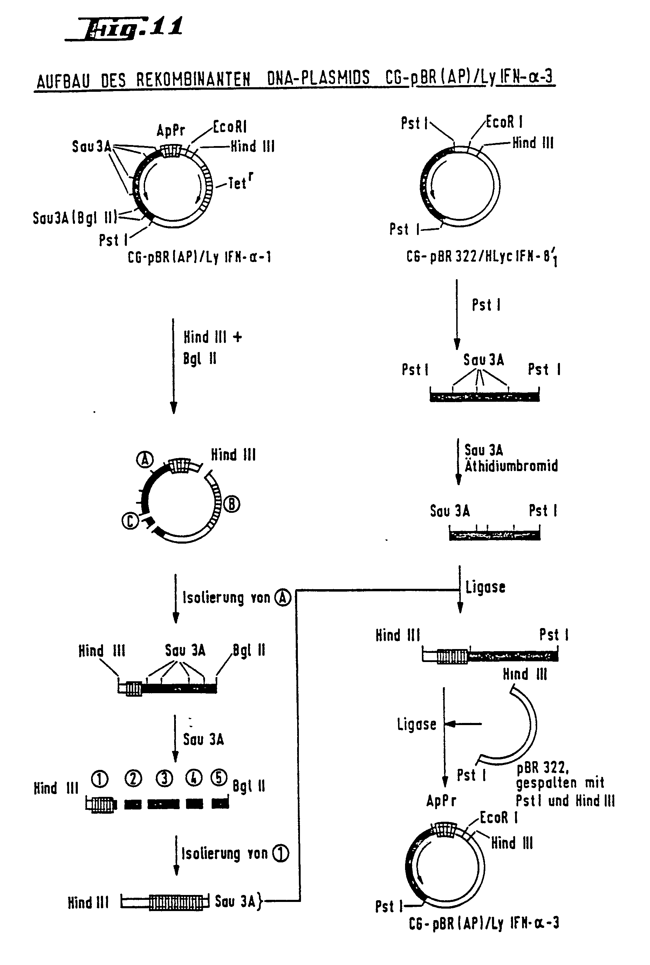

Die IFN-spezifischen Proteinausbeuten des Klons *E. coli* HB 101 CG-pBR 322/HLycIFN-8$_1'$ wird wie folgt verbessert (vergl. Fig. 11):

a. *Herstellung des DNA-Fragments, enthaltend den β-Lactamase-regulatorischen Bereich aus CG-pBR(AP)/LyIFN-α-1*

CG-pBR(AP)/LyIFN-α-1-DNA (100 µg) wird mit Hind III (Biolabs) und Bgl II (Biolabs) gespalten. Anschließend an die Phenolextraktion und die Äthanolpräzipitation wird das exzidierte DNA-Fragment durch Saccharose-Dichtegradienten-Zentrifugierung (5 bis 23%) in 50 mM Tris-HCl (pH 8,0) und 1 mM EDTA isoliert. Die Zentrifugation erfolgt 4 h bei 15°C mit 58 000 U/min in einem TST 60 Rotor (Kontron AG). 0,2 ml-Fraktionen werden, wie zuvor beschrieben, gesammelt. Die das kleine Fragment (Hind III-Bgl II) enthaltenden Fraktionen werden vereinigt und die DNA wird mit Äthanol wie üblich präzipitiert. Das Präzipitat wird erneut in 80 µl 10 mM Tris-HCl (pH 7,5) und 0,05 mM EDTA gelöst. 16 µg DNA werden isoliert, bestimmt durch Messung der optischen Dichte.

Das DNA-Fragment (Hind III-Bgl II) (4 µg) wird mit Sau 3A (Biolabs) gespalten und die Digestionsprodukte werden an 6%igem Polyacrylamidgel in Tris-Borat-EDTA, wie zuvor beschrieben, fraktioniert. Die DNA-Fragmente werden in EtBr (0,5 µg/ml) angefärbt. Das Hind III-Sau 3A-DNA-Fragment (239 bp) wird extrahiert und isoliert, wie zuvor beschrieben. Die DNA wird mit Äthanol auf übliche Weise präzipitiert. Das Präzipitat wird erneut in 20 µl 10 mM Tris-HCl (pH 7,5) und 0,05 mM EDTA gelöst.

b. *Herstellung des cDNA-Inserts*

Der cDNA-Insert wird aus dem rekombinanten Plasmid CG-pBR 322/HLycIFN-8$_1'$ mit Pst I, wie oben beschrieben (Abschnitt 7a), exzidiert.

Der cDNA-Insert (2 µg) wird mit 2,5 Einheiten Sau 3A (Biolabs) in 10 µg/ml EtBr digeriert und 60 min bei 37°C inkubiert. Die Spaltstücke werden mit Phenol extrahiert und die DNA, wie oben, in Aethanol präzipitiert. Die DNA-Fragmente werden an 1,2%igem Agarosegel in einer Lösung mit einem Gehalt an 50 mM Tris, 50 mM Borsäure, 1 mM EDTA und 0,5 µg/ml Äthidiumbromid fraktioniert.

Die zweitgrößte DNA (Sau 3A-Pst I: 693 bp) wird aus dem Gel extrahiert und gemäß Abschnitt 7a gereinigt. Die DNA wird erneut in 20 µl 10 mM Tris-HCl (pH 7,5) und 0,05 mM EDTA gelöst.

c. *Verknüpfung des Hind III-Sau 3A DNA-Fragments mit dem cDNA-Insert (Sau 3A-Pst I)*

Gleiche Mengen beider DNA-Fragmente (~50 ng) werden in einer Lösung inkubiert, die 10 mM $MgCl_2$, 10 mM DTT, 0,5 mM ATP und 30 E/µl T4 DNA-Ligase (Biolabs) enthält, und zwar 3 h bei 15°C. Die Mischung wird 15 min bei 80°C inkubiert und auf 50 mM NaCl eingestellt. Das DNA-Gemisch wird mit 0,5 Einheiten Pst I (Biolabs) und 1 Einheit Hind III (Biolabs) 20 min bei 37°C digeriert. Die DNA wird mit Phenol extahiert, mit Äthanol präzipitiert und erneut in 10 µl 10 mM Tris-HCl (pH 7,5) und 0,05 mM EDTA gelöst.

Eine Hälfte des entstehenden Gemisches wird mit dem großen Hind III-Pst I DNA-Fragment des Plasmids pBR 322 (~100 ng) in 10 mM $MgCl_2$, 10 mM DTT, 0,5 mM ATP, enthaltend 30 E/µl T4 DNA-Ligase (Biolabs), 2 h bei 15°C verknüpft. Man erhält eine Lösung, die das rekombinante Plasmid CG-pBR(AP)/LyIFN-α-3 enthält.

d. *Transformation von E. coli HB 101 mit dem Plasmid CG-pBR(AP)/LyIFN-α-3*

1/10 Volumen der obigen Lösung wird zur Transformation von *E. coli* HB 101, wie in Stufe 4 beschrieben, verwendet. Die transformierten Kolonien werden zur Prüfung der IFN-Aktivität gemäß Stufe 6 verwendet.

Der Klon, der den höchsten Wert an IFN-Aktivität synthetisiert, wird ausgewählt und mit *E. coli* HB 101 CG-pBR(AP)/LyIFN-α-3 bezeichnet.

Die IFN-Aktivität wird gemäß obiger Stufe 6 bestimmt. Es wird eine Aktivität von 70 000 (IE/ml) festgestellt, was einer 700 fachen Stimulierung im Vergleich mit dem ursprünglichen Klon *E. coli* HB 101 CG-pBR 322/HLycIFN-8$_1'$ entspricht.

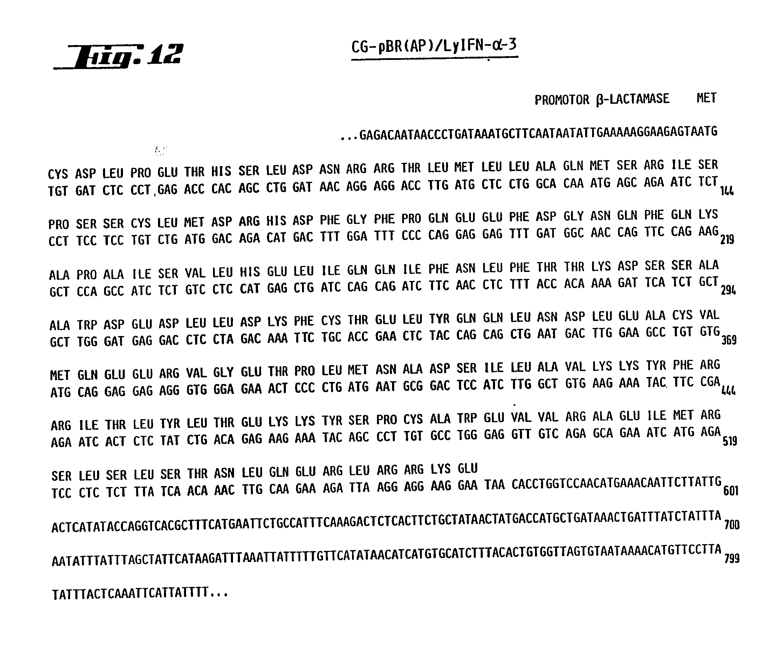

Die rekombinante Plasmid DNA des Klons CG-pBR(AP)/LyIFN-α-3 wird aus der Kultur gemäß Stufe 3c isoliert und charakterisiert, indem man die Nucleotidsequenz des cDNA-Inserts (IFN-Gen) und des β-Lactamase-regulatorischen Bereichs bestimmt. Das Ergebnis ist in Fig. 12 zusammengefaßt.

Das Aufbauprotokoll für das Plasmid CG-pBR(AP)/LyIFN-α-3 kann im allgemeinen für alle α-IFN cDNA-Gene oder entsprechend geschnittene chromosomale α-IFN-Gene verwendet werden.

Beispielsweise wird, ausgehend von dem Plasmid CG-pBR 322/HLycIFN-5$_1$, das Plasmid CG-pBR(AP)/LyIFN-α-2 auf identische Weise, wie für das Plasmid CG-pBR(AP)/LyIFN-α-3 beschrieben, erhalten. Dieses neue Plasmid enthält den DNA-Insert von CG-pBR 322/HLycIFN-5$_1$ und den β-Lactamase-regulatorischen Bereich von CG-pBR(AP)/LyIFN-α-1. Ein mit *E. coli* HB 101 CG-pBR(AP)/LyIFN-α-2 bezeichneter Klon wird, wie oben beschrieben, ausgewählt. Man stellt eine IFN-Aktivität von 50 000 (IE/ml) fest, was einer 5 fachen Stimulierung im Vergleich mit dem ursprünglichen *E. coli* HB 101 CG-pBR 322/HLycIFN-5$_1$ enspricht. Die

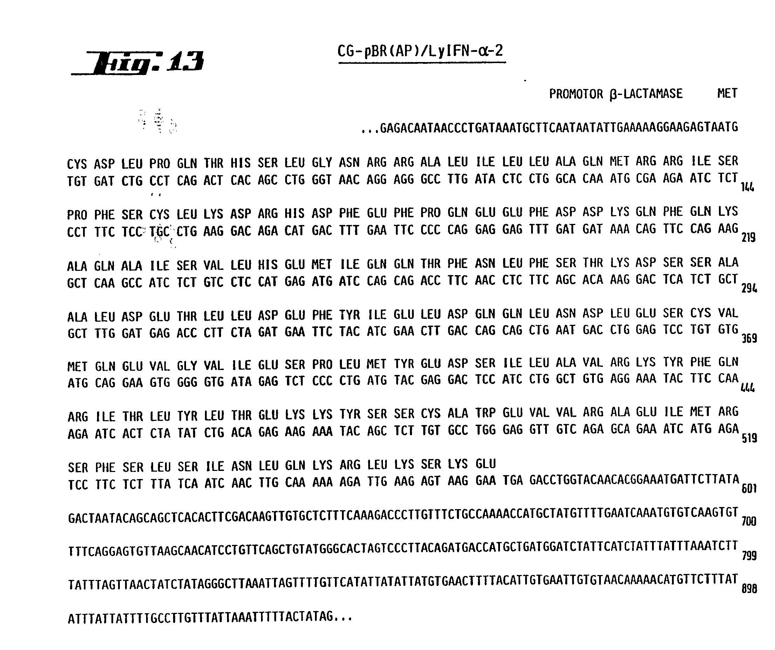

Nucleotidsequenz des cDNA-Inserts und des β-Lactamase-regulatorischen Bereichs des Plasmids CG-pBR(AP)/LyIFN-α-2 wird, wie oben beschrieben, bestimmt und ist in Fig. 13 darstellt.

8. Kultivierung des Stamms E. coli HB 101 CG-pBR(AP)/LyIFN-α-3 im Maßstab einer Fermentationseinrichtung

Der Stamm *E. coli* HB 101 CG-pBR(AP)/LyIFN-α-3 wird in dem Medium Nr. X gezüchtet, welches die folgenden Bestandteile pro 1 l Lösung enthält:

| | |
|---|---|
| $Na_2HPO_4 \cdot 7H_2O$ | 13,25 g |
| $KH_2PO_4$ | 3,0 |
| NaCl | 0,5 |
| $NH_4Cl$ | 1,0 |
| $CaCl_2 \cdot 2H_2O$ | 0,015 |
| $MgSO_4 \cdot 7H_2O$ | 0,25 |
| Eisen(III)-citrat | 0,006 |
| Casaminosäuren | 9,0 |
| Hefeextrakt | 2,0 |
| Cerelose | 8,0 |
| Tetracyclin | 0,01 |

Getrennt von der Masse des Mediums werden Cerelose und Tetracyclin durch Hitzesterilisierung bzw. sterile Filtration sterilisiert. Drei 2 l Schüttelkolben mit einem Gehalt an 500 ml Medium Nr. X werden jeweils mit den Zellen von einem Schrägagarröhrchen inokuliert. Die Schüttelkolben mit vier Einbuchtungen werden während 11 h bei 120 U/min in einer Rotationsschüttelvorrichtung inkubiert. 1,5 l dieser Vorkultur werden in einen 500 l Fermenter transferiert, welcher 300 l Medium Nr. X enthält, und dann wird unter den folgenden Bedingungen gezüchtet: Rühren bei 350 bis 500 U/min mit einem Scheibenrührer, Belüftungsrate 0,3 bis 1,0 1/1·min, Fermenterüberdruck 0,3 bar, Temperatur 30°C. Es wird verhindert, dass der Gehalt an gelöstem Sauerstoff unter 50% Sättigung fällt, indem man die Belüftungsrate und gegebenenfalls die Rührgeschwindigkeit auf die Maximalwerte erhöht. Der pH-Wert wird über 6,8 durch kontrollierte Zugabe von NaOH gehalten. Nach etwa 10 stündigem Züchten erreicht die Kultur einen maximalen interferontiter [bestimmt gemäß Armstrong (19)] und dann wird geerntet.

9. Isolierung und Reinigung von HLyIFN-α-3

a. *Herstellung der Polypeptidlösung für die monoklonale Antikörpersäule*

280 l Kulturbrühe von pH 7,2 werden auf 10°C gekühlt, und die Zellen werden mit einem Schlammseparator Alfa-Laval BRPX-207 abgetrennt. Der klare Ueberstand enthält keine IFN-Aktivität. Vor dem Gewinnen der Zellmasse, die sich im Schlammraum des Separators gesammelt hat, wird der Ueberstand mit 20 l Lysepuffer A [50 mM Tris. HCl, 50 mM EDTA, 0,2 M NaCl, 10 μM PMSF (Phenylmethylsulfonylfluorid); 1 mM L-Cystein, eingestellt mit HCl auf pH 8,2) verdrängt und der Inhalt der Separatortrommel (7 l) wird mittels einer Vollentschlammung ausgestossen. Die Separatortrommel wird dreimal mit 2 l Lysepuffer A gespült. Die erhaltene Zellmasse wird mit Puffer A auf 20 l eingestellt und besitzt einen pH-Wert von 6,9. Nach dem Kühlen auf 5 bis 10°C wird die Suspension durch eine DYNO®-Mühle (Typ KDL-Pilot, 1,4 l) geleitet, welche mit Polyurethan-Rührscheiben sowie mit 1170 ml Glasperlen mit einem Durchmesser von 0,5 bis 0,75 mm ausgerüstet ist. Die Rührgeschwindigkeit beträgt 3350 U/min und die Durchflussgeschwindigkeit 5 l/h. Dabei werden die Zellen aufgeschlossen. 800 ml Lysepuffer A (mit einem zusätzlichen Gehalt an 100 g Polyäthylenimin, eingestellt mit HCl auf pH 8,2) werden zu der erhaltenen Suspension der aufgeschlossenen Zellen bei 2°C unter mässigem Rühren zugegeben. Die Suspension mit einem pH von etwa 7,6 wird 3 h auf −2°C gekühlt und zentrifugiert. Der Ueberstand (17,2 l) wird mit 3028 g Ammoniumsulfat versetzt. Die leicht trübe Lösung wird nach einstündigem Stehenlassen bei 6°C zentrifugiert. Der Ueberstand wird mit 4324 g Ammoniumsulfat behandelt und nach Stehenlassen über Nacht mit 3000 U/min zentrifugiert. Das nasse Sediment (etwa 1224 g) wird in Puffer B (25 mM Tris. HCl, 10 μM PMSF, eingestellt mit HCl auf pH 8,5) gelöst, wobei man 2800 ml einer Lösung erhält, die das gewünschte Polypeptid enthält.

Ein aliquoter Teil von 700 ml dieser Polypeptidlösung wird bei Zimmertemperatur durch eine H1P10 Ultrafilterpatrone mittels eines Amicon DC-2 Hollow Fibre Systems unter Verwendung von 7 l Puffersystem B diafiltriert. Die Filterpatrone wird mit Puffer B gewaschen, die diafiltrierte Lösung und die Waschlösungen werden vereinigt (1440 ml) und mit einer Strömungsgeschwindigkeit von 200 ml/h auf eine DEAE-Säule (Trisacryl® M DEAE, LKB 2205-300) mit einem Bettvolumen von 450 ml geleitet, wobei die Säule zuvor mit Puffer B prä-äquilibriert wurde. Die erste Polypeptid-Fraktion mit einer UV-Absorption bei 280 nm Wellenlänge wird verworfen. Die Säule wird weiter mit Puffer B gewaschen, bis mindestens fünf Bettvolumen Waschlösung die Basislinienabsorption von 280 nm aufweisen. Die absorbierten Polypeptide werden dann mit 2,8 l Puffer C (0,2 M NaCl, 25 mM Tris-HCl, pH 8,5) eluiert. Die Säulenchromatographie erfolgt bei 4°C. Das Eluat zeigt in der Analyse gemäß Armstrong (19) eine IFN-Aktivität von $1{,}4 \cdot 10^5$ IE/mg Polypeptid. Das Eluat wird mit 2M HCl auf einen pH von 7,4 eingestellt und in 100 ml Aliquots bei −20°C eingefroren, bis sie für die monoklonale Antikörpersäule verwendet werden.

b. *Reinigung von humanem LyIFN-α-3 an einer monoklonalen Antikörpersäule*

Die monoklonale Antikörpersäule 1K2-20 (Bettvolumen 0,8 ml, siehe unten) wird mit PBS (Phosphatgepufferter Salzlösung: 0,137 M NaCl, 0,0027 M KCl, 0,0077 M $Na_2HPO_4 \cdot 12H_2O$, 0,0015 M $KH_2PO_4$, pH 7,4) äquilibriert, und 10 ml-Portionen der obigen Polypeptidlösung werden bei Zimmertemperatur auf die Säule mit einer Strömungsrate von 10 ml/h aufgebracht. Die ersten Fraktionen, die die nichtadsorbierten Polypeptide enthalten und 3 ml PBS-Waschlösungen werden verworfen. Weiterhin werden nicht spezifisch gebundene Polypeptide mit 3 ml PBS mit einem zusätzlichen Gehalt an 0,5 M NaCl und 0.2% Triton X 100 eluiert. Die Säule wird mit 3 ml PBS gewaschen, worauf die spezifisch adsorbierten Polypeptide mit 3 ml Puffer D (0,1 M Citronensäure, 0,3 M NaCl, pH 2) eluiert werden. Diese Fraktion und 4 ml einer nachfolgenden PBS-Waschlösung werden vereinigt, der pH wird mit 2N NaOH auf 6,3 eingestellt und dann wird bei 4°C auf das 10 fache mit Hilfe eines eintauchbaren CX™ Molecular-Separators (Millipore®) konzentriert. Das Konzentrat wird auf eine Sephadex G-25 Säule (2,6 × 34 cm, 200 ml Bettvolumen), äquilibriert mit 0,025 M Histidin.HCl (pH 6,3), aufgetragen. Bei 4°C und mit einer Strömungsrate von 42 ml/h wird die Säule mit dem gleichen Histidin.HCl von pH 6,3 eluiert. Es werden 20 Fraktionen gesammelt (je 10,5 ml). Die Polypeptid enthaltenden Fraktionen werden durch ihre optische Absorption bei 280 nm festgestellt. Die Fraktionen 7 und 8 enthalten das Polypeptid mit IFN-Aktivität, was durch die Analyse gemäß Armstrong (19) festgestellt wurde. Die aktiven Fraktionen, die LyIFN-α-3 enthalten, werden bei −20°C oder in einem Eisbad bis zur weiteren Verwendung gelagert. Die IFN-Aktivität der Fraktionen beträgt $1{,}8 \times 10^8$ IE/mg Polypeptid (19).

Durch Lyophilisierung der obigen Fraktionen aus 1 ml Lösung erhält man 20 bis 40 µg des Polypeptids.

Die SDS-Polyacrylamidgel-Elektrophorese [vergl. (41)] zeigt ein Molekulargewicht des erhaltenen LyIFN-α-3 von etwa 18 kDalton.

Ein Ultradünnschicht-isoelektrisches Fokussieren an Polyacrylamidgel (100 µM), durchgeführt gemäß B. J. Radola (42), innerhalb eines pH-Bereichs von 4,5 bis 6,5 zeigt das reine aktive humane LyIFN-α-3 am isoelektrischen Punkt von 5,3 bis 5,4 pH-Einheiten an.

c. *Herstellung der monoklonalen Antikörpersäule 1K2-20*

A. *Immunisierung von Mäusen*

Balb/c Mäuse (8 Wochen alt, erhalten von der Tierfarm Sisseln, Schweiz) werden mit $3 \times 10^5$ Einheiten humanem Leukocyt IFN-α (Reinheit 1%) in komplettem Freund's Adjuvans (Difco), verteilt in die vier Pfoten, injiziert. Am Tag 30 wird die gleiche Menge an IFN in inkomplettem Freund's Adjuvans auf gleiche Weise injiziert. Die dritte Injektion erfolgt am Tag 85, wenn $4 \times 10^5$ Einheiten an humanem Leukocyt IFN intraperitoneal in Salzlösung verabreicht werden. 4 Tage später wird die Milz für die Fusion entnommen.

B. *Herstellung der Hybridomas*

Alle Fusionsexperimente unter Verwendung der X63-Ag8-653 Myelomalinie (43) werden im wesentlichen entsprechend dem Verfahren von Köhler und Milstein (44) durchgeführt, indem man $10^8$ Milzzellen mit $10^7$ Myelomazellen unter Verwendung von 1 ml 50%igem Polyäthylenglykol (PEG 1500, Serva) (45) vermischt. Nach dem Waschen werden die Zellen in 48 ml Standard Dulbecco's minimum essential medium (Gibco) resuspendiert. 15% fötales Kalbsserum und $3 \times 10^6$ normale peritoneale Mäuseexsudatzellen/Fusion werden als Feederzellen zugegeben. Die Zellen werden in 48 × 1 ml Costarvertiefungen verteilt. Die Kulturen werden zweimal täglich mit Standard Selektivmedium (44) während 3 bis 6 Wochen gefüttert. Nach dem Wachstum der Hybriden werden diese gefroren und die Überstände werden auf die Anti-IFN-aktivität, wie im folgenden beschrieben, untersucht. Die Klonierung der Hybridoma-Zellen erfolgt durch Begrenzung der Verdünnung in Mikrotiterplatten.

C. *Antikörperassays*

Zur Prüfung der Anti-IFN-Aktivität des Überstands werden 50 µl IFN-α (am Ende 10 bis 20 Einheiten IFN/ml) mit 50 µl Kulturüberstand bei Zimmertemperatur inkubiert, und 30 bis 60 min später wird die Restaktivität an IFN in einem Standard IFN Assay getestet. Dieses Verfahren, das für die konventionellen Antikörper geeignet ist, versagt entweder oder ergibt keine reproduzierbaren Ergebnisse bei der Analyse der Hybridoma-Überstände. Zu diesem Zweck wurde daher der folgende, kombinierte Immuno-

präzipitations-Bioassay entwickelt. 50 µl rohes IFN-α ($10^4$ E/ml) werden (im Mikroröhrchen 3810, Eppendorf) mit gleichen Mengen Kulturüberstand vermischt und das Gemisch wird 2 bis 4 h bei 37°C inkubiert. Dann werden 50 µl zuvor titrierter Kaninchen Anti-Maus Ig-Antikörper (Nordic) zugesetzt und das Gemisch wird zuerst 1 h bei 37°C und dann 16 h bei +4°C für die Bildung der Immunkomplexe inkubiert. Die Röhrchen werden 5 min mit 12 000 U/min in einem kalten Raum zentrifugiert. Der Überstand wird gesammelt und das Präzipitat einmal mit 1 ml gepufferter Salzlösung (pH 7,2) gewaschen. Nach dem Waschen wird das Präzipitat in 200 µl Salzlösung (pH 2,2) gelöst. Entsprechend Armstrong (19) wird auf die IFN-Aktivität geprüft.

D. *Reinigung des Anti-IFN-Antikörpers, isoliert aus aszitischer Flüssigkeit*

Balb/c Mäuse werden intraperitoneal mit 0,4 ml Pristan (Carl Roth) vorbehandelt. 1 Woche später werden die Mäuse i.p. mit 2 bis 5 × $10^6$ Hybridoma-Zellen injiziert. Aszitische Flüssigkeit wird wiederholt von jeder Maus gesammelt. Die flüssigen Materialien werden gesammelt und bei −80°C gefroren. Nach dem Auftauen wird das gesammelte Material 30 min bei 16 000 U/min zentrifugiert. Das Fett am oberen Teil wird abgesaugt und der Debris-freie Überstand gesammelt. Erforderlichenfalls wird das Zentrifugieren wiederholt. Eine rohe Immunoglobulin-Fraktion wird aus der aszitischen Flüssigkeit durch 18%ige $Na_2SO_4$ Präzipitation bei Zimmertemperatur erhalten. Danach wird diese Fraktion durch Sephacryl G 200 (Pharmacia) gemäß den Angaben des Herstellers unter Verwendung von 0,1 M Tris-HCl-Puffer (pH 8,2)-geleitet. Die aktiven Fraktionen werden gesammelt und mit Amicon XM 50 Filtern (Amicon) konzentriert. Die Proteinbestimmung erfolgt durch $OD_{280}$-Messung, unter der Annahme, daß 1 mg Protein eine Absorption von 1,2 bei 280 nm in einer 1 cm Küvette ergibt.

E. *Immunoadsorbens-Säule 1K2-20*

1 ml abgesetztes Affi-Gel (Bio-Rad) wird mit 15 mg Immunoglobulin des monoklonalen Anti-IFN-Antikörpers gemäß den Angaben des Herstellers gekuppelt: Affi-Gel 10 wird auf einen Glasfrittentrichter zuerst mit kaltem destilliertem Wasser und dann mit 0,1 M $NaHCO_3$-Lösung (pH 8,0) (Kupplungspuffer) gewaschen. 50% des Gels im Kupplungspuffer werden auf ein Plastikrohr übertragen, mit den gleichen Mengen gereinigter Antikörperlösung vermischt und 4 h bei Zimmertemperatur rotiert. Nach dem Kuppeln wird das Gel mit Kupplungspuffer gewaschen. Zur Blockierung der nichtumgesetzten Stellen wird es mit 0,1 ml 1M Äthanolamin-HCl (pH 8,0)/ml Gel während 1 bis 2 h bei Zimmertemperatur behandelt. Das Gel wird mit Phosphat-gepufferter Salzlösung in Anwesenheit von 10 mM $NaN_3$ gewaschen und darin bei 4°C gehalten. 0,8 ml des entstehenden Gels werden zur Herstellung der monoklonalen Antikörpersäule (mit 1K2-20 bezeichnet) verwendet, die zur Herstellung von humanem LyIFN (vergl. oben) eingesetzt wird.

10. Pharmazeutische Zubereitungen (parenterale Verabreichung)

2 mg Lymphoblastoid-Interferon, z.B. LyIFN-α-3, isoliert aus dem Klon *E. coli* HB 101 CG-pBR(AP)/LyIFN-α-3 (vergl. Beispiel 9), mit einer spezifischen Aktivität von 1,8 × $10^8$ E/mg werden in 30 ml 5N humanem Serumalbumin gelöst. Die entstehende Lösung wird durch ein bakteriologisches Filter geleitet und die filtrierte Lösung unter aseptischen Bedingungen in 100 Ampullen, die je 3,6 × $10^6$ Einheiten reines Lymphoblastoid-Interferon enthalten, geteilt. Die für die parenterale Verabreichung geeigneten Ampullen werden bevorzugt im Kalten, z.B. bei −20°C, gelagert.

Auf gleiche Weisen können Ampullen mit einem Gehalt an 7,2 × $10^6$ oder 1,08 × $10^7$ Einheiten unter Verwendung von 4 bzw. 6 mg des obigen Lymphoblastoid-Interferons hergestellt werden.

Hinterlegung der hergestellten Mikroorganismen

Als Beispiele für die Mikroorganismen und rekombinanten DNA-Moleküle, die nach den erfindungsgemäßen Verfahren hergestellt wurden, wurden in der Sammelstelle der Agricultural Research Culture Collection (NRRL) am 14. September 1981 Kulturen hinterlegt, denen die folgenden Hinterlegungsnummern zugeordnet wurden:

| | |
|---|---|
| *E. coli* HB 101 CG-pBR 322/HLycIFN-$β_1$: | NRRL B-12528 |
| *E. coli* HB 101 CG-pBR 322/HLycIFN-$4_1$: | NRRL B-12529 |
| *E. coli* HB 101 CG-pBR 322/HLycIFN-1'b: | NRRL B-12530 |
| *E. coli* HB 101 CG-pBR 322/HLycIFN-$5_1$: | NRRL B-12531 |
| *E. coli* HB 101 CG-pBR 322/HLycIFN-$8_1'$: | NRRL B-12532 |

Literaturstellen

1. W. E. Stewart, II, The Interferon System, Springer Verlag, Vienna (1979).

2. E. A. Havell et al., "Characteristics of Human Lymphoblastoid (Namalva) Interferon", J. Gen. Virol. *38*, 51—59 (1977).

3. A. D. Sagar et al., "Heterogeneity of interferon mRNA species from Sendai virus-induced human lymphoblastoid (Namalva) cells and Newcastle disease virus-induced murin fibroblastoid (L) cells", Nucl. Acids Res. *9*, 149—160 (1981).

4. M. Rubenstein et al., "Human Leukocyte Interferon: Production, Purification to Homogeneity and Initial Characterization", Proc. Natl. Acad. Sci. USA *76*, 640—644 (1979).

5. W. E. Steward, II et al., "Effect of Glycosylation Inhibitors on the Production and Properties of Human Leukocyte Interferon", Virology *97*, 473—476 (1979).

6. K. C. Zoon et al., "Amino Terminal Sequence of the Major Component of Human Lymphoblastoid Interferon", Science *207*, 527—528 (1980).

7. P. B. Sehgal et al., "Heterogeneity of poly(I)·poly(C)-induced human fibroblast interferon in RNA species", Nature *288*, 95—97 (1980).

8. J. Weissenbach et al., "Two interferon mRNAs in human fibroblasts: In vitro translation and Escherichia coli cloning studies", Proc. Natl. Acad. Sci. USA *77*, 7152—7156 (1980).

9. S. N. Cohen and H. W. Boyer, "Process for Producing Biologically Functional Molecular Chimeras", U.S. patent No. 4,237,224 (Leland Stanford Jr. University).

10. C. Weissmann, "DNA Sequences, recombinant DNA molecules and processes for producing human interferon-like polypeptides", European patent application No. 32134 (Biogen N.V.).

11. S. Nagata et al., "Synthesis in E. coli of a polypeptide with human leukocyte interferon activity", Nature *284*, 316—320 (1980).

12. N. Mantei et al., "The nucleotide sequence of a cloned human leukocyte interferon cDNA", Gene *10*, 1—10 (1980).

13. M. Streuli et al., "At least Three Human Type α Interferons: Structure of α2", Science *209*, 1343—1347 (1980).

14. D. V. Goeddel et al, "Human leukocyte interferon produced by E. coli is biologically active", Nature *287*, 411—416 (1980).

15. D. V. Goeddel et al., "The structure of eight distinct cloned human leukocyte interferon cDNAs", Nature *290*, 20—26 (1981).

16. J. Groneberg et al., "Mikrobiologisch hergestelltes Polypeptid mit der Aminosäuresequenz des menschlichen Interferons, DNA und Plasmide, die für diese Sequenz codieren, Mikroorganismen, die diese genetische Information enthalten, und Verfahren zu deren Herstellung", European patent application No. 34307 (Hoechst Aktiengesellschaft).

17. M. D. Johnston et al., "Factors influencing production of interferon by human lymphoblastoid cells", Adv. Exp. Med. Biol. *110*, 61—74 (1978).

18. H. Strander et al., "Production of human lymphoblastoid interferon", J. Clin. Microbiol. *1*, 116—117 (1975).

19. J. A. Armstrong, "Semi-Micro Dye-Binding Assay for Rabbit Interferon", Appl. Microbiol, *21*, 723—725 (1971).

20. W. E. Stewart, II et al., "Interferon Production in Hamsters Experimentally Infected With Rabies Virus", Proc. Soc. Exp. Biol. Med. *123*, 650—653 (1966).

21. H. Sugano et al., "Novel DNA, cloned DNA, recombinant plasmid containing the DNA, microorganism containing the recombinant plasmid and process for their production", European patent application No. 28033 (Japanese Foundation of Cancer Research).

22. R. Derynck et al., "Isolation and structure of a human fibroblast interferon gene", Nature *285*, 542—547 (1980).

23. J. G. Sutcliffe, "pBR 322 restriction map derived from the DNA sequence: accurate DNA size markers up to 4361 nucleotide pairs long", Nucl. Acids Res. *5*, 2721—2728 (1978).

24. M. Mandel et al., "Calcium-dependent Bacteriophage DNA Infection", J. Mol. Biol. *53*, 159—162 (1970).

25. M. Grunstein and D. S. Hogness, "Colony hybridization: A method for the isolation of cloned DNAs that contain a specific gene", Proc. Natl. Acad. Sci. *72*, 3961—3965 (1979).

26. T. Taniguchi et al., "Human leukocyte and fibroblast interferons are structurally related", Nature *285*, 547—549 (1980).

27. K. Itakura et al., "Chemical DNA Synthesis and Recombinant DNA studies", Science *209*, 1401—1405 (1980).

28. T. Staehelin et al., J. Biol. Chem. *256*, 9750—9754 (1981).

29. J. B. Gurdon, J. Embryol. Exp. Morph. *20*, 401—414 (1968).

30. Barth, J. Embryol. Exp. Morph. *7*, 210—222 (1959).

31. A. Colman et al., "Export of Proteins from Oocytes of Xenopus laevis", Cell *17*, 517—526 (1979).

32. A. Efstratiadis et al., Full Length and Discrete Partial Reverse Transcripts of Globin and Chorion mRNAs, Cell *4*, 367—378 (1975).

33. T. Maniatis et al., "Amplification and Characterization of a β-Globin Gene Synthesized in Vitro", Cell *8*, 163—182 (1976).

34. J. H. J. Hoeijmakers et al., "The isolation of plasmids containing DNA complementary to messenger RNA for variant surface glycoproteins of Trypanosoma brucei", Gene *8*, 391—417 (1980).

35. A. C. Peacock et al., "Resolution of Multiple Ribonucleic Acid Species by Polyacrylamide Gel Electrophoresis", Biochemistry *6*, 1818—1827 (1967).

36. K. Itakura et al., "Improved Triester Approach for the Synthesis of Pentadecathymidylic Acid", J. Am. Chem. Soc. *97*, 7327—7332 (1975).

37. J. F. M. de Rooij et al., "Synthesis of complementary DNA fragments via phosphotriester intermediates", Recl. Trav. Chim. Pays-Bas *98*, 537—548 (1979).

38. W. Mueller et al., "Site-directed Mutagenesis in DNA: Generation of Point Mutations in Cloned β Globin Complementary DNA at the Positions Corresponding to Amino Acids 121 to 123", J. Mol. Biol. *124*, 343—358 (1978).

39. A. M. Maxam and W. Gilbert, "A new method for sequencing DNA", Proc. Natl. Acad. Sci. USA *74*, 560—564 (1977); see also Meth. Enzym. *65*, 499—559 (1980).

40. T. Taniguchi et al., "The nucleotide sequence of human fibroblast interferon cDNA", Gene *10*, 11—15 (1980).

41. U. K. Laemmli, Nature *227*, 680—685 (1970).

42. B. J. Radola, "Electrophoresis", p. 79—94, Walter de Gruyter, Berlin—New York 1980.

43. J. F. Kearney et al., J. Immunolog. *123*, 1548 (1979).

44. G. Köhler and C. Milstein, Nature *256*, 459 (1975).

45. G. Galfre et al., Nature *266*, 550 (1977).

**Patentansprüche für die Vertragsstaaten: BE CH DE FR IT LI LU NL SE**

1. Eine rekombinante DNA, welche eine für ein lymphoblastoides α-Interferon kodierende DNA-Sequenz, ausgewählt aus der für HLycIFN-$5_1$ kodierenden DNA-Sequenz

ATGGCCTTGACTTTTTATTTACTGGTGGCCC

TAGTGGTGCTCAGCTACAAGTCATTCAGCTCTCTGGGCTGTGATCTGCCTCAGACTCACAGCCTGGG

TAACAGGAGGGCCTTGATACTCCTGGCACAAATGCGAAGAATCTCTCCTTTCTCCTGCCTGAAGGAC

AGACATGACTTTGAATTCCCCCAGGAGGAGTTTGATGATAAACAGTTCCAGAAGGCTCAAGCCATCT

CTGTCCTCCATGAGATGATCCAGCAGACCTTCAACCTCTTCAGCACAAAGGACTCATCTGCTGCTTT

GGATGAGACCCTTCTAGATGAATTCTACATCGAACTTGACCAGCAGCTGAATGACCTGGAG

TCCTGTGTGATGCAGGAAGTGGGGGTGATAGAGTCTCCCCTGATGTACGAGGACTCCATCCTGGCTG

TGAGGAAATACTTCCAAAGAATCACTCTATATCTGACAGAGAAGAAATACAGCTCTTGTGCCTGGGA

GGTTGTCAGAGCAGAAATCATGAGATCCTTCTCTTTATCAATCAACTTGCAAAAAAGATTGAAGAGT

AAGGAA

und der für LyIFN-α-2 kodierenden DNA-Sequenz

ATGTGTGATCTGCCTC

AGACTCACAGCCTGGGTAACAGGAGGGCCTTGATACTCCTGGCACAAATGCGAAGAATCTCTCCTT

TCTCCTGCCTGAAGGACAGACATGACTTTGAATTCCCCCAGGAGGAGTTTGATGATAAACAGTTCC

AGAAGGCTCAAGCCATCTCTGTCCTCCATGAGATGATCCAGCAGACCTTCAACCTCTTCAGCACAA

AGGACTCATCTGCTGCTTTGGATGAGACCCTTCTAGATGAATTCTACATCGAACTTGACCAGCAGC

TGAATGACCTGGAGTCCTGTGTGATGCAGGAAGTGGGGGTGATAGAGTCTCCCCTGATGTACGAGG

ACTCCATCCTGGCTGTGAGGAAATACTTCCAAAGAATCACTCTATATCTGACAGAGAAGAAATACA

GCTCTTGTGCCTGGGAGGTTGTCAGAGCAGAAATCATGAGATCCTTCTCTTTATCAATCAACTTGC

AAAAAAGATTGAAGAGTAAGGAA,

enthält.

2. Eine rekombinante DNA gemäss Anspruch 1, welche die für HLycIFN-$5_1$ kodierende DNA-Sequenz enthält.

3. Eine rekombinante DNA gemäss Anspruch 1, welche die für LyIFN-α-2 kodierende DNA-Sequenz enthält.

4. Eine rekombinante DNA gemäss Anspruch 1, dadurch gekennzeichnet, dass die DNA-Sequenz operativ an die Expressionskontrollsequenz des β-Lactamase-Gens gebunden ist.

5. Verfahren zur Herstellung ener rekombinanten DNA gemäss Anspruch 1, dadurch gekennzeichnet, dass man in einen Klonierungsvektor eine DNA-Sequenz, ausgewählt aus der für HLycIFN-$5_1$ und LyIFN-α-2 kondierenden DNA-Sequenz, einführt.

6. Ein mit mindestens einer rekombinanten DNA gemäss Anspruch 1 transformierter E. coli-Wirtsstamm.

7. Ein transformierter E. coli HB101-Wirtsstamm gemäss Anspruch 6.

8. Der transformierte Wirtsstamm E. coli HB 101 CG-pBR 322/HLycIFN-$5_1$ (NRRL B-12531) gemäss Anspruch 6.

9. Verfahren zur Herstellung eines transformierten E. coli-Wirtsstammes gemäss Anspruch 6, dadurch gekennzeichnet, dass man in den E. coli-Wirtsstamm eine rekombinante DNA gemäss Anspruch 1 einbringt.

10. Das lymphoblastoide α-Interferon HLycIFN-$5_1$ der Formel

MET ALA LEU THR PHE TYR LEU LEU VAL ALA LEU VAL VAL LEU SER TYR LYS
SER PHE SER SER LEU GLY CYS ASP LEU PRO GLN THR HIS SER LEU GLY ASN
ARG ARG ALA LEU ILE LEU LEU ALA GLN MET ARG ARG ILE SER PRO PHE SER
CYS LEU LYS ASP ARG HIS ASP PHE GLU PHE PRO GLN GLU GLU PHE ASP ASP
LYS GLN PHE GLN LYS ALA GLN ALA ILE SER VAL LEU HIS GLU MET ILE GLN
GLN THR PHE ASN LEU PHE SER THR LYS ASP SER SER ALA ALA LEU ASP GLU
THR LEU LEU ASP GLU PHE TYR ILE GLU LEU ASP GLN GLN LEU ASN ASP LEU
GLU SER CYS VAL MET GLN GLU VAL GLY VAL ILE GLU SER PRO LEU MET TYR
GLU ASP SER ILE LEU ALA VAL ARG LYS TYR PHE GLN ARG ILE THR LEU TYR
LEU THR GLU LYS LYS TYR SER SER CYS ALA TRP GLU VAL VAL ARG ALA GLU
ILE MET ARG SER PHE SER LEU SER ILE ASN LEU GLN LYS ARG LEU LYS SER
LYS GLU.

11. Das lymphoblastoide α-Interferon LyIFN-α-2, dadurch gekennzeichnet, dass es von einem transformierten E. coli-Wirtsstamm, der eine rekombinante DNA enthaltend die DNA-Sequenz

ATGTGTGATCTGCCTC
AGACTCACAGCCTGGGTAACAGGAGGGCCTTGATACTCCTGGCACAAATGCGAAGAATCTCTCCTT
TCTCCTGCCTGAAGGACAGACATGACTTTGAATTCCCCCAGGAGGAGTTTGATGATAAACAGTTCC
AGAAGGCTCAAGCCATCTCTGTCCTCCATGAGATGATCCAGCAGACCTTCAACCTCTTCAGCACAA
AGGACTCATCTGCTGCTTTGGATGAGACCCTTCTAGATGAATTCTACATCGAACTTGACCAGCAGC
TGAATGACCTGGAGTCCTGTGTGATGCAGGAAGTGGGGGTGATAGAGTCTCCCCTGATGTACGAGG
ACTCCATCCTGGCTGTGAGGAAATACTTCCAAAGAATCACTCTATATCTGACAGAGAAGAAATACA
GCTCTTGTGCCTGGGAGGTTGTCAGAGCAGAAATCATGAGATCCTTCTCTTTATCAATCAACTTGC
AAAAAAGATTGAAGAGTAAGGAA

trägt, hergestellt wird.

12. Pharmazeutisches Präparat enthaltend ein lymphoblastoides α-Interferon gemäss einem der Ansprüche 10 oder 11.

13. Ein lymphoblastoides α-Interferon gemäss einem der Ansprüche 10 oder 11 zur Anwendung in einem Verfahren zur therapeutischen Behandlung des menschlichen Körpers.

14. Verfahren zur Herstellung eines lymphoblastoiden α-Interferons gemäss einem der Ansprüche 10 oder 11, dadurch gekennzeichnet, dass man einen transformierten E. coli-Wirtsstamm gemäss Anspruch 6 züchtet und das Interferon isoliert.

**Patentansprüche für den Vertragsstaat: AT**

1. Verfahren zur Herstellung einer rekombinanten DNA, welche eine für ein lymphoblastoides α-Interferon kodierende DNA-Sequenz, ausgewählt aus der für HLycIFN-$5_1$ kodierenden DNA-Sequenz

ATGGCCTTGACTTTTTATTTACTGGTGGCCC
TAGTGGTGCTCAGCTACAAGTCATTCAGCTCTCTGGGCTGTGATCTGCCTCAGACTCACAGCCTGGG
TAACAGGAGGGCCTTGATACTCCTGGCACAAATGCGAAGAATCTCTCCTTTCTCCTGCCTGAAGGAC
AGACATGACTTTGAATTCCCCCAGGAGGAGTTTGATGATAAACAGTTCCAGAAGGCTCAAGCCATCT
CTGTCCTCCATGAGATGATCCAGCAGACCTTCAACCTCTTCAGCACAAAGGACTCATCTGCTGCTTT
GGATGAGACCCTTCTAGATGAATTCTACATCGAACTTGACCAGCAGCTGAATGACCTGGAG
TCCTGTGTGATGCAGGAAGTGGGGGTGATAGAGTCTCCCCTGATGTACGAGGACTCCATCCTGGCTG
TGAGGAAATACTTCCAAAGAATCACTCTATATCTGACAGAGAAGAAATACAGCTCTTGTGCCTGGGA
GGTTGTCAGAGCAGAAATCATGAGATCCTTCTCTTTATCAATCAACTTGCAAAAAAGATTGAAGAGT
AAGGAA

und der für LyIFN-α-2 kodierenden DNA-Sequenz

ATGTGTGATCTGCCTC
AGACTCACAGCCTGGGTAACAGGAGGGCCTTGATACTCCTGGCACAAATGCGAAGAATCTCTCCTT
TCTCCTGCCTGAAGGACAGACATGACTTTGAATTCCCCCAGGAGGAGTTTGATGATAAACAGTTCC
AGAAGGCTCAAGCCATCTCTGTCCTCCATGAGATGATCCAGCAGACCTTCAACCTCTTCAGCACAA
AGGACTCATCTGCTGCTTTGGATGAGACCCTTCTAGATGAATTCTACATCGAACTTGACCAGCAGC
TGAATGACCTGGAGTCCTGTGTGATGCAGGAAGTGGGGGTGATAGAGTCTCCCCTGATGTACGAGG
ACTCCATCCTGGCTGTGAGGAAATACTTCCAAAGAATCACTCTATATCTGACAGAGAAGAAATACA
GCTCTTGTGCCTGGGAGGTTGTCAGAGCAGAAATCATGAGATCCTTCTCTTTATCAATCAACTTGC
AAAAAAGATTGAAGAGTAAGGAA,

enthält, dadurch gekennzeichnet, dass man die DNA-Sequenz in einen Klonierungsvektor einführt.

2. Verfahren gemäss Anspruch 1 zur Herstellung einer rekombinanten DNA, welche die für HLycIFN-$5_1$ kodierende DNA-Sequenz enthält.

3. Verfahren gemäss Anspruch 1 zur Herstellung einer rekombinanten DNA, welche die für LyIFN-α-2 kodierende DNA-Sequenz enthält.

4. Verfahren gemäss Anspruch 1, dadurch gekennzeichnet, dass die DNA-Sequenz derart in einen Klonierungsvektor eingeführt wird, dass sie operativ an die Expressionskontrollsequenz des β-Lactamase-Gens gebunden ist.

5. Ein mit mindestens einer gemäss Anspruch 1 erhältlichen rekombinanten DNA transformierter E.coli-Wirtsstamm.

6. Ein transformierter E. coli HB101-Wirtsstamm gemäss Anspruch 5.

7. Der transformierte Wirtsstamm E. coli HB101 CG-pBR 322/HLycIFN-$5_1$ (NRRL B-12531) gemäss Anspruch 5.

8. Verfahren zur Herstellung des lymphoblastoiden α-Interferons HLycIFN-$5_1$ der Formel

MET ALA LEU THR PHE TYR LEU LEU VAL ALA LEU VAL VAL LEU SER TYR LYS
SER PHE SER SER LEU GLY CYS ASP LEU PRO GLN THR HIS SER LEU GLY ASN
ARG ARG ALA LEU ILE LEU LEU ALA GLN MET ARG ARG ILE SER PRO PHE SER
CYS LEU LYS ASP ARG HIS ASP PHE GLU PHE PRO GLN GLU GLU PHE ASP ASP
LYS GLN PHE GLN LYS ALA GLN ALA ILE SER VAL LEU HIS GLU MET ILE GLN

GLN THR PHE ASN LEU PHE SER THR LYS ASP SER SER ALA ALA LEU ASP GLU
THR LEU LEU ASP GLU PHE TYR ILE GLU LEU ASP GLN GLN LEU ASN ASP LEU
GLU SER CYS VAL MET GLN GLU VAL GLY VAL ILE GLU SER PRO LEU MET TYR
GLU ASP SER ILE LEU ALA VAL ARG LYS TYR PHE GLN ARG ILE THR LEU TYR
LEU THR GLU LYS LYS TYR SER SER CYS ALA TRP GLU VAL VAL ARG ALA GLU
ILE MET ARG SER PHE SER LEU SER ILE ASN LEU GLN LYS ARG LEU LYS SER
LYS GLU,

dadurch gekennzeichnet, dass man einen transformierten E.coli-Wirtsstamm, der eine gemäss Anspruch 2 erhältliche rekombinante DNA-Sequenz trägt, züchtet und das Interferon isoliert.

9. Verfahren zur Herstellung des lymphoblastoiden α-Interferons LyIFN-α-2, dadurch gekennzeichnet, dass man einen transformierten E.coli-Wirtsstamm, der eine rekombinante DNA enthaltend die DNA-Sequenz

ATGTGTGATCTGCCTC
AGACTCACAGCCTGGGTAACAGGAGGGCCTTGATACTCCTGGCACAAATGCGAAGAATCTCTCCTT
TCTCCTGCCTGAAGGACAGACATGACTTTGAATTCCCCCAGGAGGAGTTTGATGATAAACAGTTCC
AGAAGGCTCAAGCCATCTCTGTCCTCCATGAGATGATCCAGCAGACCTTCAACCTCTTCAGCACAA
AGGACTCATCTGCTGCTTTGGATGAGACCCTTCTAGATGAATTCTACATCGAACTTGACCAGCAGC
TGAATGACCTGGAGTCCTGTGTGATGCAGGAAGTGGGGGTGATAGAGTCTCCCCTGATGTACGAGG
ACTCCATCCTGGCTGTGAGGAAATACTTCCAAAGAATCACTCTATATCTGACAGAGAAGAAATACA
GCTCTTGTGCCTGGGAGGTTGTCAGAGCAGAAATCATGAGATCCTTCTCTTTATCAATCAACTTGC
AAAAAAGATTGAAGAGTAAGGAA

trägt, züchtet und das Interferon isoliert.

10. Verfahren zur Herstellung eines pharmazeutischen Präparats, dadurch gekennzeichnet, dass man ein gemäss einem der Ansprüche 8 oder 9 erhältliches lymphoblastoides α-Interferon mit einem pharmazeutisch annehmbaren Trägerstoff mischt.

**Revendications pour les Etats contractants: BE CH DE FR IT LI LU NL SE**

1. Un ADN recombinant contenant une séquence d'ADN codant pour interféron α lymphoblastoïde, choisi entre la séquence d'ADN codant pur HLycIFN-$5_1$

ATGGCCTTGACTTTTTATTTACTGGTGGCCC
TAGTGGTGCTCAGCTACAAGTCATTCAGCTCTCTGGGCTGTGATCTGCCTCAGACTCACAGCCTGGG
TAACAGGAGGGCCTTGATACTCCTGGCACAAATGCGAAGAATCTCTCCTTTCTCCTGCCTGAAGGAC
AGACATGACTTTGAATTCCCCCAGGAGGAGTTTGATGATAAACAGTTCCAGAAGGCTCAAGCCATCT
CTGTCCTCCATGAGATGATCCAGCAGACCTTCAACCTCTTCAGCACAAAGGACTCATCTGCTGCTTT
GGATGAGACCCTTCTAGATGAATTCTACATCGAACTTGACCAGCAGCTGAATGACCTGGAG
TCCTGTGTGATGCAGGAAGTGGGGGTGATAGAGTCTCCCCTGATGTACGAGGACTCCATCCTGGCTG
TGAGGAAATACTTCCAAAGAATCACTCTATATCTGACAGAGAAGAAATACAGCTCTTGTGCCTGGGA
GGTTGTCAGAGCAGAAATCATGAGATCCTTCTCTTTATCAATCAACTTGCAAAAAAGATTGAAGAGT
AAGGAA

et la séquence d'ADN condant pur LyIFN-α-2

ATGTGTGATCTGCCTC

AGACTCACAGCCTGGGTAACAGGAGGGCCTTGATACTCCTGGCACAAATGCGAAGAATCTCTCCTT

TCTCCTGCCTGAAGGACAGACATGACTTTGAATTCCCCCAGGAGGAGTTTGATGATAAACAGTTCC

AGAAGGCTCAAGCCATCTCTGTCCTCCATGAGATGATCCAGCAGACCTTCAACCTCTTCAGCACAA

AGGACTCATCTGCTGCTTTGGATGAGACCCTTCTAGATGAATTCTACATCGAACTTGACCAGCAGC

TGAATGACCTGGAGTCCTGTGTGATGCAGGAAGTGGGGGTGATAGAGTCTCCCCTGATGTACGAGG

ACTCCATCCTGGCTGTGAGGAAATACTTCCAAAGAATCACTCTATATCTGACAGAGAAGAAATACA

GCTCTTGTGCCTGGGAGGTTGTCAGAGCAGAAATCATGAGATCCTTCTCTTTATCAATCAACTTGC

AAAAAAGATTGAAGAGTAAGGAA.

2. Un ADN recombinant selon la revendication 1, contenant la séquence d'ADN codant pour HLycIFN-$5_1$.

3. Un ADN recombinant selon la revendication 1, contenant la séquence d'ADN codant pour LyIFN-α-2.

4. Un ADN recombinant selon la revendication 1, caractérisé en ce que la séquence d'ADN est liée, de façon opérative, à la séquence de contrôle de l'expressioon du gène de la β-lactamase.

5. Procédé pour la préparation d'un ADN recombinant selon la revendication 1, caractérisé en ce que l'on insère dans un vecteur de clonage une séquence d'ADN choisie entre la séquence d'ADN codant pour HLyIFN-$5'_1$ et la séquence d'ADN codant pour LyIFN-α-2.

6. Une souche hôte d'*E. coli* transformée avec au moins un ADN recombinant selon la revendication 1.

7. Une souche hôte E. coli HB101 transformée selon la revendication 6.

8. La souche hôte transformée E. coli HB101 CG-pBR322/HLycIFN-$5_1$ (NRRL B12531) selon la revendication 6.

9. Procédé pour la préparation d'une souche hôte d'E. coli transformée selon la revendication 6, caractérisé en ce que l'on insère dans la souche hôte d'E. coli un ADN recombinant selon la revendication 1.

10. L'interféron α HLyIFN-$5_1$ de formule

MET ALA LEU THR PHE TYR LEU LEU VAL ALA LEU VAL VAL LEU SER TYR LYS

SER PHE SER SER LEU GLY CYS ASP LEU PRO GLN THR HIS SER LEU GLY ASN

ARG ARG ALA LEU ILE LEU LEU ALA GLN MET ARG ARG ILE SER PRO PHE SER

CYS LEU LYS ASP ARG HIS ASP PHE GLU PHE PRO GLN GLU GLU PHE ASP ASP

LYS GLN PHE GLN LYS ALA GLN ALA ILE SER VAL LEU HIS GLU MET ILE GLN

GLN THR PHE ASN LEU PHE SER THR LYS ASP SER SER ALA ALA LEU ASP GLU

THR LEU LEU ASP GLU PHE TYR ILE GLU LEU ASP GLN GLN LEU ASN ASP LEU

GLU SER CYS VAL MET GLN GLU VAL GLY VAL ILE GLU SER PRO LEU MET TYR

GLU ASP SER ILE LEU ALA VAL ARG LYS TYR PHE GLN ARG ILE THR LEU TYR

LEU THR GLU LYS LYS TYR SER SER CYS ALA TRP GLU VAL VAL ARG ALA GLU

ILE MET ARG SER PHE SER LEU SER ILE ASN LEU GLN LYS ARG LEU LYS SER

LYS GLU.

11. L'interféron LyIFN-α-2 lymphoblastoïde, caractérisé en ce qu'il est préparé à partir d'une souche hôte d'E. coli transformée, portant un ADN recombinant contenant la séquence d'ADN.

ATGTGTGATCTGCCTC

AGACTCACAGCCTGGGTAACAGGAGGGCCTTGATACTCCTGGCACAAATGCGAAGAATCTCTCCTT

TCTCCTGCCTGAAGGACAGACATGACTTTGAATTCCCCCAGGAGGAGTTTGATGATAAACAGTTCC

AGAAGGCTCAAGCCATCTCTGTCCTCCATGAGATGATCCAGCAGACCTTCAACCTCTTCAGCACAA

AGGACTCATCTGCTGCTTTGGATGAGACCCTTCTAGATGAATTCTACATCGAACTTGACCAGCAGC

TGAATGACCTGGAGTCCTGTGTGATGCAGGAAGTGGGGGTGATAGAGTCTCCCCTGATGTACGAGG

ACTCCATCCTGGCTGTGAGGAAATACTTCCAAAGAATCACTCTATATCTGACAGAGAAGAAATACA

GCTCTTGTGCCTGGGAGGTTGTCAGAGCAGAAATCATGAGATCCTTCTCTTTATCAATCAACTTGC

AAAAAAGATTGAAGAGTAAGGAA.

12. Préparation pharmaceutique contenant un interféron α lymphoblastoïde selon l'une des revendications 10 ou 11.

13. Un interféron α lymphoblastoïde selon l'une des revendications 10 ou 11 destiné à être utilisé dans un procédé pour le traitement thérapeutique du corps humain.

14. Procédé pour la préparation d'un interféron α lymphoblastoïde selon l'une des revendications 10 ou 11, caractérisé en ce que l'on cultive une souche hôte d'E. coli transformée selon la revendication 6 et en ce que l'on isole l'interféron.

**Revendications pour l'Etat contractant: AT**

1. Procédé pour la préparation d'un ADN recombinant contenant une séquence d'ADN codant pour un interféron α lymphoblastoïde, choisi entre la séquence d'ADN codant pour HLycIFN-$5_1$.

ATGGCCTTGACTTTTTATTTACTGGTGGCCC

TAGTGGTGCTCAGCTACAAGTCATTCAGCTCTCTGGGCTGTGATCTGCCTCAGACTCACAGCCTGGG

TAACAGGAGGGCCTTGATACTCCTGGCACAAATGCGAAGAATCTCTCCTTTCTCCTGCCTGAAGGAC

AGACATGACTTTGAATTCCCCCAGGAGGAGTTTGATGATAAACAGTTCCAGAAGGCTCAAGCCATCT

CTGTCCTCCATGAGATGATCCAGCAGACCTTCAACCTCTTCAGCACAAAGGACTCATCTGCTGCTTT

GGATGAGACCCTTCTAGATGAATTCTACATCGAACTTGACCAGCAGCTGAATGACCTGGAG

TCCTGTGTGATGCAGGAAGTGGGGGTGATAGAGTCTCCCCTGATGTACGAGGACTCCATCCTGGCTG

TGAGGAAATACTTCCAAAGAATCACTCTATATCTGACAGAGAAGAAATACAGCTCTTGTGCCTGGGA

GGTTGTCAGAGCAGAAATCATGAGATCCTTCTCTTTATCAATCAACTTGCAAAAAAGATTGAAGAGT

AAGGAA

et la séquence d'ADN codant pour LyIFN-α-2

ATGTGTGATCTGCCTC

AGACTCACAGCCTGGGTAACAGGAGGGCCTTGATACTCCTGGCACAAATGCGAAGAATCTCTCCTT

TCTCCTGCCTGAAGGACAGACATGACTTTGAATTCCCCCAGGAGGAGTTTGATGATAAACAGTTCC

AGAAGGCTCAAGCCATCTCTGTCCTCCATGAGATGATCCAGCAGACCTTCAACCTCTTCAGCACAA

AGGACTCATCTGCTGCTTTGGATGAGACCCTTCTAGATGAATTCTACATCGAACTTGACCAGCAGC

TGAATGACCTGGAGTCCTGTGTGATGCAGGAAGTGGGGGTGATAGAGTCTCCCCTGATGTACGAGG

ACTCCATCCTGGCTGTGAGGAAATACTTCCAAAGAATCACTCTATATCTGACAGAGAAGAAATACA

GCTCTTGTGCCTGGGAGGTTGTCAGAGCAGAAATCATGAGATCCTTCTCTTTATCAATCAACTTGC

AAAAAAGATTGAAGAGTAAGGAA.

caractérisé en ce que l'on insère la séquence d'ADN dans un vecteur de clonage.

2. Procédé pour la préparation d'un ADN recombinant contenant la séquence d'ADN codant pour LycIFN-$5_1$.

3. Procédé pour la préparation d'un ADN recombinant selon la revendication 1, contenant la séquence d'ADN codant pour LyIFN-α-2.

4. Procédé selon la revendication 1, caractérisé en ce que la séquence d'ADN est insérée dans un vecteur de clonage de telle manière qu'elle est liée de façon opérative, à la séquence de contrôle de l'expression du gène de la -lactamase.

5. Une souche hôte d'E. coli transformée avec au moins un ADN recombinant obtenu selon la revendication 1.

6. Une souche hôte d'E. coli HB101 transformée selon la revendication 5.

7. La souche hôte d'E. coli HB101 CG-pBR322/HLycIFN-$5_1$ (NRRL B-12531) transformée selon la revendication 5.

8. Procédé pour la préparation de l'interféron α HLycIFN-5 lymphoblastoïde de formule

MET ALA LEU THR PHE TYR LEU LEU VAL ALA LEU VAL VAL LEU SER TYR LYS
SER PHE SER SER LEU GLY CYS ASP LEU PRO GLN THR HIS SER LEU GLY ASN
ARG ARG ALA LEU ILE LEU LEU ALA GLN MET ARG ARG ILE SER PRO PHE SER
CYS LEU LYS ASP ARG HIS ASP PHE GLU PHE PRO GLN GLU GLU PHE ASP ASP
LYS GLN PHE GLN LYS ALA GLN ALA ILE SER VAL LEU HIS GLU MET ILE GLN
GLN THR PHE ASN LEU PHE SER THR LYS ASP SER SER ALA ALA LEU ASP GLU
THR LEU LEU ASP GLU PHE TYR ILE GLU LEU ASP GLN GLN LEU ASN ASP LEU
GLU SER CYS VAL MET GLN GLU VAL GLY VAL ILE GLU SER PRO LEU MET TYR
GLU ASP SER ILE LEU ALA VAL ARG LYS TYR PHE GLN ARG ILE THR LEU TYR
LEU THR GLU LYS LYS TYR SER SER CYS ALA TRP GLU VAL VAL ARG ALA GLU
ILE MET ARG SER PHE SER LEU SER ILE ASN LEU GLN LYS ARG LEU LYS SER
LYS GLU,

caractérisé en ce que l'on cultive une souche hôte d'E. coli transformée, portant une séquence d'ADN recombinant obtenue selon la revendication 2.

9. Procédé pour la préparation de l'interféron α LyIFN-α-2 lymphoblastoïde, caractérisé en ce que l'on cultive une souche hôte d'E. coli transformée, portant un ADN recombinant contenant la séquence d'ADN

ATGTGTGATCTGCCTC
AGACTCACAGCCTGGGTAACAGGAGGGCCTTGATACTCCTGGCACAAATGCGAAGAATCTCTCCTT
TCTCCTGCCTGAAGGACAGACATGACTTTGAATTCCCCCAGGAGGAGTTTGATGATAAACAGTTCC
AGAAGGCTCAAGCCATCTCTGTCCTCCATGAGATGATCCAGCAGACCTTCAACCTCTTCAGCACAA
AGGACTCATCTGCTGCTTTGGATGAGACCCTTCTAGATGAATTCTACATCGAACTTGACCAGCAGC
TGAATGACCTGGAGTCCTGTGTGATGCAGGAAGTGGGGGTGATAGAGTCTCCCCTGATGTACGAGG
ACTCCATCCTGGCTGTGAGGAAATACTTCCAAAGAATCACTCTATATCTGACAGAGAAGAAATACA
GCTCTTGTGCCTGGGAGGTTGTCAGAGCAGAAATCATGAGATCCTTCTCTTTATCAATCAACTTGC
AAAAAAGATTGAAGAGTAAGGAA

et en ce que l'on isole l'interféron.

10. Procédé pour la préparation d'une préparation pharmaceutique, caractérisé en ce que l'on mélange un interféron lymphoblastoïde obtenu selon l'une des revendications 8 ou 9 avec un support pharmaceutiquement acceptable.

**Claims for the Contracting States: BE CH DE FR IT LI LU NL SE**

1. A recombinant DNA which contains a DNA sequence coding for a lymphoblastoid α-interferon and selected from the DNA sequence coding for HLycIFN-$5_1$

ATGGCCTTGACTTTTTATTTACTGGTGGCCC
TAGTGGTGCTCAGCTACAAGTCATTCAGCTCTCTGGGCTGTGATCTGCCTCAGACTCACAGCCTGGG
TAACAGGAGGGCCTTGATACTCCTGGCACAAATGCGAAGAATCTCTCCTTTCTCCTGCCTGAAGGAC
AGACATGACTTTGAATTCCCCCAGGAGGAGTTTGATGATAAACAGTTCCAGAAGGCTCAAGCCATCT
CTGTCCTCCATGAGATGATCCAGCAGACCTTCAACCTCTTCAGCACAAAGGACTCATCTGCTGCTTT
GGATGAGACCCTTCTAGATGAATTCTACATCGAACTTGACCAGCAGCTGAATGACCTGGAG
TCCTGTGTGATGCAGGAAGTGGGGGTGATAGAGTCTCCCCTGATGTACGAGGACTCCATCCTGGCTG
TGAGGAAATACTTCCAAAGAATCACTCTATATCTGACAGAGAAGAAATACAGCTCTTGTGCCTGGGA
GGTTGTCAGAGCAGAAATCATGAGATCCTTCTCTTTATCAATCAACTTGCAAAAAAGATTGAAGAGT
AAGGAA

and from the DNA sequence coding for LyIFN-α-2

ATGTGTGATCTGCCTC
AGACTCACAGCCTGGGTAACAGGAGGGCCTTGATACTCCTGGCACAAATGCGAAGAATCTCTCCTT
TCTCCTGCCTGAAGGACAGACATGACTTTGAATTCCCCCAGGAGGAGTTTGATGATAAACAGTTCC
AGAAGGCTCAAGCCATCTCTGTCCTCCATGAGATGATCCAGCAGACCTTCAACCTCTTCAGCACAA
AGGACTCATCTGCTGCTTTGGATGAGACCCTTCTAGATGAATTCTACATCGAACTTGACCAGCAGC
TGAATGACCTGGAGTCCTGTGTGATGCAGGAAGTGGGGGTGATAGAGTCTCCCCTGATGTACGAGG
ACTCCATCCTGGCTGTGAGGAAATACTTCCAAAGAATCACTCTATATCTGACAGAGAAGAAATACA
GCTCTTGTGCCTGGGAGGTTGTCAGAGCAGAAATCATGAGATCCTTCTCTTTATCAATCAACTTGC
AAAAAAGATTGAAGAGTAAGGAA.

2. A recombinant DNA according to claim 1, which contains the DNA sequence coding for HLycIFN-$5_1$.

3. A recombinant DNA according to claim 1, which contains the DNA sequence coding for LyIFN-α-2.

4. A recombinant DNA according to claim 1, wherein the DNA sequence is operatively linked to the expression control sequence of the β-lactamase gene.

5. A process for the preparation of a recombinant DNA according to claim 1, which comprises introducing into a cloning vector a DNA sequence selected from the DNA sequence coding for HLycIFN-$5_1$ and LyIFN-α-2.

An E. coli host strain transformed with at least one recombinant DNA according to claim 1.

7. A tranformed E. coli HB101 host strain according to claim 6.

8. The transformed host strain E. coli HB101 CG-pBR322/HLycIFN-$5_1$ (NRRL B-12531) according to claim 6.

9. A process for the preparation of a transformed E. coli host strain according to claim 6, which comprises inserting into the E. coli host strain a recombinant DNA according to claim 1.

10. The lymphoblastoid α-interferon HLycIFN-$5_1$ of formula

MET ALA LEU THR PHE TYR LEU LEU VAL ALA LEU VAL VAL LEU SER TYR LYS
SER PHE SER SER LEU GLY CYS ASP LEU PRO GLN THR HIS SER LEU GLY ASN
ARG ARG ALA LEU ILE LEU LEU ALA GLN MET ARG ARG ILE SER PRO PHE SER
CYS LEU LYS ASP ARG HIS ASP PHE GLU PHE PRO GLN GLU GLU PHE ASP ASP
LYS GLN PHE GLN LYS ALA GLN ALA ILE SER VAL LEU HIS GLU MET ILE GLN

GLN THR PHE ASN LEU PHE SER THR LYS ASP SER SER ALA ALA LEU ASP GLU

THR LEU LEU ASP GLU PHE TYR ILE GLU LEU ASP GLN GLN LEU ASN ASP LEU

GLU SER CYS VAL MET GLN GLU VAL GLY VAL ILE GLU SER PRO LEU MET TYR

GLU ASP SER ILE LEU ALA VAL ARG LYS TYR PHE GLN ARG ILE THR LEU TYR

LEU THR GLU LYS LYS TYR SER SER CYS ALA TRP GLU VAL VAL ARG ALA GLU

ILE MET ARG SER PHE SER LEU SER ILE ASN LEU GLN LYS ARG LEU LYS SER

LYS GLU.

11. The lymphoblastoid α-interferon LyIFN-α-2 which is prepared from a transformed E. coli host strain which carries a recombinant DNA containing the DNA sequence

ATGTGTGATCTGCCTC

AGACTCACAGCCTGGGTAACAGGAGGGCCTTGATACTCCTGGCACAAATGCGAAGAATCTCTCCTT

TCTCCTGCCTGAAGGACAGACATGACTTTGAATTCCCCCAGGAGGAGTTTGATGATAAACAGTTCC

AGAAGGCTCAAGCCATCTCTGTCCTCCATGAGATGATCCAGCAGACCTTCAACCTCTTCAGCACAA

AGGACTCATCTGCTGCTTTGGATGAGACCCTTCTAGATGAATTCTACATCGAACTTGACCAGCAGC

TGAATGACCTGGAGTCCTGTGTGATGCAGGAAGTGGGGGTGATAGAGTCTCCCCTGATGTACGAGG

ACTCCATCCTGGCTGTGAGGAAATACTTCCAAAGAATCACTCTATATCTGACAGAGAAGAAATACA

GCTCTTGTGCCTGGGAGGTTGTCAGAGCAGAAATCATGAGATCCTTCTCTTTATCAATCAACTTGC

AAAAAAGATTGAAGAGTAAGGAA.

12. A pharmaceutical composition containing a lymphoblastoid α-interferon according to either claim 10 or claim 11.

13. A lymphoblastoid α-interferon according to either claim 10 or claim 11 for use in a method of therapeutic treatment of the human body.

14. A process for the preparation of lymphoblastoid α-interferon according to either claim 10 or claim 11, which comprises culturing a transformed E. coli host strain according to claim 6 and isolating the interferon.

**Claims for the Contracting State: AT**

1. A process for the preparation of recombinant DNA which contains a DNA sequence coding for a lymphoblastoid α-interferon and selected from the DNA sequence coding for $HLycIFN\text{-}5_1$

ATGGCCTTGACTTTTTATTTACTGGTGGCCC

TAGTGGTGCTCAGCTACAAGTCATTCAGCTCTCTGGGCTGTGATCTGCCTCAGACTCACAGCCTGGG

TAACAGGAGGGCCTTGATACTCCTGGCACAAATGCGAAGAATCTCTCCTTTCTCCTGCCTGAAGGAC

AGACATGACTTTGAATTCCCCCAGGAGGAGTTTGATGATAAACAGTTCCAGAAGGCTCAAGCCATCT

CTGTCCTCCATGAGATGATCCAGCAGACCTTCAACCTCTTCAGCACAAAGGACTCATCTGCTGCTTT

GGATGAGACCCTTCTAGATGAATTCTACATCGAACTTGACCAGCAGCTGAATGACCTGGAG

TCCTGTGTGATGCAGGAAGTGGGGGTGATAGAGTCTCCCCTGATGTACGAGGACTCCATCCTGGCTG

TGAGGAAATACTTCCAAAGAATCACTCTATATCTGACAGAGAAGAAATACAGCTCTTGTGCCTGGGA

GGTTGTCAGAGCAGAAATCATGAGATCCTTCTCTTTATCAATCAACTTGCAAAAAAGATTGAAGAGT

AAGGAA

and from the DNA sequence coding for LyIFN-α-2

ATGTGTGATCTGCCTC

AGACTCACAGCCTGGGTAACAGGAGGGCCTTGATACTCCTGGCACAAATGCGAAGAATCTCTCCTT
TCTCCTGCCTGAAGGACAGACATGACTTTGAATTCCCCCAGGAGGAGTTTGATGATAAACAGTTCC
AGAAGGCTCAAGCCATCTCTGTCCTCCATGAGATGATCCAGCAGACCTTCAACCTCTTCAGCACAA
AGGACTCATCTGCTGCTTTGGATGAGACCCTTCTAGATGAATTCTACATCGAACTTGACCAGCAGC
TGAATGACCTGGAGTCCTGTGTGATGCAGGAAGTGGGGGTGATAGAGTCTCCCCTGATGTACGAGG
ACTCCATCCTGGCTGTGAGGAAATACTTCCAAAGAATCACTCTATATCTGACAGAGAAGAAATACA
GCTCTTGTGCCTGGGAGGTTGTCAGAGCAGAAATCATGAGATCCTTCTCTTTATCAATCAACTTGC
AAAAAAGATTGAAGAGTAAGGAA.

which comprises introducing the DNA sequence into a cloning vector.

2. A process according to claim 1 for the preparation of a recombinant DNA which contains the DNA sequence coding for HLycIFN-$5_1$.

3. A process according to claim 1 for the preparation of a recombinant DNA which contains the DNA sequence coding for LyIFN-α-2.

4. A process according to claim 1, which comprises introducing the DNA sequence into a cloning vector such that it is operatively linked to the expression control sequence of the β-lactamase gene.

5. An E. coli host strain transformed with at least one recombinant DNA which is obtainable according to claim 1.

6. A transformed E. coli HB101 host strain according to claim 5.

7. The transformed host strain E. coli HB101 CG-pBR322/HLycIFN-$5_1$ (NRRL B-12531) according to claim 5.

8. A process for the preparation of the lymphoblastoid α-interferon HLycIFN-$5_1$ of formula

MET ALA LEU THR PHE TYR LEU LEU VAL ALA LEU VAL VAL LEU SER TYR LYS
SER PHE SER SER LEU GLY CYS ASP LEU PRO GLN THR HIS SER LEU GLY ASN
ARG ARG ALA LEU ILE LEU LEU ALA GLN MET ARG ARG ILE SER PRO PHE SER
CYS LEU LYS ASP ARG HIS ASP PHE GLU PHE PRO GLN GLU GLU PHE ASP ASP
LYS GLN PHE GLN LYS ALA GLN ALA ILE SER VAL LEU HIS GLU MET ILE GLN
GLN THR PHE ASN LEU PHE SER THR LYS ASP SER SER ALA ALA LEU ASP GLU
THR LEU LEU ASP GLU PHE TYR ILE GLU LEU ASP GLN GLN LEU ASN ASP LEU
GLU SER CYS VAL MET GLN GLU VAL GLY VAL ILE GLU SER PRO LEU MET TYR
GLU ASP SER ILE LEU ALA VAL ARG LYS TYR PHE GLN ARG ILE THR LEU TYR
LEU THR GLU LYS LYS TYR SER SER CYS ALA TRP GLU VAL VAL ARG ALA GLU
ILE MET ARG SER PHE SER LEU SER ILE ASN LEU GLN LYS ARG LEU LYS SER
LYS GLU,

which comprises culturing a transformed E. coli host strain which carries a recombinant DNA sequence which is obtainable according to claim 2 and isolating the interferon.

9. A process for the preparation of the lymphoblastoid α-interferon LyIFN-α-2, which comprises culturing a transformed E. coli host strain which carries a recombinant DNA containing the DNA sequence

ATGTGTGATCTGCCTC
AGACTCACAGCCTGGGTAACAGGAGGGCCTTGATACTCCTGGCACAAATGCGAAGAATCTCTCCTT
TCTCCTGCCTGAAGGACAGACATGACTTTGAATTCCCCCAGGAGGAGTTTGATGATAAACAGTTCC
AGAAGGCTCAAGCCATCTCTGTCCTCCATGAGATGATCCAGCAGACCTTCAACCTCTTCAGCACAA
AGGACTCATCTGCTGCTTTGGATGAGACCCTTCTAGATGAATTCTACATCGAACTTGACCAGCAGC

TGAATGACCTGGAGTCCTGTGTGATGCAGGAAGTGGGGGTGATAGAGTCTCCCCTGATGTACGAGG

ACTCCATCCTGGCTGTGAGGAAATACTTCCAAAGAATCACTCTATATCTGACAGAGAAGAAATACA

GCTCTTGTGCCTGGGAGGTTGTCAGAGCAGAAATCATGAGATCCTTCTCTTTATCAATCAACTTGC

AAAAAAGATTGAAGAGTAAGGAA

and isolating the interferon.

10. A process for the preparation of a pharmaceutical composition, which comprises mixing a lymphoblastoid α-interferon which is obtainable according to either claim 8 or claim 9 with a pharmaceutically acceptable excipient.

NAMALWA ds cDNA-SYNTHESE UND AUFBAU REKOMBINANTER DNA-MOLEKÜLE

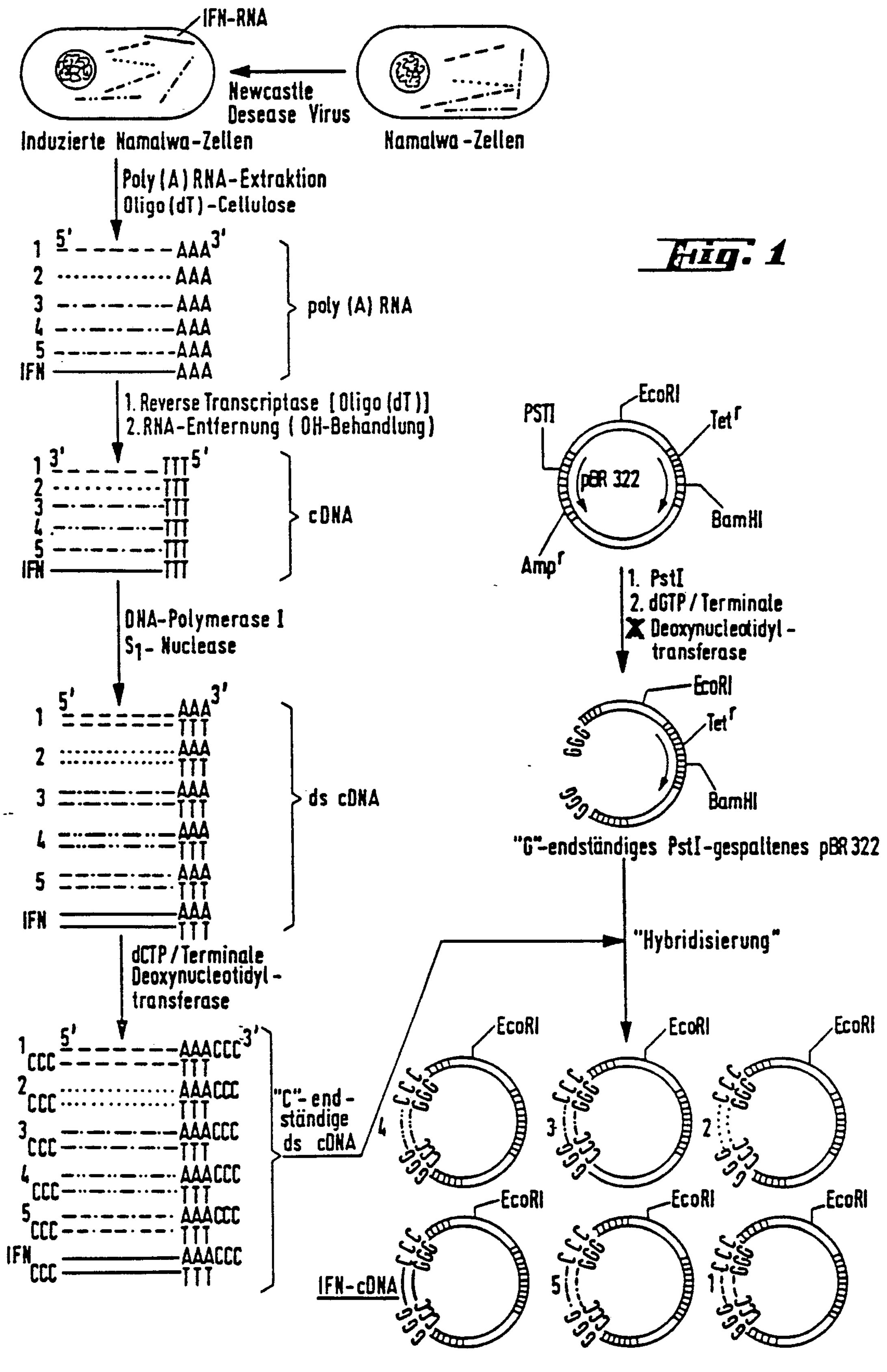


Fig. 1

**Fig. 2**

SYNTHESE DES DNA-PRIMERS (13-mer) KOMPLEMENTÄR ZU $\alpha_1$-UND β-IFN mRNA

Zeile

1

2

3

4

5

6

7

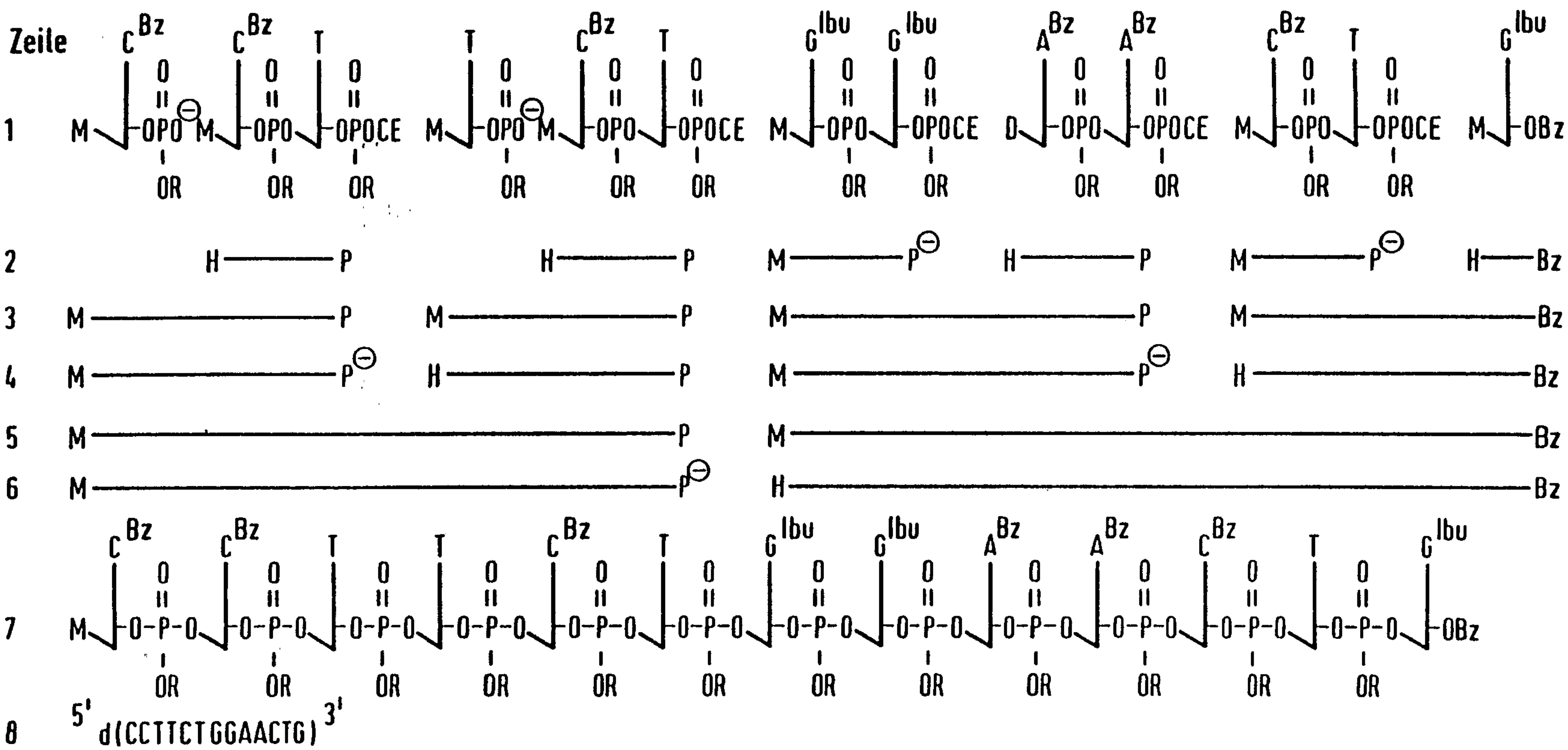

8 5' d(CCTTCTGGAACTG) 3'

Abkürzungen:

M = Monomethoxytrityl
R = 2-Chlorphenyl
CE = 2-Cyanoethyl
Bz = Benzoyl
Ibu = Isobutyl
D = Dimethoxytrityl
P = Phosphotriester
$P^{\ominus}$ = Phosphodiester
H = 5'-OH

Fig. 3

SYNTHESE EINER HuIFN-β- UND HuIFN-α-SPEZIFISCHEN SONDE UND IHRE VERWENDUNG FÜR DIE IDENTIFIZIERUNG HUMANES LYMPHOBLASTOID-IFN ENTHALTENDER KLONE

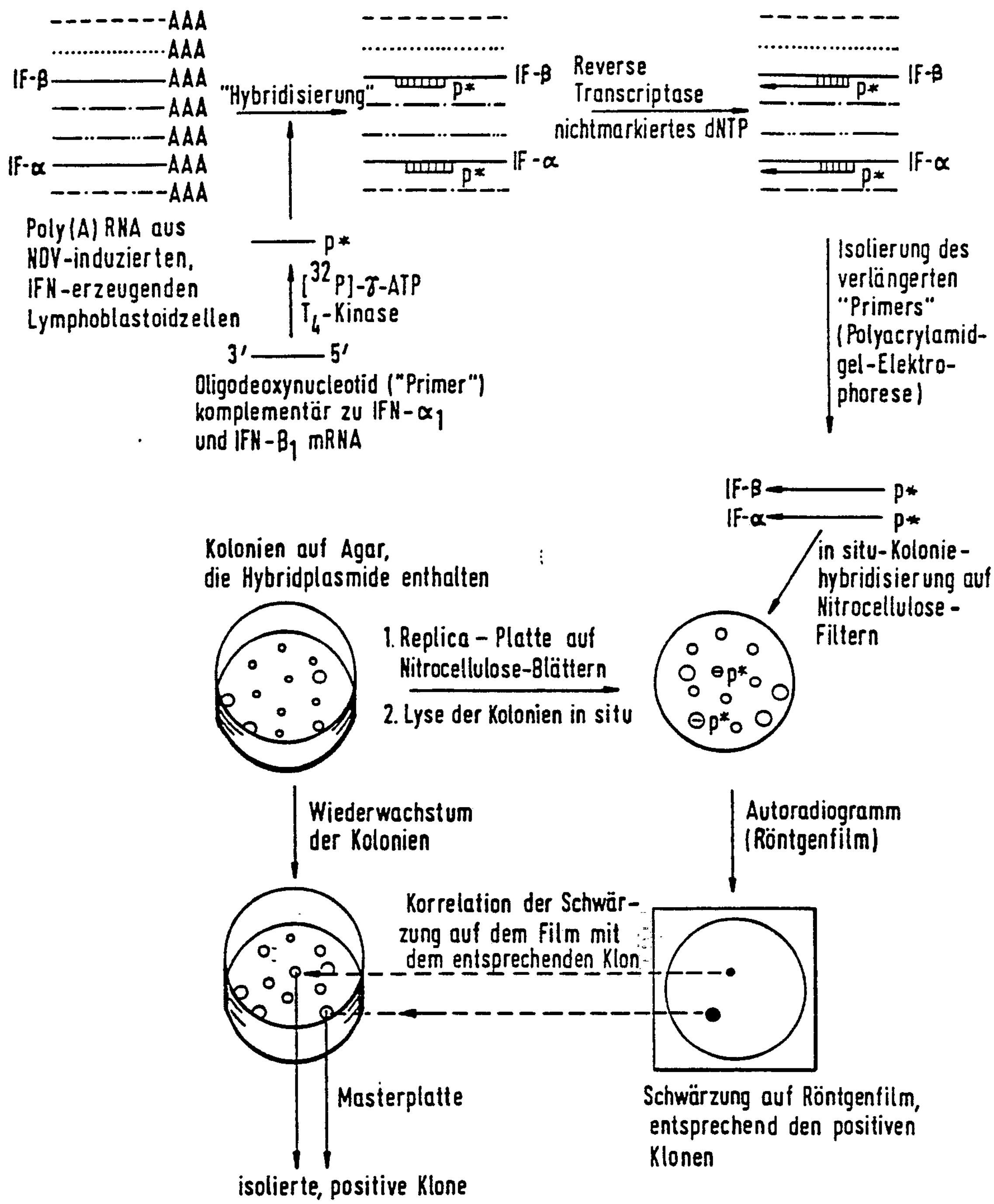

# Fig. 4

NUCLEOTIDSEQUENZ DES cDNA-INSERTS DER REKOMBINANTEN PLASMID-DNA CG-pBR 322/HLycIFN-1'b

```
         1
         MET ALA LEU SER PHE SER LEU LEU MET ALA VAL LEU VAL LEU SER TYR LYS SER ILE CYS SER LEU GLY
ACATCCCA ATG GCC CTG TCC TTT TCT TTA CTG ATG GCC GTG CTG GTG CTC AGC TAC AAA TCC ATC TGT TCT CTG GGC 69

                                                                                             PRO
CYS ASP LEU PRO GLN THR HIS SER LEU GLY ASN ARG ARG ALA LEU ILE LEU LEU ALA GLN MET GLY ARG ILE SER
TGT GAT CTG CCT CAG ACC CAC AGC CTG GGT AAT AGG AGG GCC TTG ATA CTC CTG GCA CAA ATG GGA AGA ATC CCC 144

PRO PHE SER CYS LEU LYS ASP ARG HIS ASP PHE GLY PHE PRO GLN GLU GLU PHE ASP GLY ASN GLN PHE GLN LYS
CCT TTC TCC TGC CTG AAG GAC AGA CAT GAC TTT GGA TTT CCC CAG GAG GAG TTT GAT GGC AAC CAG TTC CAG AAG 219

ALA GLN ALA ILE SER VAL LEU HIS GLU MET ILE GLN GLN THR PHE ASN LEU PHE SER THR LYS ASP SER SER ALA
GCT CAA GCC ATC TCT GTC CTC CAT GAG ATG ATC CAG CAG ACC TTC AAT CTC TTC AGC ACA AAG GAC TCA TCT GCT 294

THR TRP GLU GLN SER LEU LEU GLU LYS PHE SER THR GLU LEU ASN GLN GLN LEU ASN ASP LEU GLU ALA CYS VAL
ACT TGG GAA CAG AGC CTC CTA GAA AAA TTT TCC ACT GAA CTT AAC CAG CAG CTG AAT GAC CTG GAA GCC TGC GTG 369

ILE GLN GLU VAL GLY VAL GLU GLU THR PRO LEU MET ASN VAL ASP SER ILE LEU ALA VAL LYS LYS TYR PHE GLN
ATA CAG GAG GTT GGG GTG GAA GAG ACT CCC CTG ATG AAT GTG GAC TCC ATC CTG GCT GTG AAG AAA TAC TTC CAA 444

ARG ILE THR LEU TYR LEU THR GLU LYS LYS TYR SER PRO CYS ALA TRP GLU VAL VAL ARG ALA GLU ILE MET ARG
AGA ATC ACT CTT TAT CTG ACA GAG AAG AAA TAC AGC CCT TGT GCC TGG GAG GTT GTC AGA GCA GAA ATC ATG AGA 519

SER PHE SER LEU SER LYS ILE PHE GLN GLU ARG LEU ARG ARG LYS GLU
TCC TTC TCT TTA TCA AAA ATT TTT CAA GAA AGA TTA AGG AGG AAG GAA TGA AACCTGTTTCAACATGGAAATGATCTGTATT 601
GACTAATACACCAGTCCACACTTCTATGACTTCTGCCATTTCAAAGACTCATTTCTCCTATAACCACCGCATGAGTTGAATCAAAATTTTCAGATCTTT 700
TCAGGAGTGTAAGGAAACATCATGTTTACCTGTGCAGGCACTAGTCCTTTACAGATGACCATGCTGATAGATCTAATTATCTATCTATTGAAATATTTA 799
TTTATTTATTAGATTTAAATTATTTTTGTCCATGTAATATTATGTGTACTTTTACATTGTGTTATATCAAAATATGTTATTTATATTTAGTCAATATAT 898
TATTTTCTTTTTATTAATTTTTACTATTAAAACTTCTTATATTATTTGTTTATTG
```

# Fig. 5

NUCLEOTIDSEQUENZ DES cDNA-INSERTS DER REKOMBINANTEN PLASMID-DNA CG-pBR 322/HLycIFN-$\beta_1$

```
                                                  SER SER ASN PHE GLN CYS GLN LYS LEU LEU TRP GLN LEU ASN
                                                  AGC AGC AAT TTT CAG TGT CAG AAG CTC CTG TGG CAA TTG AAT 138

GLY ARG LEU GLU TYR CYS LEU LYS ASP ARG MET ASN PHE ASP ILE PRO GLU GLU ILE LYS GLN LEU GLN GLN PHE
GGG AGG CTT GAA TAC TGC CTC AAG GAC AGG ATG AAC TTT GAC ATC CCT GAG GAG ATT AAG CAG CTG CAG CAG TTC 213

GLN LYS GLU ASP ALA ALA LEU THR ILE TYR GLU MET LEU GLN ASN ILE PHE ALA ILE PHE ARG GLN ASP SER SER
CAG AAG GAG GAC GCC GCA TTG ACC ATC TAT GAG ATG CTC CAG AAC ATC TTT GCT ATT TTC AGA CAA GAT TCA TCT 288

SER THR GLY TRP ASN GLU THR ILE VAL GLU ASN LEU LEU ALA ASN VAL TYR HIS GLN ILE ASN HIS LEU LYS THR
AGC ACT GGC TGG AAT GAG ACT ATT GTT GAG AAC CTC CTG GCT AAT GTC TAT CAT CAG ATA AAC CAT CTG AAG ACA 363

VAL LEU GLU GLU LYS LEU GLU LYS GLU ASP PHE THR ARG GLY LYS LEU MET SER SER LEU HIS LEU LYS ARG TYR
GTC CTG GAA GAA AAA CTG GAG AAA GAA GAT TTC ACC AGG GGA AAA CTC ATG AGC AGT CTG CAC CTG AAA AGA TAT 438

TYR GLY ARG ILE LEU HIS TYR LEU LYS ALA LYS GLU TYR SER HIS CYS ALA TRP THR ILE VAL ARG VAL GLU ILE
TAT GGG AGG ATT CTG CAT TAC CTG AAG GCC AAG GAG TAC AGT CAC TGT GCC TGG ACC ATA GTC AGA GTG GAA ATC 513

LEU ARG ASN PHE TYR PHE ILE ASN ARG LEU THR GLY TYR LEU ARG ASN
CTA AGG AAC TTT TAC TTC ATT AAC AGA CTT ACA GGT TAC CTC CGA AAC TGAAGATCTCCTAGCCTGTGCCTCTGGGACTGGAC 596

AATTGCTTCAAGCATTCTTCAACCAGCAGATGCTGTTTAAGTGACTGATGGCTAATGTACTGCATATGAAAGGACACTAGAAGATTTTGAAATTTTTAT 695

TAAATTATGAGTTATTTTTATTTATTTAAATTTTATTTTGGAAAATAAATTATTTTTGGTGCAAAAGTC
```

# Fig. 6

NUCLEOTIDSEQUENZ DES cDNA-INSERTS DER REKOMBINANTEN PLASMID-DNA CG-pBR 322/HLycIFN-$4_1$

```
                                                                                      SER SER ALA
                                                                                      TCA TCT GCT 294

ALA TRP ASP GLU THR LEU LEU ASP LYS PHE TYR THR GLU LEU TYR GLN GLN LEU ASN ASP LEU GLU ALA CYS VAL
GCT TGG GAT GAG ACC CTC CTA GAC AAA TTC TAC ACT GAA CTC TAC CAG CAG CTG AAT GAC CTG GAA GCC TGT GTG 369

ILE GLN GLY VAL GLY VAL THR GLU THR PRO LEU MET LYS GLU ASP SER ILE LEU ALA VAL ARG LYS TYR PHE GLN
ATA CAG GGG GTG GGG GTG ACA GAG ACT CCC CTG ATG AAG GAG GAC TCC ATT CTG GCT GTG AGG AAA TAC TTC CAA 444

ARG ILE THR LEU TYR LEU LYS GLU LYS LYS TYR SER PRO CYS ALA TRP GLU VAL VAL ARG ALA GLU ILE MET ARG
AGA ATC ACT CTC TAT CTG AAA GAG AAG AAA TAC AGC CCT TGT GCC TGG GAG GTT GTC AGA GCA GAA ATC ATG AGA 519

SER PHE SER LEU SER THR ASN LEU GLN GLU SER LEU ARG SER LYS GLU
TCT TTT TCT TTG TCA ACA AAC TTG CAA GAA AGT TTA AGA AGT AAG GAA TGA AAACTGGTTCAACATGGAAATGATTTTCATT 601

GATTCGTATGCCAGCTCACCTTTTTATGATCTGCCATTTCAAAGACTCATGTTTCTGCTATGACCATGACACGATTTAAATCTTTTCAAATGTTTTTAG 700

GAGTATTAATCAACATTGTATTCAGCTCTTAAGGCACTAGTCCCTTACAGAGGACCATGCTGACTGATCCATTATCTATTTAAATATTTTTAAAATATT 799

ATTTATTTAACTATTTATAAAACAACTTATTTTTGTTCATATTATGTCATGTGCACCTTTGCACAGTGGTTAATGTAATAAAATGTGTTCTTTGTATTT 898

GGTATATTTATTTTGTGTTGTTCATTGAACTTTTGCTATGGAACTTTTGTACTTGTTTATTCTTTAAAATGAAATTCCAAGCCTAATTGTGCAACCTGA 997

TTACAGAATAACTGGTACACTTCATTTATCCATCAATATTATATTCAAGATATAAGTAAAAATAAACTTTCTGTAAACCAGTTG
```

## Fig. 7

NUCLEOTIDSEQUENZ DES cDNA-INSERTS DER REKOMBINANTEN PLASMID-DNA CG-pBR 322/HLycIFN-$8'_1$

```
                                                         CCCAAGGTTCAGAGTCACCCATCTCAGCAAGCCCAGAAGCATCTGCAAT

        1
        MET ALA SER PRO PHE ALA LEU LEU MET ALA LEU VAL VAL LEU SER CYS LYS SER SER CYS SER LEU GLY
ATCTATG ATG GCC TCG CCC TTT GCT TTA CTG ATG GCC CTG GTG GTG CTC AGC TGC AAG TCA AGC TGC TCT CTG GGC 69

CYS ASP LEU PRO GLU THR HIS SER LEU ASP ASN ARG ARG THR LEU MET LEU LEU ALA GLN MET SER ARG ILE SER
TGT GAT CTC CCT GAG ACC CAC AGC CTG GAT AAC AGG AGG ACC TTG ATG CTC CTG GCA CAA ATG AGC AGA ATC TCT 144

PRO SER SER CYS LEU MET ASP ARG HIS ASP PHE GLY PHE PRO GLN GLU GLU PHE ASP GLY ASN GLN PHE GLN LYS
CCT TCC TCC TGT CTG ATG GAC AGA CAT GAC TTT GGA TTT CCC CAG GAG GAG TTT GAT GGC AAC CAG TTC CAG AAG 219

ALA PRO ALA ILE SER VAL LEU HIS GLU LEU ILE GLN GLN ILE PHE ASN LEU PHE THR THR LYS ASP SER SER ALA
GCT CCA GCC ATC TCT GTC CTC CAT GAG CTG ATC CAG CAG ATC TTC AAC CTC TTT ACC ACA AAA GAT TCA TCT GCT 294

ALA TRP ASP GLU ASP LEU LEU ASP LYS PHE CYS THR GLU LEU TYR GLN GLN LEU ASN ASP LEU GLU ALA CYS VAL
GCT TGG GAT GAG GAC CTC CTA GAC AAA TTC TGC ACC GAA CTC TAC CAG CAG CTG AAT GAC TTG GAA GCC TGT GTG 369

MET GLN GLU GLU ARG VAL GLY GLU THR PRO LEU MET ASN ALA ASP SER ILE LEU ALA VAL LYS LYS TYR PHE ARG
ATG CAG GAG GAG AGG GTG GGA GAA ACT CCC CTG ATG AAT GCG GAC TCC ATC TTG GCT GTG AAG AAA TAC TTC CGA 444

ARG ILE THR LEU TYR LEU THR GLU LYS LYS TYR SER PRO CYS ALA TRP GLU VAL VAL ARG ALA GLU ILE MET ARG
AGA ATC ACT CTC TAT CTG ACA GAG AAG AAA TAC AGC CCT TGT GCC TGG GAG GTT GTC AGA GCA GAA ATC ATG AGA 519

SER LEU SER LEU SER THR ASN LEU GLN GLU ARG LEU ARG ARG LYS GLU
TCC CTC TCT TTA TCA ACA AAC TTG CAA GAA AGA TTA AGG AGG AAG GAA TAA CACCTGGTCCAACATGAAACAATTCTTATTG 601

ACTCATATACCAGGTCACGCTTTCATGAATTCTGCCATTTCAAAGACTCTCACTTCTGCTATAACTATGACCATGCTGATAAACTGATTTATCTATTTA 700

AATATTTATTTAGCTATTCATAAGATTTAAATTATTTTTGTTCATATAACATCATGTGCATCTTTACACTGTGGTTAGTGTAATAAAACATGTTCCTTA 799

TATTTACTCAAATTCATTATTTTA
```

Fig. 8

NUCLEOTIDSEQUENZ DES cDNA-INSERTS DER REKOMBINANTEN PLASMID-DNA CG-pBR 322/HLycIFN-5[1]

```
                                                                                   ATCTGAACCAGCTCAGCAGCATCCACAAC
        1
        MET ALA LEU THR PHE TYR LEU LEU VAL ALA LEU VAL VAL LEU SER TYR LYS SER PHE SER SER LEU GLY
ATCTACA ATG GCC TTG ACT TTT TAT TTA CTG GTG GCC CTA GTG GTG CTC AGC TAC AAG TCA TTC AGC TCT CTG GGC 69

CYS ASP LEU PRO GLN THR HIS SER LEU GLY ASN ARG ARG ALA LEU ILE LEU LEU ALA GLN MET ARG ARG ILE SER
TGT GAT CTG CCT CAG ACT CAC AGC CTG GGT AAC AGG AGG GCC TTG ATA CTC CTG GCA CAA ATG CGA AGA ATC TCT 144

PRO PHE SER CYS LEU LYS ASP ARG HIS ASP PHE GLU PHE PRO GLN GLU GLU PHE ASP ASP LYS GLN PHE GLN LYS
CCT TTC TCC TGC CTG AAG GAC AGA CAT GAC TTT GAA TTC CCC CAG GAG GAG TTT GAT GAT AAA CAG TTC CAG AAG 219

ALA GLN ALA ILE SER VAL LEU HIS GLU MET ILE GLN GLN THR PHE ASN LEU PHE SER THR LYS ASP SER SER ALA
GCT CAA GCC ATC TCT GTC CTC CAT GAG ATG ATC CAG CAG ACC TTC AAC CTC TTC AGC ACA AAG GAC TCA TCT GCT 294

ALA LEU ASP GLU THR LEU LEU ASP GLU PHE TYR ILE GLU LEU ASP GLN GLN LEU ASN ASP LEU GLU SER CYS VAL
GCT TTG GAT GAG ACC CTT CTA GAT GAA TTC TAC ATC GAA CTT GAC CAG CAG CTG AAT GAC CTG GAG TCC TGT GTG 369

MET GLN GLU VAL GLY VAL ILE GLU SER PRO LEU MET TYR GLU ASP SER ILE LEU ALA VAL ARG LYS TYR PHE GLN
ATG CAG GAA GTG GGG GTG ATA GAG TCT CCC CTG ATG TAC GAG GAC TCC ATC CTG GCT GTG AGG AAA TAC TTC CAA 444

ARG ILE THR LEU TYR LEU THR GLU LYS LYS TYR SER SER CYS ALA TRP GLU VAL VAL ARG ALA GLU ILE MET ARG
AGA ATC ACT CTA TAT CTG ACA GAG AAG AAA TAC AGC TCT TGT GCC TGG GAG GTT GTC AGA GCA GAA ATC ATG AGA 519

SER PHE SER LEU SER ILE ASN LEU GLN LYS ARG LEU LYS SER LYS GLU
TCC TTC TCT TTA TCA ATC AAC TTG CAA AAA AGA TTG AAG AGT AAG GAA TGA GACCTGGTACAACACGGAAATGATTCTTATA 601

GACTAATACAGCAGCTCACACTTCGACAAGTTGTGCTCTTTCAAAGACCCTTGTTTCTGCCAAAACCATGCTATGTTTTGAATCAAATGTGTCAAGTGT 700

TTTCAGGAGTGTTAAGCAACATCCTGTTCAGCTGTATGGGCACTAGTCCCTTACAGATGACCATGCTGATGGATCTATTCATCTATTTATTTAAATCTT 799

TATTTAGTTAACTATCTATAGGGCTTAAATTAGTTTTGTTCATATTATATTATGTGAACTTTTACATTGTGAATTGTGTAACAAAAACATGTTCTTTAT 898

ATTTATTATTTTGCCTTGTTTATTAAATTTTTACTATAG
```

AUFBAU DER CG-pBR (AP)/Ly IFN-α-1 REKOMBINANTEN DNA

ApPr: β-Lactamase - Expressionskontrollbereich

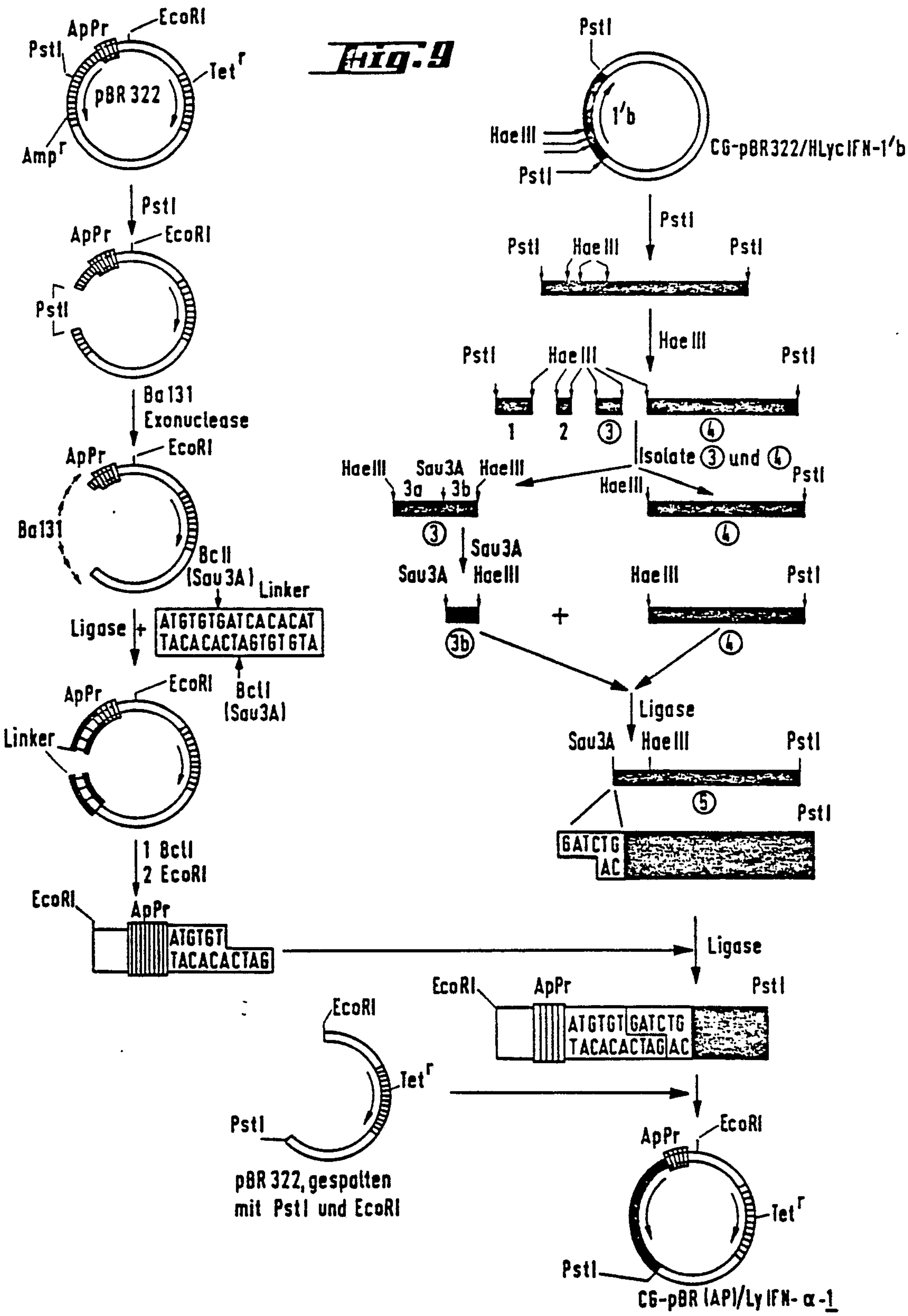


Fig. 9

**Fig. 10**

CG-pBR(AP)/LyIFN-α-1

```
                                                    PROMOTOR β-LACTAMASE       MET
                                        ...GAGACAATAACCCTGATAAATGCTTCAATAATATTGAAAAAGGAAGAGTAATG
                                                                                          PRO
CYS ASP LEU PRO GLN THR HIS SER LEU GLY ASN ARG ARG ALA LEU ILE LEU LEU ALA GLN MET GLY ARG ILE SER
TGT GAT CTG CCT CAG ACC CAC AGC CTG GGT AAT AGG AGG GCC TTG ATA CTC CTG GCA CAA ATG GGA AGA ATC CCC 144

PRO PHE SER CYS LEU LYS ASP ARG HIS ASP PHE GLY PHE PRO GLN GLU GLU PHE ASP GLY ASN GLN PHE GLN LYS
CCT TTC TCC TGC CTG AAG GAC AGA CAT GAC TTT GGA TTT CCC CAG GAG GAG TTT GAT GGC AAC CAG TTC CAG AAG 219

ALA GLN ALA ILE SER VAL LEU HIS GLU MET ILE GLN GLN THR PHE ASN LEU PHE SER THR LYS ASP SER SER ALA
GCT CAA GCC ATC TCT GTC CTC CAT GAG ATG ATC CAG CAG ACC TTC AAT CTC TTC AGC ACA AAG GAC TCA TCT GCT 294

THR TRP GLU GLN SER LEU LEU GLU LYS PHE SER THR GLU LEU ASN GLN GLN LEU ASN ASP LEU GLU ALA CYS VAL
ACT TGG GAA CAG AGC CTC CTA GAA AAA TTT TCC ACT GAA CTT AAC CAG CAG CTG AAT GAC CTG GAA GCC TGC GTG 369

ILE GLN GLU VAL GLY VAL GLU GLU THR PRO LEU MET ASN VAL ASP SER ILE LEU ALA VAL LYS LYS TYR PHE GLN
ATA CAG GAG GTT GGG GTG GAA GAG ACT CCC CTG ATG AAT GTG GAC TCC ATC CTG GCT GTG AAG AAA TAC TTC CAA 444

ARG ILE THR LEU TYR LEU THR GLU LYS LYS TYR SER PRO CYS ALA TRP GLU VAL VAL ARG ALA GLU ILE MET ARG
AGA ATC ACT CTT TAT CTG ACA GAG AAG AAA TAC AGC CCT TGT GCC TGG GAG GTT GTC AGA GCA GAA ATC ATG AGA 519

SER PHE SER LEU SER LYS ILE PHE GLN GLU ARG LEU ARG ARG LYS GLU
TCC TTC TCT TTA TCA AAA ATT TTT CAA GAA AGA TTA AGG AGG AAG GAA TGA AACCTGTTTCAACATGGAAATGATCTGTATT 601

GACTAATACACCAGTCCACACTTCTATGACTTCTGCCATTTCAAAGACTCATTTCTCCTATAACCACCGCATGAGTTGAATCAAAATTTTCAGATCTTT 700

TCAGGAGTGTAAGGAAACATCATGTTTACCTGTGCAGGCACTAGTCCTTTACAGATGACCATGCTGATAGATCTAATTATCTATCTATTGAAATATTTA 799

TTTATTTATTAGATTTAAATTATTTTTGTCCATGTAATATTATGTGTACTTTTACATTGTGTTATATCAAAATATGTTATTTATATTTAGTCAATATAT 898

TATTTTCTTTTTATTAATTTTTACTATTAAAACTTCTTATATTATTTGTTTATTG...
```

Fig. 11

AUFBAU DES REKOMBINANTEN DNA-PLASMIDS CG-pBR (AP)/Ly IFN-α-3

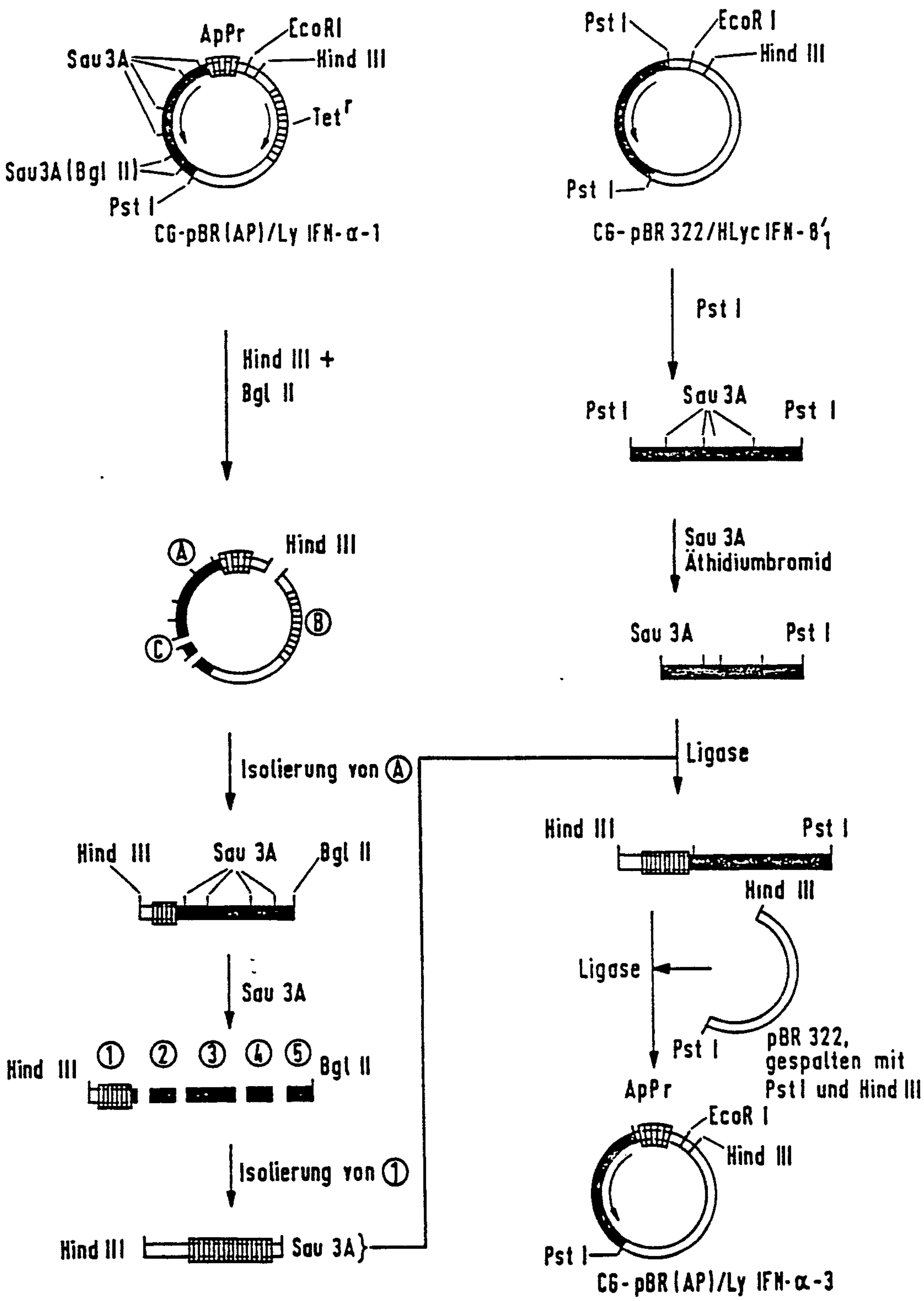

## Fig. 12

CG-pBR(AP)/LyIFN-α-3

PROMOTOR β-LACTAMASE MET

...GAGACAATAACCCTGATAAATGCTTCAATAATATTGAAAAAGGAAGAGTAATG

CYS ASP LEU PRO GLU THR HIS SER LEU ASP ASN ARG ARG THR LEU MET LEU LEU ALA GLN MET SER ARG ILE SER
TGT GAT CTC CCT GAG ACC CAC AGC CTG GAT AAC AGG AGG ACC TTG ATG CTC CTG GCA CAA ATG AGC AGA ATC TCT 144

PRO SER SER CYS LEU MET ASP ARG HIS ASP PHE GLY PHE PRO GLN GLU GLU PHE ASP GLY ASN GLN PHE GLN LYS
CCT TCC TCC TGT CTG ATG GAC AGA CAT GAC TTT GGA TTT CCC CAG GAG GAG TTT GAT GGC AAC CAG TTC CAG AAG 219

ALA PRO ALA ILE SER VAL LEU HIS GLU LEU ILE GLN GLN ILE PHE ASN LEU PHE THR THR LYS ASP SER SER ALA
GCT CCA GCC ATC TCT GTC CTC CAT GAG CTG ATC CAG CAG ATC TTC AAC CTC TTT ACC ACA AAA GAT TCA TCT GCT 294

ALA TRP ASP GLU ASP LEU LEU ASP LYS PHE CYS THR GLU LEU TYR GLN GLN LEU ASN ASP LEU GLU ALA CYS VAL
GCT TGG GAT GAG GAC CTC CTA GAC AAA TTC TGC ACC GAA CTC TAC CAG CAG CTG AAT GAC TTG GAA GCC TGT GTG 369

MET GLN GLU GLU ARG VAL GLY GLU THR PRO LEU MET ASN ALA ASP SER ILE LEU ALA VAL LYS LYS TYR PHE ARG
ATG CAG GAG GAG AGG GTG GGA GAA ACT CCC CTG ATG AAT GCG GAC TCC ATC TTG GCT GTG AAG AAA TAC TTC CGA 444

ARG ILE THR LEU TYR LEU THR GLU LYS LYS TYR SER PRO CYS ALA TRP GLU VAL VAL ARG ALA GLU ILE MET ARG
AGA ATC ACT CTC TAT CTG ACA GAG AAG AAA TAC AGC CCT TGT GCC TGG GAG GTT GTC AGA GCA GAA ATC ATG AGA 519

SER LEU SER LEU SER THR ASN LEU GLN GLU ARG LEU ARG ARG LYS GLU
TCC CTC TCT TTA TCA ACA AAC TTG CAA GAA AGA TTA AGG AGG AAG GAA TAA CACCTGGTCCAACATGAAACAATTCTTATTG 601

ACTCATATACCAGGTCACGCTTTCATGAATTCTGCCATTTCAAAGACTCTCACTTCTGCTATAACTATGACCATGCTGATAAACTGATTTATCTATTTA 700

AATATTTATTTAGCTATTCATAAGATTTAAATTATTTTTGTTCATATAACATCATGTGCATCTTTACACTGTGGTTAGTGTAATAAAACATGTTCCTTA 799

TATTTACTCAAATTCATTATTTT...

**Fig. 13**

CG-pBR(AP)/LyIFN-α-2

PROMOTOR β-LACTAMASE MET

...GAGACAATAACCCTGATAAATGCTTCAATAATATTGAAAAAGGAAGAGTAATG

CYS ASP LEU PRO GLN THR HIS SER LEU GLY ASN ARG ARG ALA LEU ILE LEU LEU ALA GLN MET ARG ARG ILE SER
TGT GAT CTG CCT CAG ACT CAC AGC CTG GGT AAC AGG AGG GCC TTG ATA CTC CTG GCA CAA ATG CGA AGA ATC TCT 144

PRO PHE SER CYS LEU LYS ASP ARG HIS ASP PHE GLU PHE PRO GLN GLU GLU PHE ASP ASP LYS GLN PHE GLN LYS
CCT TTC TCC TGC CTG AAG GAC AGA CAT GAC TTT GAA TTC CCC CAG GAG GAG TTT GAT GAT AAA CAG TTC CAG AAG 219

ALA GLN ALA ILE SER VAL LEU HIS GLU MET ILE GLN GLN THR PHE ASN LEU PHE SER THR LYS ASP SER SER ALA
GCT CAA GCC ATC TCT GTC CTC CAT GAG ATG ATC CAG CAG ACC TTC AAC CTC TTC AGC ACA AAG GAC TCA TCT GCT 294

ALA LEU ASP GLU THR LEU LEU ASP GLU PHE TYR ILE GLU LEU ASP GLN GLN LEU ASN ASP LEU GLU SER CYS VAL
GCT TTG GAT GAG ACC CTT CTA GAT GAA TTC TAC ATC GAA CTT GAC CAG CAG CTG AAT GAC CTG GAG TCC TGT GTG 369

MET GLN GLU VAL GLY VAL ILE GLU SER PRO LEU MET TYR GLU ASP SER ILE LEU ALA VAL ARG LYS TYR PHE GLN
ATG CAG GAA GTG GGG GTG ATA GAG TCT CCC CTG ATG TAC GAG GAC TCC ATC CTG GCT GTG AGG AAA TAC TTC CAA 444

ARG ILE THR LEU TYR LEU THR GLU LYS LYS TYR SER SER CYS ALA TRP GLU VAL VAL ARG ALA GLU ILE MET ARG
AGA ATC ACT CTA TAT CTG ACA GAG AAG AAA TAC AGC TCT TGT GCC TGG GAG GTT GTC AGA GCA GAA ATC ATG AGA 519

SER PHE SER LEU SER ILE ASN LEU GLN LYS ARG LEU LYS SER LYS GLU
TCC TTC TCT TTA TCA ATC AAC TTG CAA AAA AGA TTG AAG AGT AAG GAA TGA GACCTGGTACAACACGGAAATGATTCTTATA 601

GACTAATACAGCAGCTCACACTTCGACAAGTTGTGCTCTTTCAAAGACCCTTGTTTCTGCCAAAACCATGCTATGTTTTGAATCAAATGTGTCAAGTGT 700

TTTCAGGAGTGTTAAGCAACATCCTGTTCAGCTGTATGGGCACTAGTCCCTTACAGATGACCATGCTGATGGATCTATTCATCTATTTATTTAAATCTT 799

TATTTAGTTAACTATCTATAGGGCTTAAATTAGTTTTGTTCATATTATATTATGTGAACTTTTACATTGTGAATTGTGTAACAAAAACATGTTCTTTAT 898

ATTTATTATTTTGCCTTGTTTATTAAATTTTTACTATAG...